(19) Europäisches Patentamt
European Patent Office
Office européen des brevets

(11) Veröffentlichungsnummer: **0 045 049**
**B1**

(12) **EUROPÄISCHE PATENTSCHRIFT**

(45) Veröffentlichungstag der Patentschrift: **26.06.85**

(21) Anmeldenummer: **81105781.9**

(22) Anmeldetag: **22.07.81**

(51) Int. Cl.⁴: **C 07 C 103/48,** A 61 K 37/22, C 07 D 307/68, C 07 D 307/16, C 07 D 249/10, C 07 C 143/13, C 07 C 143/86, C 07 C 147/05, C 07 C 103/375, C 07 C 103/737, C 07 K 5/00

(54) **Neue Arylamin-derivate, Verfahren zu deren Herstellung und deren Verwendung als Mikrobizide.**

(30) Priorität: **25.07.80 CH 5709/80**
**25.07.80 CH 5710/80**

(43) Veröffentlichungstag der Anmeldung:
**03.02.82 Patentblatt 82/05**

(45) Bekanntmachung des Hinweises auf die Patenterteilung:
**26.06.85 Patentblatt 85/26**

(84) Benannte Vertragsstaaten:
**AT BE CH DE FR GB IT LI NL SE**

(56) Entgegenhaltungen:
**AT-B- 268 254**
**DE-A-2 515 091**
**DE-A-3 010 412**
**FR-A-2 443 454**
**US-A-3 597 482**

**Dr. F. Klages, Einführung in die organische Chemie (1965), S. 206 und S. 285**

(73) Patentinhaber: **CIBA-GEIGY AG**
**Postfach**
**CH-4002 Basel (CH)**

(72) Erfinder: **Hubele, Adolf, Dr.**
**Obere Egg 9**
**CH-4465 Magden (CH)**
Erfinder: **Eckhardt, Wolfgang, Dr.**
**Breslauerstrasse 16**
**D-7850 Lörrach (DE)**
Erfinder: **Riebli, Peter**
**Dittingerstrasse 12**
**CH-4053 Basel (CH)**

(74) Vertreter: **Zumstein, Fritz sen., Dr. et al**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 045 049 B1

**Beschreibung**

Die vorliegende Erfindung betrifft Verbindungen der allgemeinen Formel I

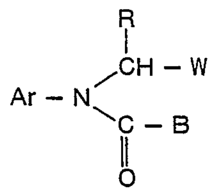
(I)

worin

R für Wasserstoff oder Methyl steht;

Ar eine der aromatischen Gruppe n

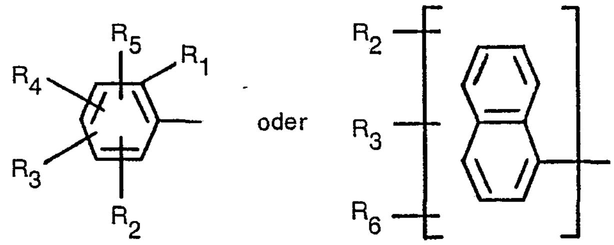

repräsentiert, wobei

$R_1$ $C_1$—$C_3$-Alkyl, $C_1$—$C_3$-Alkoxy oder Halogen bedeutet;

$R_2$ für $NO_2$ oder $NH_2$ steht;

$R_3$ Wasserstoff, $C_1$—$C_3$-Alkyl, $C_1$—$C_3$-Alkoxy oder Halogen;

$R_4$ Wasserstoff oder $C_1$—$C_3$-Alkyl;

$R_5$ Wasserstoff oder $C_1$—$C_3$-Alkyl;

$R_6$ Wasserstoff, $C_1$—$C_3$-Alkyl oder Halogen bedeutet;

W für Cyano, —$COOR_8$, —$C(R_9)$=$C(R_{10})$ $(R_{11})$ oder —C≡C—$R_{12}$ steht, wobei

$R_8$ $C_1$—$C_3$-Alkyl;

$R_9$, $R_{10}$, $R_{11}$ und $R_{12}$ unabhängig voneinander Wasserstoff, $C_1$—$C_3$-Alkyl oder Halogen bedeuten und

B für $C_3$—$C_4$-Alkyl, $C_2$—$C_4$-Alkenyl, Cyclopropyl, 2-Furyl, 2-Tetrahydrofuryl, β-($C_1$—$C_2$-Alkoxy)ethyl oder die Gruppe $CH_2Z$ steht, wobei

Z für 1H—1,2,4-Triazolyl, Methylsulfonyl, X—$R_{13}$, $OSO_2$—$R_{14}$ oder —$N(R_{15})$ $(R_{16})$ steht, wobei

X Sauerstoff oder Schwefel bedeutet;

$R_{13}$ für eine Alkyl-, Alkenyl- oider Alkinylgruppe mit maximal 4 Kohlenstoffatomen steht;

$R_{14}$ $C_1$—$C_3$-Alkyl oder NH($C_1$—$C_3$-Alkyl) bedeutet und

$R_{15}$ und $R_{16}$ unabhängig voneinander für $C_1$—$C_3$-Alkyl stehen.

Die vorliegende Erfindung umfasst somit Anilin-derivate, deren Substituenten $R_2$, $R_3$, $R_4$ und $R_5$ im Anilinring

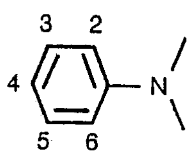

fluktuieren, so dass jeder dieser Substituenten die Positionen 3, 4, 5 oder 6 einnehmen kann; dabei sind diejenigen Verbindungen bevorzugt die in beiden ortho-Positionen (= 2,6-Positionen) disubstituiert sind. Die Erfindung umfasst ebenso α-Naphthylamin-derivate, deren Substituenten $R_2$, $R_3$ und $R_6$ im Naphthalin-Ringsystem

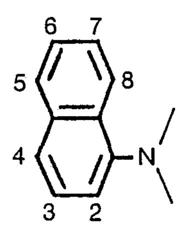

fluktuieren, so dass jeder dieser Substituenten die Positionen 2, 3, 4, 5, 6, 7 oder 8 einnehmen kann; dabei sind α-Naphthylamin-derivate bevorzugt, die in der 2-Position substituiert sind.

Unter Alkyl oder Alkylanteil eines anderen Substituenten sind je nach Zahl der angegebenen Kohlenstoffatome beispielsweise folgende Gruppen zu verstehen: Methyl, Ethyl, Propyl oder Butyl sowie ihre Isomeren wie Isopropyl, iso-Butyl, sek.-Butyl oder tert.-Butyl. Alkenyl steht beispielsweise für Vinyl, Propenyl-(1), Allyl, Butenyl-(1), Butenyl-(2) oder Butenyl-(3), und Alkenyl bedeutet insbesondere Propargyl. Halogen steht heir und im folgenden für Fluor, Chlor, Brom oder Jod, vorzugsweise fur Chlor oder Brom.

Verbindungen der Formel I sind bei Raumtemperatur luftstabile Harze oder Feststoffe, die sich durch sehr wertvolle mikrobizide Eigenschaften auszeichnen. Diese Wirkstoffe eignen sich sehr gut zur Bekämpfung von schädlichen Mikroorganismen, insbesondere zur Unterdrückung oder Vernichtung von Pilzen, vor allem auf dem Agrarsektor. Besonders bevorzugt sind die Wirkstoffe im Umfang der Formel I, bei denen der Substituent $R_2$ die 3-Position im Molekül einnimmt.

Es sind bereits substituierte Acylanilin-derivate als Mikrobizide vorgeschlagen worden — vgl. z.B. DE—OS: 2,643,477; 2,741,437; 2,513,788: 2,513,732; 2,515,091 oder 2,927,461. Die bekannt gewordenen Präparate zeigen jedoch häufig eine Reihe von Nachteilen. Hierzu gehören Applikationsnachteile im Zusammenhang mit den physikochemischen Eigenschaften der Substanzen und ihrem Eindringungs- bzw. Haftungsvermögen an der zu schützenden Pflanze. In der Praxis werden Blattfungizide üblicherweise versprüht und bedecken in feinster Verteilung die Pflanzenoberfläche. Dabei sind sie zwangsläufig den herrschenden Witterungseinflüssen unterworfen, wie Regen, Wind, Sonneneinstrahlung und Temperaturen. Bevor sie ihre volle Wirkung entfalten können, werden Aktivesubstanzen häufig vom Pflanzenkörper entfernt, etwa aufgrund unerwünscht hoher Wasserlöslichkeit, geringer Lichtstabilität, grosser Flüchtigkeit usw. Es erweist sich als ausserordentlich schwierig, hochwirksame Mikrobizide zu finden, bie denen diese physikochemischen Nachteile nicht auftreten.

Es besteht daher in der Praxis ein grosser Bedarf an Fungiziden, die bie sehr kleinen Wirkstoffkonzentrationen hohe fungizide Aktivität aufweisen und gleichzeitig zu einer ausreichend grossen Verweildauer auf der Pflanze befähigt sind, so dass ein anhaltender Schutz gegen phytopathogene Pilze zustandekommt.

Es hat sich nun überraschend gezeigt, dass die neuen Wirkstoffe der Formel I gleichzeitig mehrere für die Praxis vorteilhafte Eigenschaften aufweisen. Sie besitzen nicht nur ein wertvolles Mikrobizidspektrum gegen wichtige Schadpilze, sondern zusätzliche Applikationsvorteile. Durch eine deutlich verringerte Wasserlöslichkeit und einen stark reduzierten Dampfdruck können sie ihre fungizide Wirkung weitgehend unabhängig von den Witterungseinflüssen besser entfalten, da ein deutlich längerer Kontakt mit der Pflanze gewährleistet ist. Aus diesem Grund sind die Verbindungen der Formel I in der Praxis effektiver einsetzbar als vergleichbare Substanzen des Standes der Technik.

Mikrobizide der Formel I mit folgenden Substituententypen oder Kombinationen dieser untereinander sind bevorzugt:

Bei R: Wasserstoff, Methyl
Ar: substituiertes Phenyl, substituiertes α-Naphthyl
$R_1$: Methyl, Ethyl, Methoxy, Ethoxy, Halogen
$R_2$: $NO_2$, $NH_2$
$R_3$, $R_6$: Wasserstoff, Methyl, Ethyl, Methoxy, Ethoxy, Halogen
$R_4$, $R_5$: Wasserstoff, Methyl, Ethyl
W: CN, $COOR_8$, $—C(R_9)=C(R_{10})(R_{11})$, $—C≡C—R_{12}$
$R_8$: $C_1—C_3$-Alkyl
$R_9$, $R_{10}$, $R_{11}$, $R_{12}$: Wasserstoff, Methyl, Chlor, Brom
B: $C_3—C_4$-Alkyl, $C_2—C_4$-Alkenyl, Cyclopropyl, 2-Furyl, 2-Tetrahydrofuryl, $CH_2O(C_1—C_3$-Alkyl), 1H—1,2,4-Triazolylmethyl, $CH_2SO_2CH_3$, $CH_2OSO_2CH_3$, $CH_2OCH_2CH=CH_2$, $CH_2OCH_2C≡CH$, $CH_2OSO_2NHCH_3$, $CH_2N(CH_3)_2$, $CH_2N(C_2H_5)_2$, $CH_2N(C_3H_7\text{-}n)_2$.

Zu einer weiteren bevorzugten Gruppe von Mikrobiziden zählen Verbindungen der Formel I mit folgenden Substituentenkombinationen:

Bei R: Methyl
Ar: substituiertes Phenyl, substituiertes α-Naphthyl
$R_1$: Methyl, Methoxy, Chlor, Brom
$R_2$: $NO_2$, $NH_2$
$R_3$, $R_6$: Wasserstoff, Methyl, Chlor, Brom
$R_4$, $R_5$: Wasserstoff, Methyl
W: CN, $COOR_8$, $—C(R_9)=C(R_{10})(R_{11})$, $—C≡C—R_{12}$
$R_8$: $C_1—C_3$-Alkyl
$R_9$, $R_{10}$, $R_{11}$, $R_{12}$: Wasserstoff, Methyl, Chlor
B: 2-Furyl, 2-Tetrahydrofuryl, $CH_2O(C_1—C_3$-Alkyl, 1H-1,2,4-Triazolylmethyl, $CH_2SO_2CH_3$, $CH_2OSO_2CH_3$, $CH_2OCH_2CH=CH_2$, $CH_2OCH_2C≡CH$, $CH_2OSO_2NHCH_3$, $CH_2N(CH_3)_2$, $CH_2N(C_2H_5)_2$, $CH_2N(C_3H_7\text{-}n)_2$.

Die Anilin-derivate im Umfang der Formel I sind besonders bevorzugt, sie bilden z.B. folgende bevorzugte Untergruppe mit nachfolgenden Substituententypen oder -kombination:

Bei $R_1$: Halogen, Methoxy, Methyl
$R_2$: $NO_2$, $NH_2$
$R_3$: Wasserstoff, Methoxy, Methyl, Halogen
$R_4$, $R_5$, R: Wasserstoff, Methyl
W: $COOR_8$
$R_8$: $C_1$—$C_3$-Alkyl
B: 2-Furyl, 2-Tetrahydrofuryl, $CH_2O(C_1$—$C_3$-Aklyl), $CH_2CH_2OCH_3$, 1H-1,2,4-Triazolylmethyl, $CH_2$—$SO_2CH_3$, $CH_2OSO_2CH_3$, $CH_2OSO_2NHCH_3$, $CH_2OCH_2CH{=}CH_2$, $CH_2OCH_2C{\equiv}CH$.

Eine bevorzugte Gruppe von Mikrobiziden besteht aus Anilin-derivaten der Formel I mit folgenden Substituenten-Kombinationen:

Bei $R_1$: Methyl
$R_2$: $NO_2$, $NH_2$
$R_3$: H, Methyl, Methoxy, Chlor, Brom
$R_4$, $R_5$, R: Wasserstoff, Methyl
W: $COOR_8$
$R_8$: Methyl, iso-Propyl
B: 2-Furyl, 2-Tetrahydrofuryl, $CH_2O(C_1$—$C_3$-Aklyl), $CH_2CH_2OCH_3$, 1H-1,2,4-Triazolylmethyl, $CH_2$—$SO_2CH_3$, $CH_2OSO_2CH_3$, $CH_2OSO_2NHCH_3$ , $CH_2OCH_2CH{=}CH_2$, $CH_2OCH_2C{\equiv}CH$.

Eine weitere bevorzugte Gruppe von Anilin-derviaten der Formel I wird durch folgende Substituentenkombination charakterisiert:

Bei $R_1$: Chlor, Methoxy
$R_2$: $NO_2$, $NH_2$
$R_3$: H, Methyl, Methoxy, Chlor, Brom
$R_4$, $R_5$, R: Wasserstoff, Methyl
W: $COOR_8$
$R_8$: Methyl, iso-Propyl
B: 2-Furyl, 2-Tetrahydrofuryl, $CH_2O(C_1$—$C_3$-Alkyl), $CH_2CH_2OCH_3$, 1H-1,2,4-Triazolylmethyl, $CH_2$—$SO_2CH_3$, $CH_2OSO_2CH_3$, $CH_2OSO_2NHCH_3$, $CH_2OCH_2CH{=}CH_2$, $CH_2OCH_2C{\equiv}CH$.

Verbindungen im Umfang der Formel I, worin X für Sauerstoff steht und $R_{13}$ eine $C_1$—$C_3$-Alkylgruppe bedeutet, sind besonders bevorzugt, vor allem aber diejenigen, in denen $R_{13}$ für Methyl, Ethyl oder iso-Propyl steht.

Zu den bevorzugten α-Naphthylamin-derivaten im Umfang der Formel I zählen diejenigen Verbindungen mit folgenden Substituententypen oder -kombinationen:

Bei $R_2$: $NO_2$, $NH_2$,
$R_3$: Wasserstoff, Methoxy, Methyl, Halogen,
$R_6$: Wasserstoff, Methyl, Halogen,
R: Methyl, Wasserstoff,
W: $COOR_8$,
$R_8$: $C_1$—$C_3$-Alkyl,
B: 2-Furyl, 2-Tetrahydrofuryl, $CH_2O(C_1$—$C_3$-Alkyl), 1H-1,2,4-Triazolylmethyl, $CH_2SO_2CH_3$, $CH_2OSO_2CH_3$, $CH_2OCH_2CH{=}CH_2$, $CH_2OCH_2C{\equiv}CH$, $CH_2OSO_2NHCH_3$.

Eine bevorzugte Gruppe von Mikrobiziden besteht aus α-Naphthylamin-derivaten der Formel I mit folgenden Substituenten-Kombinationen:

Bei $R_2$: $NO_2$, $NH_2$,
$R_3$: Wasserstoff, Methyl, Methoxy, Chlor, Brom,
$R_6$: Wasserstoff, Methyl, Chlor, Brom,
R: Methyl,
W: $COOR_8$,
$R_8$: Methyl, iso-Propyl,
B: 2-Furyl, 2-Tetrahydrofuryl, $CH_2O(C_1$—$C_3$-Alkyl), $CH_2CH_2OCH_3$, 1H-1,2,4-Triazolylmethyl, $CH_2$—$SO_2CH_3$, $CH_2OSO_2CH_3$, $CH_2OCH_2CH{=}CH_2$, $CH_2OCH_2C{\equiv}CH$, $CH_2OSO_2NHCH_3$.

Eine weitere bevorzugte Gruppe von α-Naphthylamin-dervaten der Formel I wird durch folgende Substituentenkombination charakterisiert:

Bei $R_2$: $NO_2$, $NH_2$,
$R_3$: Wasserstoff, Methyl,
$R_6$: Wasserstoff, Methyl,
R: Methyl,
W: $COOR_8$
$R_8$: Methyl, iso-Propyl,
B: 2-Furyl, 2-Tetrahydrofuryl, $CH_2O(C_1—C_3\text{-Alkyl})$, $CH_2CH_2OCH_3$, 1H-1,2,4-Triazolylmethyl, $CH_2—SO_2CH_3$, $CH_2OSO_2CH_3$, $CH_2OCH_2CH{=}CH_2$, $CH_2OCH_2C{\equiv}CH$, $CH_2OSO_2NHCH_3$.

Eine Gruppe von besonders bevorzugten Mikrobiziden besteht aus Verbindungen der Formel I, worin Ar für die Gruppe

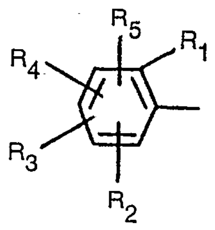

steht;

$R_1$ $C_1—C_3$-Alkyl, $C_1—C_3$-Alkoxy oder Halogen bedeutet,
$R_2$ für $NO_2$ oder $NH_2$ steht,
$R_3$ Wasserstoff, $C_1—C_3$-Alkyl, $C_1—C_3$-Alkoxy oder Halogen,
$R_4$ Wasserstoff oder $C_1—C_3$-Alkyl,
$R_5$ Wasserstoff oder $C_1—C_3$-Alkyl bedeuten, mit der Massgabe, dass stets beide ortho-Positionen im Anilinteil des Molëkuls substituiert sind,
R Wasserstoff oder Methyl bedeutet,
W für $COOR_8$ steht,
$R_8$ für $C_1—C_3$-Alkyl steht,
B 2-Furyl, 2-Tetrahydrofuryl, β-($C_1—C_2$-Alkoxy)ethyl oder dei Gruppe $CH_2Z$ bedeutet, wobei Z für 1H-1,2,4-Triazolyl, Methylsulfonyl, X—$R_{13}$ oder $OSO_2—R_{14}$ steht, worin X Sauerstoff oder Schwefel bedeutet, $R_{13}$ für eine Alkyl-, Alkenyl, oder Alkinylgruppe mit maximal 4 Kohlenstoffatomen steht und $R_{14}$ $C_1—C_3$-Alkyl oder NH($C_1—C_3$-Alkyl) bedeutet.

Eine weitere besonders bevorzugte Gruppe von Mikrobiziden besteht aus Verbindungen der Formel I, worin Ar für die Gruppe

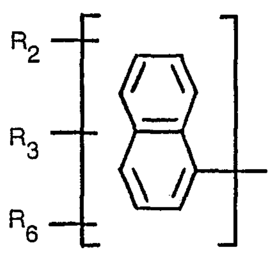

steht,

$R_2$ für $NO_2$ oder $NH_2$ steht,
$R_3$ Wasserstoff, $C_1—C_3$-Alkyl, $C_1—C_3$-Alkoxy oder Halogen,
$R_6$ Wasserstoff, $C_1—C_3$-Alkyl oder Halogen bedeutet, mit der Massgabe, dass stets die β-Position im Molekül substituiert ist;
R Wasserstoff oder Methyl bedeutet,
W für $COOR_8$ steht,
$R_8$ für $C_1—C_3$-Alkyl steht,
B 2-Furyl, 2-Tetrahydrofuryl, β-($C_1—C_2$-Alkoxy)ethyl oder dei Gruppe $CH_2Z$ bedeutet, wobei Z für 1H-1,2,4-Triazolyl, Sulfonylmethyl, X—$R_{13}$ oder $OSO_2—R_{14}$ steht, worin X Sauerstoff oder Schwefel bedeutet, $R_{13}$ für eine Alkyl-, Alkenyl- oder Alkinylgruppe mit maximal 4 Kohlenstoffatomen steht und $R_{14}$ $C_1—C_3$-Alkyl oder NH($C_1—C_3$-Alkyl) bedeutet.

Folgende Einzelverbindungen werden infolge ihrer ausgeprägten Wirkung gegen phytopathogene Mikroorganismen besonders bevorzugt:

N-(1'-Methoxycarbonyl-ethyl)-N-methoxyacetyl-2-methyl-6-aminoanilin.
N-(1'-Methoxycarbonyl-ethyl)-N-methoxyacetyl-2-methyl-6-nitroanilin.
N-(1'-Methoxycarbonyl-ethyl)-N-methoxyacetyl-2,6-dimethyl-3-nitroanilin.
N-(1'-Methoxycarbonyl-ethyl)-N-ethoxyacetyl-2,6-dimethyl-3-nitroanilin.
N-(1'-Isopropyloxycarbonyl-ethyl)-N-methoxyacetyl-2,6-dimethyl-3-nitroanilin.

N-(1'-Methoxycarbonyl-ethyl)-N-methoxyacetyl-2,6-dimethyl-3-aminoanilin.
N-(1'-Methoxycarbonyl-ethyl)-N-methoxyacetyl-2,3-dimethyl-6-aminoanilin.
N-(1'-Methoxycarbonyl-ethyl)-N-methoxyacetyl-2,3-dimethyl-6-nitroanilin.
N-(1'-Methoxycarbonyl-ethyl)-N-(2-furyl)-2-methyl-6-nitroanilin.
N-(1'-Methoxycarbonyl-ethyl)-N-(2-tetrahydrofuryl)-2-methyl-6-nitroanilin.
1N-(1'-Methoxycarbonyl-ethyl)-1N-methoxyacetyl-1,2-diamino-naphthalin.
N-(1'-Methoxycarbonyl-ethyl)-N-methoxyacetyl-amino-2-nitro-naphthalin.
N-(1'-Methoxycarbonyl-ethyl)-N-methoxyacetyl-amino-2-methyl-3-nitro-naphthalin.
N-(1'-Methoxycarbonyl-ethyl)-N-ethoxyacetyl-amino-2-ethyl-3-nitro-naphthalin.
N-(1'-Isopropyloxycarbonyl-ethyl)-N-methoxyacetyl-amino-2-methyl-3-nitro-naphthalin.
1N-(1'-Methoxycarbonyl-ethyl)-1N-methoxyacetyl-2-methyl-1,3-diamino-naphthalin.
1N-(1'-Methoxycarbonyl-ethyl)-1N-methoxyacetyl-3-methyl-1,2-diamino-naphthalin.
N-(1'-Methoxycarbonyl-ethyl)-N-methoxyacetyl-amino-3-methyl-2-nitro-naphthalin.
N-(1'-Methoxycarbonyl-ethyl)-N-(2-furyl)-amino-2-nitro-naphthalin.
N-(1'-Methoxycarbonyl-ethyl)-N-(2-tetrahydrofuryl)-amino-2-nitro-naphthalin.

Die Verbindungen der Formel I können, wie nachfolgend unter A bis J aufgeführt, je nach Art der Substitution, nach einer ganzen Reihe von Methoden hergestellt werden. In den Formeln Ia, Ib, Ia', Ib', II bis XVIII haben die Substituenten $R_1$ bis $R_{16}$, X, B, W und Ar die unter Formel I angegebenen Bedeutungen. Y steht für eine der überlichen Abgangsgruppen, wir beispielswiese für Benzolsulfonyloxy, p-Brombenzolsulfonyloxy, p-Tosyloxy, Trifluoroacetyloxy, Niederalkylsulfonyloxy wie Mesyloxy oder insbesondere für Halogen wie Fluor, Chlor, Brom oder Jod, bevorzugt Chlor oder Brom. Hal steht als Symbol für die Halogene Fluor, Chlor, Brom oder Jod, insbesondere für Chlor oder Brom.

Bei dem erfindungsgemässen Herstellungsverfahren kann man im einzelnen folgendemassen vorgehen:

A. Wirkstoffe der Formel I können durch Acylierung einer Verbindung der Formel II

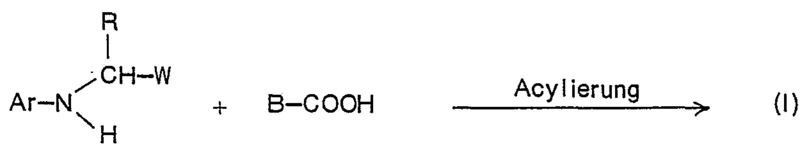

(I)

(II) (III)

mit einer Carbonsäure der Formel III oder einem ihrer reaktionsfähigen Derivate hergestellt werden, oder

B. durch Reaktion einer Verbindung der Formel IV

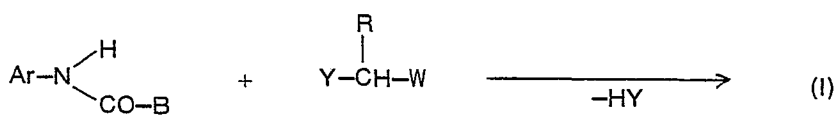

(I)

(IV) (V)

mit einer Verbindung der Formel V, worin Y für eine übliche Abgangsgruppe steht, oder

C. durch Nitrierung einer Verbindung der Formel VI bzw. VI'

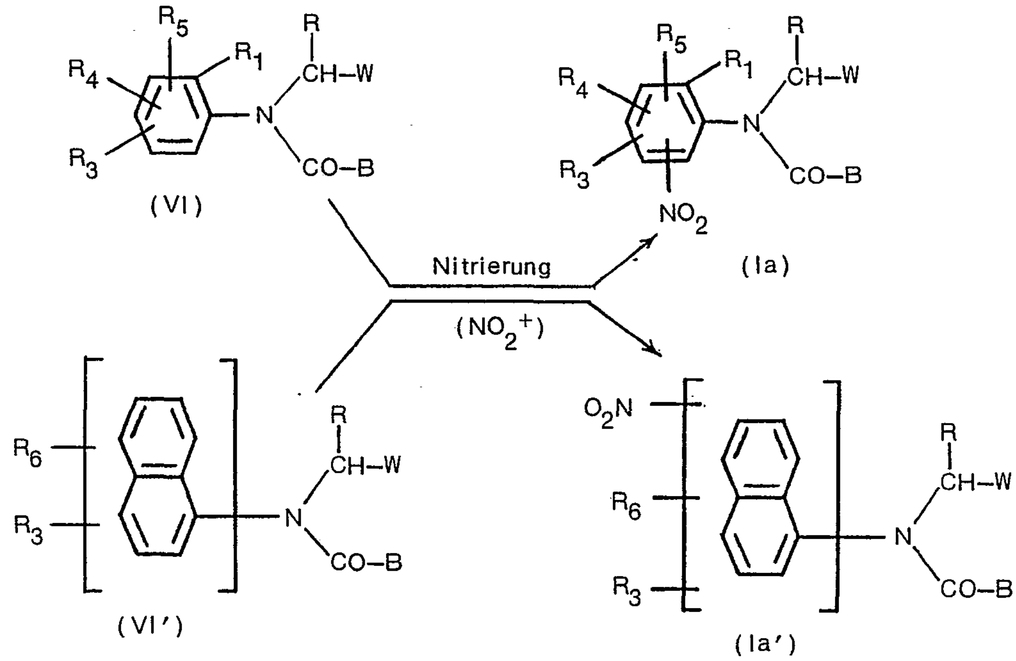


(VI) (Ia) (VI') (Ia')

mit einem reaktionsfähigen Nitrierungsreagenz zu einer Verbindung der Formel I, die hier und im folgenden die Bezeichnung Ia bzw. Ia' trägt, oder

D. durch Hydrierung einer Verbindung aus der Untergruppe Ia bzw. Ia'

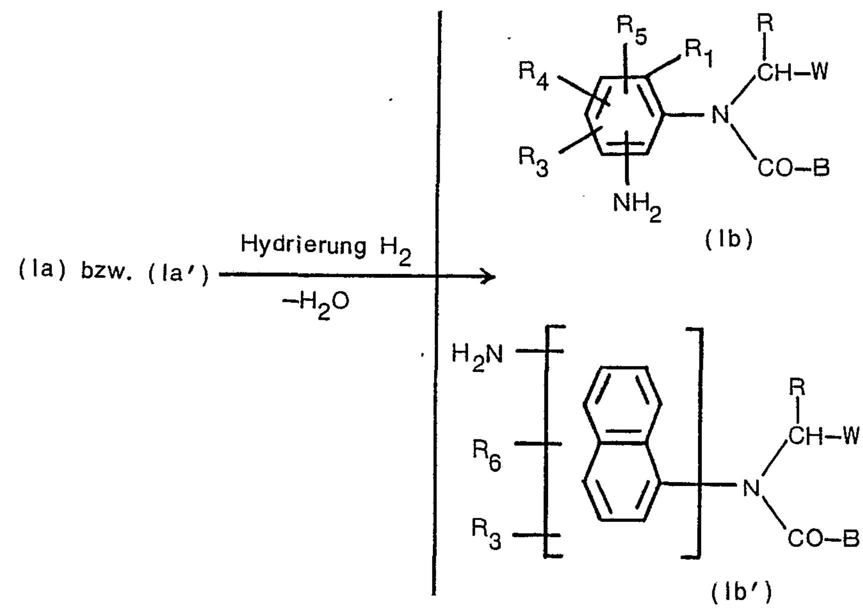


(Ib) (Ib')

mit einem üblichen Hydrierungsmittel zu einer Verbindung der Formel I, die hier und im folgenden die Bezeichnung Ib bzw. Ib' trägt, oder

E. in den Fällen, in denen bei den Wirkstoffen der Formel I der Substituent B für die Gruppe $CH_2XR_{13}$ oder eine 1H-1,2,4-Triazolylmethylgruppe steht, kann die Herstellung auch durch Reaktion einer Verbindung der Formel VII

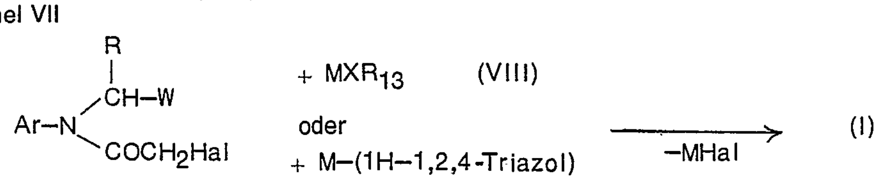


(VII) (IX)

mit einer Verbindung der Formel VIII oder IX erfolgen, oder

F. in den Fällen, in denen bei den Wirkstoffen der Formel I der Substituent B für β-($C_1$—$C_2$-Alkoxy)ethyl steht, kann die Herstellung auch durch Reaktion einer Verbindung der Formel X oder XI

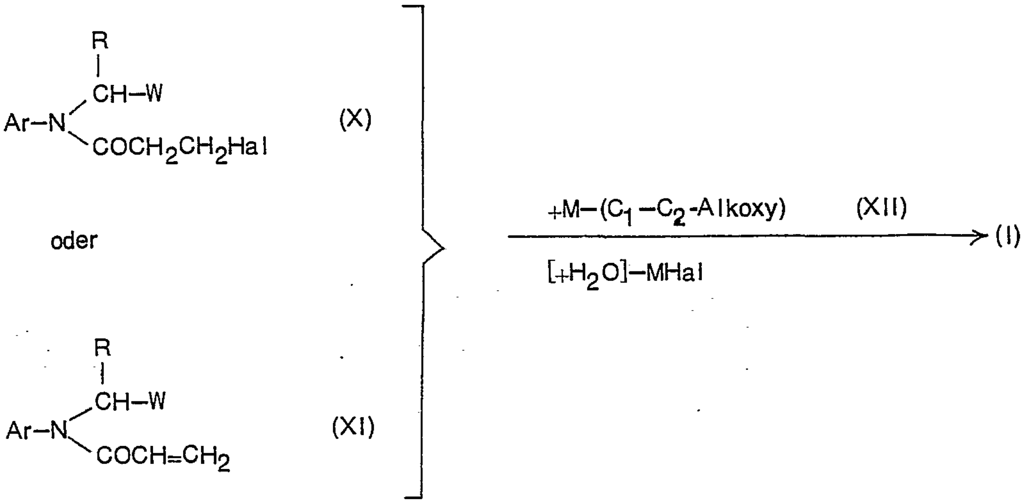


(X) oder (XI)

mit einer Verbindung der Formel XII, wobei die Umsetzung von XI mit XII zunächst zu einem Additionsprodukt führt, das durch Hydrolyse in das Endprodukt der Formel I überführt werden kann, oder

G. die Verbindungen der Formel I lassen sich auch durch Veresterungs- oder Umesterungsreaktion einer Verbindung der Formel XIII

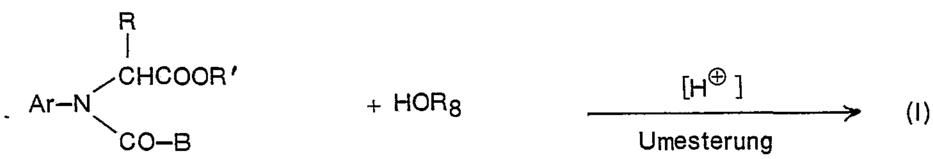


(XIII) (XIV)

in Gegenwart einer starken Säure mit einer Verbindung der Formel XIV, wobei R′ für Wasserstoff oder einen Kohlenwasserstoffrest steht, oder

H. in den Fällen, in denen bei den Wirkstoffen der Formel I der Substituent Z für Methylsulfonyl steht, kann die Herstellung auch durch Reaktion einer Verbindung der Formel VII mit einer Verbindung der Formel XV

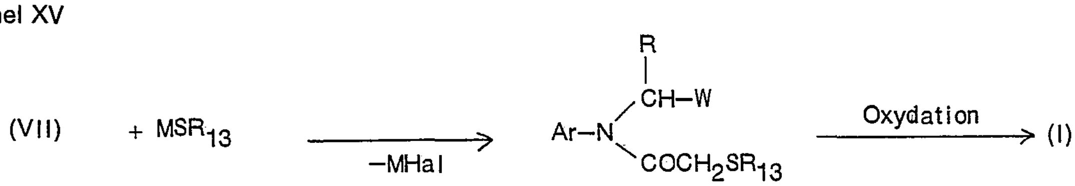


(XV) (XVI) = (Ic)

zu einem Zwischenprodukt der Formel XVI und anschliessender Oxydation von XVI zu I erfolgen, wobei die Verbindungen der Formel XVI als Untergruppe Ic bezeichnet werden sollen, der

J. in den Fällen, in denen bei den Wirkstoffen der Formel I der Substituent B für $CH_2OSO_2R_{14}$ steht, so kann die Herstellung auch durch Reaktion einer Verbindung der Formel XVII

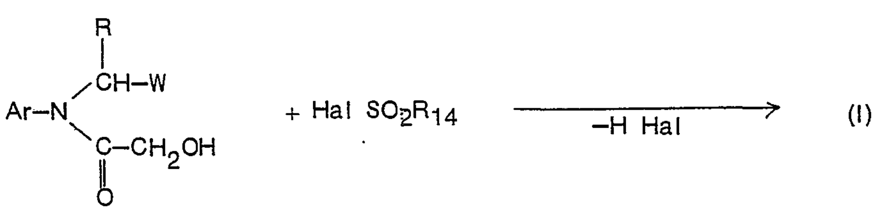


(XVII) (XVIII)

mit einer Verbindung der Formel XVIII, wobei in der Varianten A bis J in den Formeln Ia, Ib, Ia′, Ib′, II bis

XVIII die Substituenten $R_1$ bis $R_{16}$, X, W, B und Ar die unter Formel I angegebenen Bedeutungen haben, M für Wasserstoff oder ein Metallkation steht und Hal ein Halogenatom bedeutet.

Die Ausgangssubstanzen sind grösstenteils bekannt oder können nach an sich bekannten Methoden hergestellt werden. Die Ausgangsverbindungen der Formeln II, IV, VII, X, XI und XII lassen sich beispielsweise analog zu den hier beschriebenen Herstellungsvarianten C und D durch Nitrierung der zugrundeliegenden Anilinderivate und, sofern gewünscht, durch deren anschliessende Hydrierung herstellen.

Für die einzelnen Herstellungsvarianten sind folgende Reaktionsbedingungen vorteilhaft:

*Variante A:* Es kann hierbei vorzugsweise ein reaktionsfähiges Derivat einer Verbindung der Formel III, wie z.B. das Säurehalogenid, Säureanhydrid oder der Ester eingesetzt werden. Besonders geeignet ist das Säurechlorid und -bromid.

Die Reaktionstemperaturen liegen zwischen 0° und 180°C, vorzungsweise 0° und 150°C bzw. am Siedpunkt des Lösungsmittels bzw. Lösungsmittelgemisches. In manchen Fällen ist die Verwendung von säurebindenden Mitteln bzw. Kondensationsmitteln vorteilhaft. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Carbonate und Hydrogencarbonate von Alkali- und Erdalkalimetallen sowie Alkaliacetate.

Entstehender Halogenwasserstoff kann in manchen Fällen auch mittels Durchleiten von Inertgas, z.B. Stickstoff aus dem Reaktionsgemisch vertrieben werden.

Die N-Acylierung kann in Anwesenheit von reaktionsinerten Lösungs- oder Verdünnungsmitteln durchgeführt werden. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Pripionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann das Acylierungsmittel selbst als Lösungsmittel dienen.

Bei der Acylierung kann die Gegenwart eines Reaktionskatalysators wie Dimethylformamid von Vorteil sein.

*Variante B:* Hierbei steht der Substituent Y in Formel V für eine der üblichen Abgangsgruppen, beispeilsweise für Benzolsulfonyloxy, p-Brombenzolsulfonyloxy, p-Tosyloxy, Trifluoracetyloxy, Niederalkylsulfonyloxy wie Mesyloxy oder insbesondere für ein Halogen wie Fluor, Chlor, Brom oder Jod, bevorzugt für Chlor oder Brom. Bei dieser Variante führt man zweckmässigerweise die Ausgangsverbindung der Formel IV zuerst mit Butyl-Lithium, Natriumhydrid oder mit Alkalicarbonat (z.B. Soda) in das entsprechende Alkalisalz über oder aber man arbeitet in Gegenwart eines säurebindenden Mittels und bei Temperaturen analog zu Variante A, vorzugsweise unter Zusatz katalytischer Mengen Alkalijodid.

*Variante C:* Als Nitrierungsmittel kommen bei dieser Herstellungsvariante alle Substanzen in Frage, die das eigentliche Nitrierungsreagens, nämlich das Nitroniumion $NO_2^{\oplus}$ freizusetzen vermögen. So können ausser Salpetersäure und Nitriersäure ($1HNO_3$:2 $H_2SO_4$) auch organische Nitrate wie Acylnitrate (z.B. Acetylnitrat) in ihrer praktikablen Anwendungsform (z.B. Gemisch aus Eisessig/Acetanhydrid/ 65—100% ige $HNO_3$) als Nitrierungsmittel eingesetzt werden. Zur Erhöhung der Reaktionsgeschwindigkeit kann das Reaktionsgemisch auch erwärmt werden, wobei die Temperatur jedoch nicht so stark erhäht sein sollte, dass nitrose Gase gebildet werden.

*Variante D:* Bei der Hydrierung der Nitrogruppe können ausser den üblichen reaktionsinerten organischen Solventien insbesondere niederatomige Alkanole wie Methanol, Ethanol, iso-Propanol eingesetzt werden. Geeignet sind auch aliphatische und aromatische Kohlenwasserstoffe wie Benzol, Toluol, Xylole, Petrolether; Ether und etherartige Verbindungen wie Dialkylether (Diisopropylether, tert.-Butylmethylether usw.), Anisol, Dioxan, Tetrahydrofuran; Dimethylsulfoxid oder Ester wie Ethylacetat, Propylacetat usw.

In den Fällen, in denen das Ausgangsprodukt der Formel Ia flüssig ist, kann auch ohne Lösungsmittel hydriert werden.

Darüberhinaus ist auch eine Hydrierung in Mehrphasensystemen wie z.B. in Toluol/$H_2O$ oder Xylol/$H_2O$ möglich, daneban auch in Eisessig.

Die Hydrierung kann auf sehr unterschiedliche Arten erfolgen. Neben den üblichen Methoden mit nascierendem Wasserstoff (unedle Metalle in saurer Lösung), auf elektrolytischem Wege oder mit Reduktionsmitteln wie Alkalihydriden oder Lithiumaluminiumhydrid, eignen sich insbesondere die katalytische Hydrierungsmethoden.

Als Hydrierkatalysatoren kommen hierbei *Schwarzkatalysatoren,* (feinst verteilte Metallniederschläge, z.B. reduktiv abgeschiedene (Edel)metalle aus Lösungen ihrer Salzen) wie Platin, Palladium oder Nickel in

Frage; *Edelmetalloxide* wie Platin(IV)oxid; *Skelettkatalysatoren,* bestehend aus Metallschwämmen binärer Legierungen z.B. RANEY-Nickel. *Trägerkatalysatoren* bestehend aus Schwarzkatalysatoren, die an der Oberfläche einer Trägersubstanz haften. Als Träger dienen im allgemeinen Substanzen wie Kohle, Siliciumdioxid, Aluminiumoxid, Sulfate und Carbonate der Erdalkalien. *Oxid-* und *Sulfidkatalysatoren* wie Kuperchromit (Kupferchromoxid), Zinkchromit, Molybdänsulfid, Wolframsulfid u.a.

Die Hydrierung wird bevorzugt unter Normaldruck oder bis zu Drucken von 20 bar durchgeführt, dabei liegen die Reaktionstemperaturen zwischen 0° und 150°C. Werden statt der genannten Katalysatoren andere Metalle z.B. Eisen, Chrom, Kobalt oder Kupfer eingesetzt, so erfordert dies in der Regel höhere Reaktionstemperaturen und höhere Drucke.

*Variante E:* Wenn M = Wasserstoff bedeutet, ist die Verwendung eines salzbildenden Mittels angebracht, wie z.B. eines Oxids, Hydroxids, Hydrids von Alkali- oder Erdalkalimetallen.

*Variante F:* Bei der Reaktion (X) + (XII) → (I) wird analog zu Variante E gearbeitet.

Die Reaktion (XI) + (XII) → (I) wird mit dem Alkohol bzw. dem Alkoholat (XII) (M = Metallatom) im Sinne einer Michaeladdition durchgeführt.

*Variante G:* Diese Reaktion wird durch Säuren und Basen katalytisch beeinflusst. Um das Gleichgewicht möglichst nach der gewünschten Richtung zu verschieben, wird mit einem Ueberschuss an $HOR_8$ gearbeitet. Die Bedeutungen von R' und $R_8$ sind verschieden. R' steht für Wasserstoff oder einem Kohlenwasserstoffrest, insbesondere jedoch für Wasserstoff oder Niederalkyl wie Methyl oder Ethyl.

*Variante H:* Die Reaktion (VII) + (XV) → (XVI) erfolgt analog zu Variante. E. Die Oxydation von (XVI) zu (I) kann mit Persäuren wie $H_2O_2$, Perbenzoesäure, Metachlorperbenzoesäure, $HJO_4$ oder auch Kaliumpermanganat erfolgen. Als Oxydationsmittel werden Persäuren, insbesondere $H_2O_2$ bevorzugt.

*Variante J:* Die Reaktion wird analog zu Variante E durchgeführt, wobei die Verbindung der Formel XVII zuerst auch in das entsprechende Alkoholat überführt und anschliessend mit Verbindung XVIII umgesetzt werden. kann.

Bei allen Varianten kann sich die Verwendung wasserfreier und/oder schutzgasgesättigter organischer Lösungsmittel vorteilhaft auswirken. Auch das Arbeiten unter Schutzgasatmosphäre z.B. $N_2$ kann den Reaktionsverlauf günstig beeinflussen.

Sofern bei den Herstellungsvarianten A bis J keine spezifischen Lösungsmittel genannt sind, können grundsätzlich alle Lösungsmittel verwendet werden, die sich gegenüber den Reaktionspartnern inert verhalten.

Beispiele solcher Lösungsmittel sind:

Aliphatische und aromatische Kohlenwasserstoffe wie Benzol, Toluol, Xylole, Petrolether, halogenierte Kohlenwasserstoffe wie Chlorbenzol, Methylenchlorid, Ethylenchlorid, Chloroform; Ether und etherartige Verbindungen wie Dialkylether, Dioxan, Tetrahydrofuran; Nitrile wie Acetonitrile; N,N-dialkylierte Amide wie Dimethylformamide; Dimethylsulfoxid, Ketone wie Methylethylketon und Gemische solcher Lösungsmittel untereinander. Bei dem beschriebenen Verfahren ist es in der Regel nicht notwendig, die Zwischenprodukte zu isolieren. Das Verfahren kann auch in einer sogenannten Eintopfreaktion erfolgen

Das Herstellungsverfahren ist einschliesslich aller Teilschritte (Varianten A bis J) ein Teil der Erfindung.

In den Fällen, in denen bei den Verbindungen der Formel I der Substituent R für Methyl steht, besitzen diese Substanzen nachbarständig zu W ein asymmetrische Kohlenstoffatom in der Seitenkette und können auf übliche Art in optische Antipoden gespalten werden; so z.B. durch fraktionierte Kristallisation der Salze von II mit einer optisch aktiven Säure und Weiterreaktion der optisch reinen Verbindungen von II zu I oder z.B. durch fraktionierte Kristallisation der freien Säure XIII (R'=H) mit einer optisch aktiven Base und Weiterreaktion der optisch reinen Verbindungen von XIII zu I. Die optischen Antipoden von I besitzen unterschiedliche mikrobizide Wirkungen.

Je nach Substitution können weitere asymmetrische Kohlenstoffatome im Molekül vorhanden sein.

Unabhängig von der gennanten optischen Isomerie wird eine Atropisomerie um die

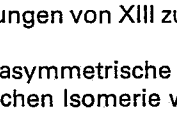

Achse beobachtet, wenn der Phenylring unsymmetrisch zu dieser Achse substituiert ist.

Sofern keine gezielte Synthese zur Isolierung reiner Isomeren durchgeführt wird, fällt normalerweise ein Produkt der Formel I als Gemisch dieser möglichen Isomeren an.

Es wurde überraschend gefunden, dass Verbindungen der Formel I ein für praktische Bedürfnisse sehr günstiges Mirkobizid-Spektrum aufweisen. Sie lassen sich beispielsweise zum Schutz von Kulturpflanzen verwenden.

Das Haupteinsatzgebiet von Verbindungen der Formel I liegt in der Bekämpfung von schädlichen Mikroorganismen, vor allem von phytopathogenen Pilzen. So besitzen die Verbindungen der Formel I eine für praktische Bedürfnisse sehr günstige kurative, präventive und systemische Wirkung zum Schutz von Kulturpflanzen ohne diese durch unerwünschte Nebenwirkungen zu beeinflussen. Kulturpflanzen seien im

Rahmen vorliegender Erfindung beispielsweise: Getreide: (Weizen, Gerste, Roggen, Hafer, Reis); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen- und Naturkautschukgewächse sowie Zierpflanzen.

Mit den Wirkstoffen der Formel I können an Pflanzen oder an Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Mikroorganismen verschont bleiben. Die Wirkstoffe sind gegen die den folgenden Klassen angehörenden phytopathogenen Pilze wirksam: Gegen die der Klasse der Phycomycetes angehörenden Oomycetes wie Peronosporales (Phytophthora, Pythium, Plasmopara), sowie insbesondere gegen Ascomycetes wie Erysiphe- und Venturia-Erreger.

Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden aufgtretende phytopathogene Pilze eingesetzt werden.

Die Erfindung betrifft somit ferner die Verwendung der Verbindungen der Formel I zur Bekämpfung phytopathogener Mikroorganismen bzw. zur präventiven Verhütung eines Befalls an Pflanzen.

Wirkstoffe der Formel I können gleichzeitig oder nacheinander mit weiteren Wirkstoffen auf die zu behandelnde Fläche oder Pflanze gegeben werden. Diese Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematozide, Molluskizide oder Gemische mehrerer dieser Präparate sein, zusammen mit gegebenenfalls weiteren in der Fromulierungstechnik üblichen Trägerstoffen, Tensiden oder anderen applikationsfördernden Zusätzen.

Geeignete Träger und Zusätze können fest oder flüssig sein und entsprechen den in der Fromulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Unter Tensiden sollen hierbei grenzflächen- oder oberflächen-aktive Verbindungen verstanden werden, die meistens in einer Flüssigkeit gelöst oder dispergiert sind und bevorzugt an Grenzflächen adsorbiert werden. Ein Tensidmolekül besitzt mindestens eine Gruppe, die eine Affinität zu Substanzen starker Polarität aufweist — wodurch im allgemeinen die Löslichkeit in Wasser verursacht wird — und mindestens eine weitere Gruppe mit geringer Affinität zu Wasser. Tenside sind somit Moleküle mit einem lipophilen = hydrophoben, d.h. wasserabweiseden oder fettfreundlichen Molekülteil, meist einem Kohlenwasserstoffrest mit Alkyl- und/oder Arylkomponenten und ausserdem mit einem hydrophilen = lipophoben, d.h. wasserfreundlichen und fettabweisenden Molekülteil, z.B. einem Perfluoralkylrest. Die in der Praxis gebrauchten Produkte sind meistens Mischungen solcher Verbindungen. Tenside ermöglichen nicht nur eine feine Verteiluing der Aktivsubstanz in einem flüssigen z.B. wässrigen Medium, sondern auch eine erhöhte Benetzbarkeit der Pflanzen, dies führt zu einer Reduktion des Wirkstoffanteils im gebrauchsfähigen Mittel und damit zu einer geringeren Umweltbelastung.

Der Gehalt an Wirkstoff in handelsfähigen Mitteln liegt zwischen 0,01 bis 90 Gewichtsprozent, der Gehalt an Zuschlagstoffen 10 bis 99,99 Gewichtsprozent, wobei sich im allgemeinen unter den Zuschlagstoffen 0 und 33 Gewichtsprozent eines Tensides befinden.

Die Erfindung umfasst auch Mittel, die als mindestens eine Aktivsubstanz eine Verbindung der Formel I enthalten, sowie die Verwendung der Mittel zur Bekämpfung und/oder Verhütung eines Befalls von Mikroorganismen. Darüberhinaus schliesst die vorliegende Erfindung auch die Herstellung der Mittel ein, die gekennzeichnet ist durch das innige Vermischen der Akitivsubstanz mit einem oder mehreren hierin beschriebenen Substanzen bzw. Substanzgruppen. Eingeschlossen ist auch ein Verfahren zur Bekämpfung von Mikroorganismen, das sich durch Applikation der Verbindungen der Formel I bzw. der neuen Mittel auszeichnet.

Die nachfolgenden Beispiele dienen zur nähreren Erläuterung der Erfindung. Die Temperaturen sind in Celsiusgraden angegeben. Prozente und Teile beziehen sich auf Gewicht. Sofern nicht anders vermerkt, ist bei der Nunnung eines Wirkstoffs der Formel I stets das Isomeren-Gemisch gemeint. RT bedeutet Raumtemperatur, h steht für Stunde, min für Minute, DMSO für Dimethylsulfoxid, THF für Tetrahydrofuran, DMF für Dimethylformamid.

Beispiel 1

Herstellung von

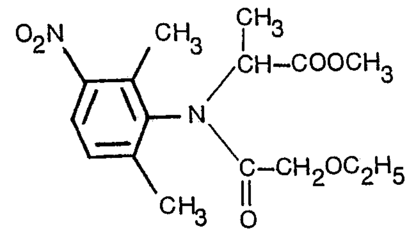

(31)

N-(1'-Methoxycarbonyl-ethyl)-N-ethoxyacetyl-2,6-dimethyl-3-nitroanilin

Zu 3 g 55%igem Natriumhydrid in 70 m wasserfreiem und mit Stickstoff gesättigtem Tetrahydrofuran werden bei Raumtemperatur unter Rühren und gleichzeitigem Durchleiten von Stickstoff 16 g N-Ethoxyacetyl-2,6-dimethyl-3-nitroanilin in 30 ml des gleichen Lösungsmittels zugetropft. Nach Abklingen der schwach exothermen Reaktion wird das Gemisch auf 55°C erwärmt und mit 12,7 g 2-Brompropionsäuremethylester in 20 ml Tetrahydrofuran versetzt, wobei die Temperatur etwa auf 60°C ansteigt. Nach 12 stündigem Rühren bei 55°C wird das Gemisch auf Raumtemperatur abgekühlt auf 100 ml Wasser gegossen und zweimal mit je 100 ml Ethylacetat extrahiert. Die vereinigten Extrakte werden mit Wasser gewaschen über Natriumsulfat getrocknet und überschüssiges Lösungsmittel im Vacuum entfernt. Das ölige Rohprodukt wird säulenchromatographisch über Kieselgel gereinigt. Als Eluierungsmittel dient ein Chloroform-Diethylether-Gemisch (1:1). Die Fraktionen Nr. 5 bis 30 werden vereinigt und das Eluierungsmittel im Vacuum entfernt. Verbindung Nr. 31 ist ein viskoses Oel mit Brechungsindex $n_D^{24} = 1.5325$.

Beispiel 2

Herstellung von

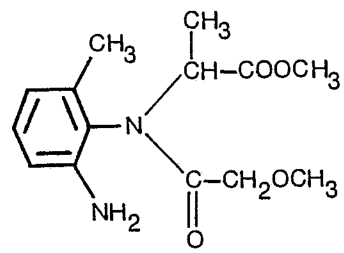

(2)

N-(1'-Methoxycarbonyl-ethyl)-N-methoxyacetyl-2-amino-6-methylanilin

257,4 g N-(1'-Methoxycarbonyl-ethyl)-N-methoxyacetyl-2-methyl-6-nitro-anilin werden in 2,6 Liter wasserfreiem Methanol gelöst und 26 Stunden bie 20—25°C und unter Normaldruck in Gegenwart von 26 g RANEY-Nickel hydriert. ($H_2$—Aufnahme: 56,2 Liter = ca. 100% der Theorie). Anschliessend wird der Katalysator abfiltriert, das Filtrat eingeengt und der ölige Rückstand durch Zugabe von Diethylether zur Kristallisation gebracht. Ausbeute 170,8 g der Verbindung Nr. 2, Fp. 86—88°C.

Beispiel 3

Herstellung von

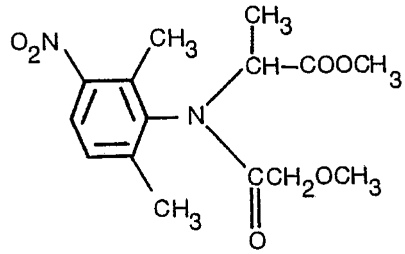

(28)

N-(1'-Methoxycarbonyl-ethyl)-N-methoxyacetyl-2,6-dimethyl-3-nitroanilin

Zu 32,0 g N-(1'-Methoxycarbonyl-ethyl)-2,6-dimethyl-3-nitroanilin in 300 ml wasserfreiem Toluol werden bei RT 15,2 g Methoxyessigsäurechlorid zugetropft. Nach Abklingen der schwach exothermen Reaktion wird die Reaktionslösung 20 h bei RT nachgerührt. Nach Entfernen des Lösungsmittels wird der dunkelrote, ölige Rückstand am Hochvakuum destilliert. Dabei destilliert die Verbindung Nr. 28 bei einer Temperatur von 170—174° und einem Druck von 0,07 mbar als hellgelbes Oel.

Beispiel 4

Herstellung von

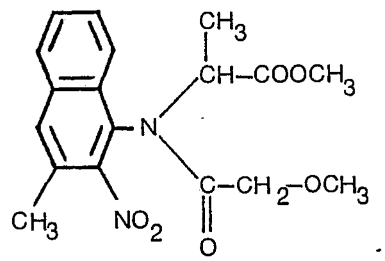

(402)

N-(1'-Methoxycarbonyl-ethyl)-N-methoxyacetyl-amino-3-methyl-nitro-naphthalin

Zu 3 g 55%igem Natriumhydrid in 70 ml wasserfreiem und mit Stickstoff gesättigtem Tetrahydrofuran werden bei Raumtemperatur unter Rühren und gleichzeitigem Durchleiten von Stickstoff 14 g N-Methoxyacetyl-amino-3-methyl-2-nitro-naphthalin in 30 ml des gleichen Lösungsmittels zugetropft. Nach Abklingen der schwach exothermen Reaktion wird das Gemisch auf 55°C erwärmt und mit 9,5 g 2-Bromopropionsäuremethylester in 20 ml Tetrahydrofuran versetzt, wobei die Temperatur etwa auf 60°C ansteigt. Nach 12 stündigem Rühren bei 55°C wird das Gemisch auf Raumtemperatur abgekühlt, auf 100 ml Wasser gegossen und zweimal mit je 100 ml Ethylacetat extrahiert. Die vereinigten Extrakte werden mit Wasser gewaschen, über Natriumsulfat getrocknet und überschüssiges Lösungsmittel im Vakuum entfernt. Das ölige Rohprodukt wird säulenchromatographisch über Kieselgel gereinigt. Als Eluierungsmittel dient ein Chloroform-Diethylether-Gemisch (1:1). Dir Fraktionen Nr. 5 bis 30 werden vereinigt und das Eluierungsmittel im Vakuum entfernt. Verbindung Nr. 402 fällt als viskoses Harz aus.

Beispiel 5

Herstellung von

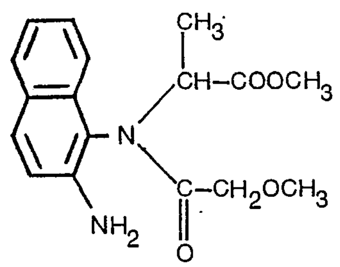

(701)

1N-(1'-Methoxycarbonyl-ethyl)-1N-methoxycarbonyl-1,2-diamino-naphthalin

3,0 g N-(1'-Methoxycarbonyl-ethyl)-N-methoxyacetyl-amino-2-nitronaphthalin werden in 100 ml Dioxan gelöst und 26 Stunden bei 20—25°C und unter Normaldruck in Gegenwart von 4 g Raney-Nickel hydriert ($H_2$-Aufnahme: 590 ml = ca. 100% der Theorie). Anschliessend wird der Katalysator abfiltriert, das Filtrat eingeengt und der Rückstand mit wenig Petrolether digeriert. Ausbeute 2 g. Smp. 159—162°C.

Beispiel 6

Herstellung von

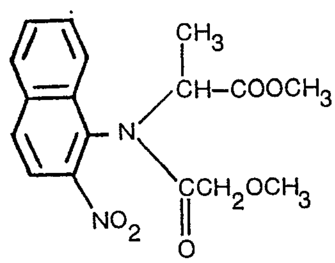

(400)

N-(1'-Methoxycarbonyl-ethyl)-N-methoxyacetyl-2-nitro-naphthyl-amin

Zu 0,8 g 80%igem Natriumhydrid in 70 ml wasserfreiem Tetrahydrofuran werden bei RT unter Rühren 7,2 g N-Methoxyacetyl-2-nitro-naphthylamin in 50 ml des gleichen Lösungsmittels zugetropft. Nach Abklingen der schwach exothermen Reaktion wird die klare Lösung 1 h bei RT ausgerührt und dann mit 4,8 g 2-Brompropionsäuremethylester in 20 ml Tetrahydrofuran versetzt. Nach 20 stündigem Rühren bei 50°C wird die Suspension auf RT abgekühlt, auf 200 ml Wasser gegossen und 2 mal mit je 100 ml Diethylether extrahiert. Die vereinigten Extrakte werden mit Wasser gewaschen, über Natriumsulfat getrocknet

und überschüssiges Lösungsmittel im Vakuum entfernt. Das ölige Rohprodukt wird über eine Kieselgelsäule mittels Chloroform/Diethylether 1:1 chromatographiert. Die Fraktion en Nr. 11 bis 26 werden vereinigt und das Eluierungsmittel im Vakuum entfernt. Die Verbindung Nr. 400 fällt in Form gelber Kristalle vom Smp. 103—107° an.

Auf analoge Weise werden auch die nachfolgenden Verbindung der Formel I hergestellt:

TABELLE 1

Verbindungen der Formel I mit Ar = substituiertes Phenyl, W = $COOR_8$, $R_5$ = H

| Verb. Nr. | $R_1$ | $R_2$ | $R_3$ | $R_4$ | R | $R_8$ | B | Physik. Konst. [°C] |
|---|---|---|---|---|---|---|---|---|
| 1 | $CH_3$ | 6–$NH_2$ | H | H | H | $CH_3$ | $CH_2OCH_3$ | |
| 2 | $CH_3$ | 6–$NH_2$ | H | H | $CH_3$ | $CH_3$ | $CH_2OCH_3$ | Fp. 103–109 |
| 3 | $C_2H_5$ | 6–$NH_2$ | H | H | H | $CH_3$ | $CH_2OCH_3$ | |
| 4 | $CH_3$ | 6–$NH_2$ | H | H | $CH_3$ | $C_2H_5$ | $CH_2OCH_3$ | |
| 5 | $CH_3$ | 6–$NH_2$ | H | H | $CH_3$ | $C_3H_7$-i | $CH_2OCH_3$ | |
| 6 | Cl | 6–$NH_2$ | H | H | $CH_3$ | $CH_3$ | $CH_2OCH_3$ | Fp. 103–105 |
| 7 | $CH_3$ | 6–$NH_2$ | 3–$CH_3$ | H | H | $CH_3$ | $CH_2OCH_3$ | |
| 8 | $CH_3$ | 6–$NH_2$ | 3–$CH_3$ | H | $CH_3$ | $CH_3$ | $CH_2OCH_3$ | Fp. 158–164 |
| 9 | $CH_3$ | 3–$NH_2$ | H | 6–$CH_3$ | H | $CH_3$ | $CH_2OCH_3$ | |
| 10 | $CH_3$ | 3–$NH_2$ | H | 6–$CH_3$ | $CH_3$ | $CH_3$ | $CH_2OCH_3$ | Fp. 117–118,5 |
| 11 | $CH_3$ | 4–$NH_2$ | 3–$CH_3$ | 6–$CH_3$ | $CH_3$ | $CH_3$ | $CH_2OCH_3$ | |
| 12 | $CH_3$ | 6–$NH_2$ | H | H | $CH_3$ | $CH_3$ | 2–Furyl | |
| 13 | $CH_3$ | 6–$NH_2$ | H | H | $CH_3$ | $CH_3$ | 2–Tetrahydrofuryl | |
| 14 | $CH_3$ | 3–$NH_2$ | H | 6–$CH_3$ | $CH_3$ | $CH_3$ | $CH_2SO_2CH_3$ | Fp. 106–110 |
| 15 | $CH_3$ | 6–$NH_2$ | H | H | $CH_3$ | $CH_3$ | $CH_2CH_2OCH_3$ | |
| 16 | $CH_3$ | 6–$NH_2$ | H | H | $CH_3$ | $CH_3$ | 1H–1,2,4-Triazolyl-methyl | |

TABELLE 1 (Fortsetzung)

| Verb. Nr. | $R_1$ | $R_2$ | $R_3$ | $R_4$ | R | $R_8$ | B | Physik. Konst. [°C] |
|---|---|---|---|---|---|---|---|---|
| 17 | $CH_3$ | 6–$NH_2$ | H | H | $CH_3$ | $CH_3$ | $CH_2OCH_2CH{=}CH_2$ | Harz |
| 18 | $CH_3$ | 6–$NH_2$ | H | H | $CH_3$ | $CH_3$ | $CH_2OCH_2C{\equiv}CH$ | Harz |
| 19 | Cl | 6–$NH_2$ | 3–$CH_3$ | 4–$CH_3$ | $CH_3$ | $C_3H_7$-i | 2–Furyl | |
| 20 | $CH_3$ | 4–$NH_2$ | 6–$CH_3$ | H | $CH_3$ | $C_3H_7$-i | $CH_2SO_2CH_3$ | |
| 21 | $OCH_3$ | 3–$NH_2$ | 6–$CH_3$ | H | $CH_3$ | $CH_3$ | 2-Tetrahydrofuryl | |
| 22 | $OCH_3$ | 6–$NH_2$ | H | H | $CH_3$ | $CH_3$ | $CH_2OCH_3$ | |
| 23 | $OCH_3$ | 6–$NH_2$ | H | H | $CH_3$ | $CH_3$ | $CH_2CH_2OCH_3$ | |
| 24 | $OCH_3$ | 6–$NH_2$ | 3–Cl | H | $CH_3$ | $CH_3$ | 2–Furyl | |
| 25 | $OCH_3$ | 3–$NH_2$ | 6–$CH_3$ | 4–$CH_3$ | $CH_3$ | $CH_3$ | $CH_2OCH{=}CH_2$ | |
| 26 | Cl | 3–$NO_2$ | 6–$CH_3$ | H | H | $CH_3$ | –2-Tetrahydrofuryl | |
| 27 | Cl | 3–$NO_2$ | 6–$CH_3$ | H | $CH_3$ | $CH_3$ | $CH_2OCH_3$ | |
| 28 | $CH_3$ | 3–$NO_2$ | 6–$CH_3$ | H | $CH_3$ | $CH_3$ | $CH_2OCH_3$ | Kp. 170–174/0,07 mbar |
| 29 | $CH_3$ | 3–$NO_2$ | 6–$CH_3$ | 4–$CH_3$ | $CH_3$ | $CH_3$ | $CH_2OCH_3$ | Fp. 105–112° |
| 30 | $CH_3$ | 3–$NO_2$ | 6–$CH_3$ | H | H | $CH_3$ | $CH_2OCH_3$ | |
| 31 | $CH_3$ | 3–$NO_2$ | 6–$CH_3$ | H | $CH_3$ | $CH_3$ | $CH_2OC_2H_5$ | Oel; $n_D^{24}$ = 1.5325 |
| 32 | $C_2H_5$ | 3–$NO_2$ | 6–$CH_3$ | H | $CH_3$ | $CH_3$ | $CH_2OC_2H_5$ | |
| 33 | $CH_3$ | 3–$NO_2$ | 6–$CH_3$ | H | $CH_3$ | $C_3H_7$-i | $CH_2OCH_3$ | Oel |

TABELLE 1 (Fortsetzung)

| Verb. Nr. | $R_1$ | $R_2$ | $R_3$ | $R_4$ | R | $R_8$ | B | Physik. Konst. [°C] |
|---|---|---|---|---|---|---|---|---|
| 34 | $CH_3$ | $3-NO_2$ | $6-CH_3$ | H | $CH_3$ | $CH_3$ | $CH_2CH_2OCH_3$ | |
| 35 | $CH_3$ | $3-NO_2$ | $6-CH_3$ | H | $CH_3$ | $CH_3$ | 2-Tetrahydrofuryl | Fp. 97–104 |
| 36 | $CH_3$ | $3-NO_2$ | $6-CH_3$ | $4-CH_3$ | $CH_3$ | $C_3H_7$-i | 2-Furyl | |
| 37 | $CH_3$ | $3-NO_2$ | $6-CH_3$ | H | $CH_3$ | $CH_3$ | $CH_2OSO_2CH_3$ | |
| 38 | $CH_3$ | $3-NO_2$ | $6-CH_3$ | H | $CH_3$ | $CH_3$ | $CH_2SO_2CH_3$ | Fp. 168–172 |
| 39 | $CH_3$ | $3-NO_2$ | $6-CH_3$ | H | $CH_3$ | $CH_3$ | $CH_2OCH_2CH{=}CH_2$ | |
| 40 | $OCH_3$ | $3-NO_2$ | $6-CH_3$ | H | $CH_3$ | $CH_3$ | $CH_2OCH_2C{\equiv}CH$ | semikristallin |
| 41 | $OC_2H_5$ | $3-NO_2$ | $6-CH_3$ | H | $CH_3$ | $CH_3$ | $CH_2SO_2CH_3$ | |
| 42 | $CH_3$ | $3-NO_2$ | $6-CH_3$ | H | $CH_3$ | $CH_3$ | $CH_2OSO_2NHC_3H_7$-n | |
| 43 | $CH_3$ | $3-NO_2$ | $6-CH_3$ | H | $CH_3$ | $CH_3$ | $CH_2OSO_2NHCH_3$ | Harz |
| 44 | $CH_3$ | $6-NO_2$ | H | H | $CH_3$ | $CH_3$ | 2-Furyl | |
| 45 | $CH_3$ | $6-NO_2$ | H | H | $CH_3$ | $CH_3$ | 2-Tetrahydrofuryl | |
| 46 | $CH_3$ | $6-NO_2$ | H | H | $CH_3$ | $CH_3$ | $CH_2CH_2OCH_3$ | |
| 47 | $CH_3$ | $6-NO_2$ | H | H | H | $CH_3$ | $CH_2CH_2OCH_3$ | |
| 48 | $CH_3$ | $6-NO_2$ | H | H | $CH_3$ | $CH_3$ | $CH_2OCH_3$ | Fp. 59–61 |
| 49 | $CH_3$ | $6-NO_2$ | H | H | $CH_3$ | $CH_3$ | $CH_2OCH_2CH{=}CH_2$ | Harz |
| 50 | $CH_3$ | $6-NO_2$ | H | H | $CH_3$ | $CH_3$ | $CH_2OCHC{\equiv}CH$ | |

TABELLE 1 (Fortsetzung)

| Verb. Nr. | $R_1$ | $R_2$ | $R_3$ | $R_4$ | R | $R_8$ | B | Physik. Konst. [°C] |
|---|---|---|---|---|---|---|---|---|
| 51 | $CH_3$ | 6–$NO_2$ | H | H | $CH_3$ | $CH_3$ | $CH_2SO_2CH_3$ | |
| 52 | $CH_3$ | 6–$NO_2$ | H | H | $CH_3$ | $CH_3$ | $CH_2OSO_2CH_3$ | |
| 53 | $CH_3$ | 6–$NO_2$ | H | H | $CH_3$ | $CH_3$ | $CH_2OSO_2NH_2$ | |
| 54 | $CH_3$ | 6–$NO_2$ | H | H | $CH_3$ | $CH_3$ | $CH_2OSO_2NHC_3H_7$-n | |
| 55 | $CH_3$ | 6–$NO_2$ | H | H | H | $CH_3$ | $CH_2OCH_3$ | |
| 56 | $OCH_3$ | 6–$NO_2$ | H | H | $CH_3$ | $CH_3$ | $CH_2OCH_3$ | |
| 57 | Cl | 6–$NO_2$ | H | H | $CH_3$ | $CH_3$ | $CH_2OCH_3$ | Fp. 95–99 |
| 58 | Br | 6–$NO_2$ | H | H | $CH_3$ | $CH_3$ | $CH_2OCH_3$ | |
| 59 | $CH_3$ | 6–$NO_2$ | 3–$CH_3$ | H | $CH_3$ | $CH_3$ | $CH_2OCH_3$ | Fp. 73–74 |
| 60 | $CH_3$ | 3–$NO_2$ | 6–$CH_3$ | H | $CH_3$ | $CH_3$ | 1H–1,2,4-Triazolyl-methyl | Fp. 150–159 |
| 61 | $CH_3$ | 6–$NO_2$ | 3–$CH_3$ | 5–$CH_3$ | $CH_3$ | $CH_3$ | $CH_2OCH_3$ | |
| 62 | $CH_3$ | 6–$NO_2$ | 3–Cl | 5–$CH_3$ | $CH_3$ | $CH_3$ | $CH_2OCH_3$ | |
| 63 | $CH_3$ | 6–$NO_2$ | 4–Cl | 3–$CH_3$ | $CH_3$ | $CH_3$ | $CH_2OCH_3$ | |
| 64 | $CH_3$ | 6–$NO_2$ | 3–$CH_3$ | 4–$CH_3$ | $CH_3$ | $CH_3$ | $CH_2OCH_3$ | |
| 65 | $OCH_3$ | 6–$NO_2$ | 3–$OCH_3$ | H | $CH_3$ | $CH_3$ | $CH_2OCH_3$ | |
| 66 | $OCH_3$ | 6–$NO_2$ | 3–$OCH_3$ | H | $CH_3$ | $C_3H_7$-i | $CH_2OCH_3$ | |
| 67 | $OCH_3$ | 6–$NO_2$ | 3–$OCH_3$ | H | H | $CH_3$ | 2-Furyl | |

TABELLE 1 (Fortsetzung)

| Verb. Nr. | $R_1$ | $R_2$ | $R_3$ | $R_4$ | R | $R_8$ | B | Physik. Konst. [°C] |
|---|---|---|---|---|---|---|---|---|
| 68 | Cl | $6-NO_2$ | 3-Cl | H | $CH_3$ | $CH_3$ | 2-Furyl | Harz |
| 69 | Cl | $6-NO_2$ | 3-Cl | $4-CH_3$ | $CH_3$ | $CH_3$ | 2-Furyl | |
| 70 | Cl | $6-NO_2$ | H | H | $CH_3$ | $CH_3$ | 1H-1,2,4-Triazolyl-methyl | |
| 71 | Br | $6-NO_2$ | 3-Br | H | $CH_3$ | $CH_3$ | $CH_2OCH_2CH=CH_2$ | |
| 72 | $CH_3$ | $6-NO_2$ | $3-CH_3$ | $4-CH_3$ | $CH_3$ | $C_3H_7$-i | $CH_2OC_2H_5$ | |
| 73 | $CH_3$ | $3-NO_2$ | $6-CH_3$ | H | $CH_3$ | $C_3H_7$-i | $CH_2OC_2H_5$ | visk. Oel |
| 74 | $CH_3$ | $3-NO_2$ | $6-CH_3$ | H | $CH_3$ | $C_2H_5$ | $CH_2OCH_3$ | rotes Oel |
| 75 | $CH_3$ | $4-NO_2$ | $6-CH_3$ | H | $CH_3$ | $CH_3$ | $CH_3OCH_3$ | |
| 76 | Cl | $4-NO_2$ | 6-Cl | H | $CH_3$ | $CH_3$ | $CH_3OCH_3$ | |
| 77 | $OCH_3$ | $4-NO_2$ | $6-OCH_3$ | H | $CH_3$ | $CH_3$ | $CH_3OCH_3$ | |
| 78 | $CH_3$ | $4-NO_2$ | $6-CH_3$ | $3-CH_3$ | $CH_3$ | $CH_3$ | $CH_3OCH_3$ | Fp. 58–66 |
| 79 | $CH_3$ | $4-NO_2$ | $6-CH_3$ | H | $CH_3$ | $CH_3$ | 2-Furyl | |
| 80 | $CH_3$ | $4-NO_2$ | $6-CH_3$ | H | $CH_3$ | $CH_3$ | 2-Tetrahydrofuryl | |
| 81 | $CH_3$ | $4-NO_2$ | $6-CH_3$ | H | $CH_3$ | $CH_3$ | 1H-1,2,4-Triazolyl-methyl | |
| 82 | $CH_3$ | $4-NO_2$ | $6-CH_3$ | H | $CH_3$ | $CH_3$ | $CH_2OC_2H_5$ | |
| 83 | $CH_3$ | $4-NO_2$ | $6-CH_3$ | H | $CH_3$ | $CH_3$ | $CH_2CH_2OCH_3$ | |
| 84 | $CH_3$ | $4-NO_2$ | $6-CH_3$ | H | $CH_3$ | $C_3H_7$-i | $CH_2OCH_3$ | |

TABELLE 1 (Fortsetzung)

| Verb. Nr. | $R_1$ | $R_2$ | $R_3$ | $R_4$ | R | $R_8$ | B | Physik. Konst. [°C] |
|---|---|---|---|---|---|---|---|---|
| 85 | $CH_3$ | 4–$NO_2$ | 6–$CH_3$ | H | H | $CH_3$ | $CH_3OCH_3$ | |
| 86 | $CH_3$ | 4–$NO_2$ | 6–$CH_3$ | H | $CH_3$ | $CH_3$ | $CH_2OCH_2CH{=}CH_2$ | |
| 87 | $CH_3$ | 4–$NO_2$ | 6–$CH_3$ | H | $CH_3$ | $CH_3$ | $CH_2OCH_2C{\equiv}CH$ | |
| 88 | $CH_3$ | 3–$NH_2$ | 6–Cl | H | $CH_3$ | $CH_3$ | $CH_2OC_2H_5$ | viscos; $n_D^{22}$ = 1.5532 |
| 89 | $CH_3$ | 4–$NO_2$ | 6–$CH_3$ | H | $CH_3$ | $CH_3$ | $CH_2OSO_2CH_3$ | |
| 90 | $CH_3$ | 4–$NO_2$ | H | 6–$CH_3$ | $CH_3$ | $CH_3$ | $CH_2OSO_2NHC_3H_7$-n | |
| 91 | $CH_3$ | 6–$NH_2$ | 5–$CH_3$ | H | $CH_3$ | $CH_3$ | $CH_2OCH_3$ | |
| 92 | $CH_3$ | 3–$NH_2$ | 5–$CH_3$ | 6–$CH_3$ | $CH_3$ | $CH_3$ | $CH_2CH_2OCH_3$ | |
| 93 | $CH_3$ | 6–$NH_2$ | 5–$CH_3$ | H | $CH_3$ | $CH_3$ | 2-Furyl | |
| 94 | $CH_3$ | 6–$NO_2$ | 5–$CH_3$ | H | $CH_3$ | $CH_3$ | $CH_2OCH_3$ | |
| 95 | $CH_3$ | 6–$NO_2$ | 5–$CH_3$ | H | $CH_3$ | $CH_3$ | 2-Tetrahydrofuryl | |
| 96 | $CH_3$ | 6–$NO_2$ | 5–$CH_3$ | H | $CH_3$ | $CH_3$ | $CH_2OCH_2CH{=}CH_2$ | |
| 97 | $CH_3$ | 3–$NO_2$ | 5–$CH_3$ | 6–$CH_3$ | $CH_3$ | $CH_3$ | $CH_2OCH_3$ | |
| 98 | Cl | 3–$NO_2$ | 5–Cl | 6–$CH_3$ | $CH_3$ | $CH_3$ | $CH_2OCH_3$ | |
| 99 | Cl | 3–$NO_2$ | 6–Cl | 4–Cl | $CH_3$ | $CH_3$ | $CH_2OCH_3$ | |
| 100 | Cl | 6–$NH_2$ | 5–Cl | 4–Cl | $CH_3$ | $CH_3$ | 2-Furyl | |
| 101 | $CH_3$ | 6–$NH_2$ | 5–$CH_3$ | 4–$CH_3$ | $CH_3$ | $CH_3$ | 1H-1,2,4-Triazolyl-methyl | |

TABELLE 1 (Fortsetzung)

| Verb. Nr. | $R_1$ | $R_2$ | $R_3$ | $R_4$ | R | $R_8$ | B | Physik. Konst. [°C] |
|---|---|---|---|---|---|---|---|---|
| 102 | $CH_3$ | 3–$NO_2$ | H | H | $CH_3$ | $CH_3$ | $CH_2OCH_3$ | F.p. 85–86 |
| 103 | $CH_3$ | 4–$NO_2$ | H | H | $CH_3$ | $CH_3$ | $CH_2OCH_3$ | Fp. 116–117 |
| 104 | $CH_3$ | 3–$NO_2$ | H | H | $CH_3$ | $CH_3$ | $CH_2OC_2H_5$ | gelbrotes Oel |
| 105 | $CH_3$ | 3–$NO_2$ | H | H | $CH_3$ | $CH_3$ | $CH_2OC_2H_5$ | gelbrotes Oel |
| 106 | F | 3–$NO_2$ | H | H | $CH_3$ | $CH_3$ | $CH_2OCH_3$ | Fp. 108–109 |
| 107 | $CH_3$ | 3–$NO_2$ | 6–Cl | H | $CH_3$ | $CH_3$ | $CH_2OC_2H_5$ | Harz; $n_D^{30}$: 1.5336 |

TABELLE 2

Verbindungen der Formel I mit Ar = substituiertem Phenyl, $R_5$ = H

| Verb. Nr. | $R_1$ | $R_2$ | $R_3$ | $R_4$ | R | W | B | Physik. Konst. [°C] |
|---|---|---|---|---|---|---|---|---|
| 108 | $CH_3$ | 3–$NO_2$ | 6–$CH_3$ | H | H | C(Cl)=$CH_2$ | $CH_2OCH_3$ | Oel; $n_D^{25}$ : 1.5596 |
| 109 | $CH_3$ | 3–$NO_2$ | H | H | H | C(Cl)=$CH_2$ | 2-Furyl | |
| 110 | $CH_3$ | 3–$NO_2$ | 6–$CH_3$ | H | $CH_3$ | C(Cl)=$CH_2$ | $CH_2OC_2H_5$ | |
| 111 | $CH_3$ | 3–$NO_2$ | 6–$CH_3$ | H | H | CH=$CH_2$ | $CH_2OCH_3$ | Oel; $n_D^{28}$ : 1.5500 |
| 112 | $CH_3$ | 3–$NH_2$ | 6–$CH_3$ | H | H | CH=$CH_2$ | $CH_2OCH_3$ | Oel; $n_D^{28}$ : 1.5695 |
| 113 | $CH_3$ | 3–$NH_2$ | 6–$CH_3$ | H | H | C(Cl)=$CH_2$ | $CH_2OCH_3$ | |
| 114 | $CH_3$ | 3–$NH_2$ | 6–$CH_3$ | H | H | C(Cl)=$CCl_2$ | $CH_2OCH_3$ | |
| 115 | $CH_3$ | 3–$NH_2$ | H | H | $CH_3$ | C(Cl)=$CCl_2$ | $CH_2OCH_3$ | |
| 116 | $CH_3$ | 3–$NO_2$ | H | H | H | C(Cl)=$CCl_2$ | $CH_2OCH_3$ | |
| 117 | $CH_3$ | 3–$NO_2$ | 6–$CH_3$ | H | H | C≡CH | $CH_2OCH_3$ | Fp. 63–64 |
| 118 | $CH_3$ | 3–$NO_2$ | H | H | H | C≡CH | $CH_2OCH_3$ | Fp. 69–73 |
| 119 | $CH_3$ | 3–$NO_2$ | H | H | $CH_3$ | C≡CH | $CH_2SO_2CH_3$ | |
| 120 | $CH_3$ | 3–$NH_2$ | 6–$CH_3$ | H | H | C≡CH | $CH_2$–$OSO_2NHCH_3$ | |
| 121 | $CH_3$ | 3–$NH_2$ | 6–$CH_3$ | H | H | C≡CH | $CH_2OC_2H_5$ | Oel; $n_D^{20}$ : 1.5568 |
| 122 | $CH_3$ | 3–$NO_2$ | 6–$CH_3$ | H | H | C≡CH | $CH_2OC_2H_5$ | Fp. 65–66 |
| 123 | $CH_3$ | 3–$NO_2$ | 6–$CH_3$ | H | $CH_3$ | $COOCH_3$ | CH=$CH_2$ | |

TABELLE 2 (Fortsetzung)

| Verb. Nr. | $R_1$ | $R_2$ | $R_3$ | $R_4$ | R | W | B | Physik. Konst. [°C] |
|---|---|---|---|---|---|---|---|---|
| 124 | $CH_3$ | 3–$NO_2$ | 6–$CH_3$ | H | $CH_3$ | $COOCH_3$ | Cyclopropyl | |
| 125 | $CH_3$ | 3–$NO_2$ | 6–$CH_3$ | H | $CH_3$ | $COOCH_3$ | $CH_2CH_2CH_3$ | Fp. 145–147 |
| 126 | $CH_3$ | 3–$NH_2$ | 6–$CH_3$ | H | $CH_3$ | $COOCH_3$ | $CH{=}CH_2$ | |
| 127 | $CH_3$ | 3–$NH_2$ | 6–$CH_3$ | H | $CH_3$ | $COOCH_3$ | Cyclopropyl | |
| 128 | $CH_3$ | 3–$NH_2$ | 6–$CH_3$ | H | $CH_3$ | $COOCH_3$ | $CH_2CH_2CH_3$ | |
| 129 | $CH_3$ | 3–$NO_2$ | 6–$CH_3$ | H | $CH_3$ | $COOCH_3$ | $CH{=}CHCH_3$ | Fp. 138–145 |
| 130 | $CH_3$ | 3–$NH_2$ | 6–$CH_3$ | H | $CH_3$ | $COOCH_3$ | $CH{=}CHCH_3$ | semikristallin |
| 131 | $CH_3$ | 3–$NO_2$ | 6–Cl | H | $CH_3$ | $COOCH_3$ | $CH_2CH_2CH_3$ | Fp. 112–114 |
| 132 | $CH_3$ | 3–$NO_2$ | 6–$CH_3$ | H | $CH_3$ | $COOCH_3$ | Cyclopropyl | Fp. 136–157 |
| 133 | $CH_3$ | 3–$NO_2$ | 6–$CH_3$ | 5–$CH_3$ | $CH_3$ | $COOCH_3$ | Cyclopropyl | Fp. 90–107 |
| 134 | $CH_3$ | 3–$NO_2$ | 6–$CH_3$ | H | $CH_3$ | CN | $CH_2OCH_3$ | Oel; $n_D^{25}$: 1.5589 |
| 135 | $CH_3$ | 3–$NH_2$ | 6–$CH_3$ | H | $CH_3$ | CN | $CH_2OCH_3$ | |
| 136 | $CH_3$ | 3–$NO_2$ | 6–$CH_3$ | 5–$CH_3$ | $CH_3$ | CN | $CH_2OCH_3$ | |
| 137 | $CH_3$ | 3–$NH_2$ | 6–$CH_3$ | 5–$CH_3$ | $CH_3$ | CN | $CH_2OCH_3$ | |
| 138 | Cl | 4–$NO_2$ | H | H | $CH_3$ | $COOCH_3$ | Cyclopropyl | Fp. 138–143 |
| 139 | Cl | 4–$NH_2$ | H | H | $CH_3$ | $COOCH_3$ | Cyclopropyl | Fp. 184–187 |

TABELLE 2 (Fortsetzung)

| Verb. Nr. | $R_1$ | $R_2$ | $R_3$ | $R_4$ | R | W | B | Physik. Konst. [°C] |
|---|---|---|---|---|---|---|---|---|
| 139a | $CH_3$ | 6–$NO_2$ | H | H | $CH_3$ | $COOCH_3$ | $CH_2N(CH_3)_2$ | |
| 139b | $CH_3$ | 6–$NO_2$ | H | H | $CH_3$ | $COOCH_3$ | $CH_2N(C_2H_5)_2$ | Harz |
| 139c | $CH_3$ | 6–$NO_2$ | H | H | $CH_3$ | $COOCH_3$ | $CH_2N(C_3H_7\text{-n})_2$ | |
| 139d | $CH_3$ | 6–$NH_2$ | H | H | $CH_3$ | $COOCH_3$ | $CH_2N(CH_3)_2$ | |
| 139e | $CH_3$ | 3–$NO_2$ | H | H | $CH_3$ | CN | $CH_2N(C_2H_5)_2$ | |

TABELLE 3

Verbindungen der Formel I mit Ar = substituiertes Phenyl, $R_4$ = 5–$CH_3$, $R_5$ = 6–$CH_3$

| Verb. Nr. | $R_1$ | $R_2$ | $R_3$ | R | $R_8$ | B | Physik. Konst. [°C] |
|---|---|---|---|---|---|---|---|
| 140 | $CH_3$ | 4–$NO_2$ | 3–$CH_3$ | H | $CH_3$ | $CH_2OCH_3$ | |
| 141 | $CH_3$ | 4–$NO_2$ | 3–$CH_3$ | $CH_3$ | $CH_3$ | $CH_2OCH_3$ | Fp. 118–120 |
| 142 | $CH_3$ | 4–$NO_2$ | 3–$CH_3$ | $CH_3$ | $CH_3$ | $CH_2OC_2H_5$ | Fp. 124–128 |
| 143 | $CH_3$ | 4–$NO_2$ | 3–$CH_3$ | $CH_3$ | $CH_3$ | $CH_2OC_3H_{7\text{-}i}$ | |
| 144 | $CH_3$ | 4–$NO_2$ | 3–$CH_3$ | $CH_3$ | $CH_3$ | 2-Tetrahydrofuryl | Fp. 163–168 |
| 145 | $CH_3$ | 4–$NO_2$ | 3–$CH_3$ | $CH_3$ | $CH_3$ | 2-Furyl | |
| 146 | $CH_3$ | 4–$NO_2$ | 3–$CH_3$ | $CH_3$ | $CH_3$ | $CH_2OCH_2CH{=}CH_2$ | |
| 147 | $CH_3$ | 4–$NO_2$ | 3–$CH_3$ | $CH_3$ | $CH_3$ | $CH_2OCH_2C{\equiv}CH$ | |
| 148 | $CH_3$ | 4–$NO_2$ | 3–$CH_3$ | $CH_3$ | $CH_3$ | $CH_2OSO_2NHCH_3$ | |
| 149 | $CH_3$ | 4–$NO_2$ | 3–$CH_3$ | H | $CH_3$ | –Furyl | |
| 150 | $CH_3$ | 4–$NO_2$ | 3–$CH_3$ | $CH_3$ | $CH_3$ | $CH_2OCH_3$ | |
| 151 | $CH_3$ | 4–$NO_2$ | 3–$CH_3$ | $CH_3$ | $C_2H_5$ | $CH_2OCH_3$ | |
| 152 | $CH_3$ | 4–$NO_2$ | 3–$CH_3$ | $CH_3$ | $C_3H_{7\text{-}i}$ | $CH_2OCH_3$ | |
| 153 | $CH_3$ | 4–$NO_2$ | 3–$CH_3$ | $CH_3$ | $C_3H_{7\text{-}n}$ | $CH_2OCH_3$ | |
| 154 | $C_2H_5$ | 4–$NO_2$ | 3–$CH_3$ | $CH_3$ | $CH_3$ | $CH_2OCH_3$ | |
| 155 | $CH_3$ | 4–$NO_2$ | 3–Cl | $CH_3$ | $CH_3$ | $CH_2OCH_3$ | |
| 156 | $CH_3$ | 4–$NO_2$ | 3–Cl | $CH_3$ | $CH_3$ | 2-Furyl | |

TABELLE 3 (Fortsetzung)

| Verb. Nr. | $R_1$ | $R_2$ | $R_3$ | R | $R_8$ | B | Physik. Konst. [°C] |
|---|---|---|---|---|---|---|---|
| 157 | $CH_3$ | $4-NH_2$ | $3-CH_3$ | H | $CH_3$ | $CH_2OCH_3$ | |
| 158 | $CH_3$ | $4-NH_2$ | $3-CH_3$ | $CH_3$ | $CH_3$ | $CH_2OCH_3$ | Fp. 120,5–123 |
| 159 | $CH_3$ | $4-NH_2$ | $3-CH_3$ | $CH_3$ | $CH_3$ | $CH_2OC_2H_5$ | Fp. 75,5–82 |
| 160 | $CH_3$ | $4-NH_2$ | $3-CH_3$ | $CH_3$ | $CH_3$ | $CH_2OC_3H_7$-i | |
| 161 | $CH_3$ | $4-NH_2$ | $3-CH_3$ | $CH_3$ | $CH_3$ | 2-Tetrahydrofuryl | Fp. 156–161 |
| 162 | $CH_3$ | $4-NH_2$ | $3-CH_3$ | $CH_3$ | $CH_3$ | 2–Furyl | |
| 163 | $CH_3$ | $4-NH_2$ | $3-CH_3$ | $CH_3$ | $CH_3$ | $CH_2OCH_2CH=CH_2$ | |
| 164 | $CH_3$ | $4-NH_2$ | $3-CH_3$ | $CH_3$ | $CH_3$ | $CH_2OCH_2C\equiv CH$ | |
| 165 | $CH_3$ | $4-NH_2$ | $3-CH_3$ | $CH_3$ | $CH_3$ | $CH_2OSO_2NHCH_3$ | |

TABELLE 4

Verbindungen de Formel I mit Ar = $\alpha$-Naphthyl, $R_2$ = 2–$NO_2$

| Verb. Nr. | $R_3$ | $R_6$ | R | W | B | Physik. Konst. [°C] |
|---|---|---|---|---|---|---|
| 400 | H | H | $CH_3$ | $COOCH_3$ | $CH_2OCH_3$ | Fp. 103–107 |
| 401 | H | H | H | $COOCH_3$ | $CH_2OCH_3$ | |
| 402 | $CH_3$ | H | $CH_3$ | $COOCH_3$ | $CH_2OCH_3$ | viskoses Harz |
| 403 | H | H | $CH_3$ | $COOCH_3$ | $CH_2OC_2H_5$ | |
| 404 | H | H | H | $COOCH_3$ | 2-Furyl | |
| 405 | H | H | H | $COOCH_3$ | 2-Tetrahydrofuryl | |
| 406 | H | H | $CH_3$ | $COOCH_3$ | $CH_2SO_2CH_3$ | |
| 407 | H | H | H | $COOCH_3$ | $CH_2CH_2OCH_3$ | |
| 408 | H | H | $CH_3$ | $COOCH_3$ | $CH_2OCH_2CH{=}CH_2$ | viskoses Harz |
| 409 | H | H | $CH_3$ | $COOCH_3$ | $CH_2OCH_2C{\equiv}CH$ | semikristallin |
| 410 | H | H | $CH_3$ | $COOCH_3$ | 1H–1,2,4-Triazolyl-methyl | |
| 411 | H | H | $CH_3$ | $COOCH_3$ | $CH_2OSO_2CH_3$ | |
| 412 | H | H | H | $COOCH_3$ | $CH_2OSO_2NHCH_3$ | |
| 413 | H | H | $CH_3$ | $COOCH_3$ | $CH_2OSO_2NHCH_3$ | |
| 414 | H | H | H | $C{\equiv}CH$ | $CH_2OCH_3$ | |
| 415 | H | H | H | $C{\equiv}CH$ | $CH_2OC_2H_5$ | |

TABELLE 4 (Fortsetzung)

| Verb. Nr. | $R_3$ | $R_6$ | R | W | B | Physik. Konst. [°C] |
|---|---|---|---|---|---|---|
| 416 | H | H | $CH_3$ | $C{\equiv}CH$ | $CH_2OCH_3$ | |
| 417 | H | H | H | CN | $CH_2OCH_3$ | |
| 418 | H | H | $CH_3$ | CN | $CH_2OCH_3$ | |
| 419 | H | H | $CH_3$ | $CH{=}CH_2$ | $CH_2OCH_3$ | zähes Oel |
| 420 | H | H | $CH_3$ | $C(Cl){=}CCl_2$ | $CH_2OCH_3$ | |
| 421 | 5–Br | H | H | $COOCH_3$ | $CH_2OCH_3$ | |
| 422 | 5–Br | H | $CH_3$ | $COOC_2H_5$ | $CH_2OCH_3$ | |
| 423 | 5–Br | H | $CH_3$ | $COOCH_3$ | $CH_2OCH_3$ | |
| 424 | 5–Br | 4–$CH_3$ | $CH_3$ | $C{\equiv}CH$ | 2-Furyl | |
| 425 | 5–Br | H | $CH_3$ | CN | 2-Tetrahydrofuryl | Harz |
| 426 | 5–Br | H | $CH_3$ | $CH{=}CH_2$ | $CH_2OCH_3$ | |
| 427 | 5–Br | H | $CH_3$ | $C(Cl){=}CCl_2$ | $CH_2OCH_3$ | |
| 428 | 5–Br | H | $CH_3$ | $COOCH_3$ | Cyclopropyl | |
| 429 | H | H | $CH_3$ | $COOCH_3$ | Cyclopropyl | |
| 430 | H | 4–$CH_3$ | $CH_3$ | $COOCH_3$ | $CH_2OCH_3$ | |

TABELLE 5

Verbindungen der Formel I mit Ar = $\alpha$-Naphthyl, $R_2$ = 5–$NO_2$

| Verb. Nr. | $R_3$ | $R_6$ | R | W | B | Physik. Konst. [°C] |
|---|---|---|---|---|---|---|
| 501 | 2–$CH_3$ | H | H | $COOCH_3$ | $CH_2OCH_3$ | |
| 502 | 2–$CH_3$ | H | $CH_3$ | $COOCH_3$ | $CH_2OCH_3$ | |
| 503 | 2–$CH_3$ | H | $CH_3$ | $COOC_2H_5$ | $CH_2OCH_3$ | |
| 504 | 2–$CH_3$ | H | $CH_3$ | $C\equiv CH$ | $CH_2OCH_3$ | |
| 505 | 2–$CH_3$ | H | $CH_3$ | $C\equiv CCl$ | $CH_2OCH_3$ | Harz |
| 506 | 2–$CH_3$ | H | $CH_3$ | $CH=CH_2$ | $CH_2OCH_3$ | |
| 507 | 2–$CH_3$ | H | H | $C(Cl)=CH_2$ | $CH_2OCH_3$ | |
| 508 | 2–$CH_3$ | H | $CH_3$ | CN | $CH_2OCH_3$ | |
| 509 | 2–$CH_3$ | H | $CH_3$ | $COOCH_3$ | $CH_2OSO_2NHCH_3$ | |
| 510 | 2–$CH_3$ | H | $CH_3$ | $COOCH_3$ | $CH_2CH_2OCH_3$ | |
| 511 | 2–$CH_3$ | H | $CH_3$ | $COOCH_3$ | $CH_2SO_2CH_3$ | |
| 512 | 2–$CH_3$ | H | $CH_3$ | $COOCH_3$ | $CH_2OCH_2C\equiv CH$ | Harz |
| 513 | 2–$CH_3$ | 3–$CH_3$ | $CH_3$ | $CH=CH_2$ | $CH_2OCH_3$ | |
| 514 | 2–$CH_3$ | 3–$CH_3$ | H | $CH=CH_2$ | $CH_2OCH_3$ | |
| 516 | 2–$CH_3$ | H | H | $COOCH_3$ | $CH_2N(CH_3)_2$ | |

## TABELLE 6

Verbindungen der Formel I mit Ar = $\alpha$-Naphthyl, $R_2$ = 4–$NO_2$

| Verb. Nr. | $R_3$ | $R_6$ | R | W | B | Physik. Konst. [°C] |
|---|---|---|---|---|---|---|
| 601 | 2–$CH_3$ | 3–$CH_3$ | H | $COOCH_3$ | $CH_2OCH_3$ | |
| 602 | 2–$CH_3$ | 3–$CH_3$ | $CH_3$ | $COOCH_3$ | $CH_2OCH_3$ | |
| 603 | 3–$CH_3$ | 3–$CH_2$ | $CH_3$ | $COOC_2H_5$ | $CH_2OCH_3$ | |
| 604 | 2–$CH_3$ | 3–$CH_3$ | $CH_3$ | CN | 2-Furyl | |
| 605 | 2–$CH_3$ | 3–$CH_3$ | $CH_3$ | $COOCH_3$ | $CH_2SO_2CH_3$ | |
| 606 | 2–$CH_3$ | H | $CH_3$ | $COOCH_3$ | $CH_2OCH_3$ | Fp. 93,5–99 |
| 607 | 2–$CH_3$ | H | $CH_3$ | $COOCH_3$ | 2-Tetrahydrofuryl | viscos; $n_D^{21}$: 1.5948 |
| 608 | 2–$CH_3$ | H | H | C≡CH | $CH_2OCH_3$ | |
| 609 | H | H | $CH_3$ | $COOC_2H_5$ | $CH_2OCH_3$ | Fp. 103–105 |
| 610 | J | H | H | $COOCH_3$ | $CH_2OCH_3$ | |
| 611 | H | H | $CH_3$ | $COOCH_3$ | $CH_2OCH_3$ | Fp. 110–116 |
| 612 | H | H | $CH_3$ | $COOCH_3$ | 1H-1,2,4-Triazolyl-methyl | |
| 613 | 2–$CH_3$ | 3–$CH_3$ | $CH_3$ | $COOCH_3$ | 1H-1,2,4-Triazolyl-methyl | |
| 614 | 2–$CH_3$ | 3–$CH_3$ | H | CH=$CH_2$ | $CH_2OCH_3$ | |
| 615 | H | H | H | C≡CH | $CH_2OCH_3$ | Fp. 111–114 |

TABELLE 7

Verbindungen der Formel I mit Ar = α-Naphthyl, $R_2 = 2-NH_2$

| Verb. Nr. | $R_3$ | $R_6$ | R | W | B | Physik. Konst. [°C] |
|---|---|---|---|---|---|---|
| 701 | H | H | $CH_3$ | $COOCH_3$ | $CH_2OCH_3$ | Fp. 159–162 |
| 702 | H | H | H | $COOCH_3$ | $CH_2OCH_3$ | |
| 703 | H | H | $CH_3$ | $COOCH_3$ | 2-Tetrahydrofuryl | |
| 704 | H | H | $CH_3$ | $COOCH_3$ | 2-Furyl | |
| 705 | H | H | $CH_3$ | $COOCH_3$ | $CH_2SO_2CH_3$ | |
| 706 | H | H | $CH_3$ | $COOCH_3$ | $CH_2OSO_2CH_3$ | |
| 707 | H | H | H | $C\equiv CH$ | $CH_2OCH_3$ | |
| 708 | H | H | H | $CH=CH_2$ | $CH_2CH_2CH=CH_2$ | |
| 709 | H | H | H | $C(Cl)=CCl_2$ | $CH_2OCH_3$ | |
| 710 | 5–Br | H | $CH_3$ | $COOCH_3$ | $CH_2OCH_3$ | |
| 711 | 5–Br | H | $CH_3$ | $COOC_2H_5$ | $CH_2OCH_3$ | |
| 712 | 5–Br | H | $CH_3$ | $COOCH_3$ | 2-Furyl | Harz |
| 713 | H | 5–$CH_3$ | $CH_3$ | $COOCH_3$ | $CH_2OCH_3$ | |
| 714 | H | H | $CH_3$ | $COOCH_3$ | $CH_2OSO_2NHCH_3$ | |
| 715 | H | H | $CH_3$ | $COOCH_3$ | $CH_2N(CH_3)_2$ | |

TABELLE 8

Verbindungen der Formel I mit Ar = $\alpha$-Naphthyl

| Verb. Nr. | $R_2$ | $R_3$ | $R_6$ | R | W | B | Physik. Konst. [°C] |
|---|---|---|---|---|---|---|---|
| 801 | 4—$NH_2$ | H | H | H | $COOCH_3$ | $CH_2OCH_3$ | |
| 802 | 4—$NH_2$ | H | H | $CH_3$ | $COOCH_3$ | $CH_2OCH_3$ | |
| 803 | 4—$NH_2$ | H | H | H | $CH{=}CH_2$ | $CH_2OCH_3$ | |
| 804 | 4—$NH_2$ | H | H | $CH_3$ | $COOC_2H_5$ | $CH_2OC_2H_5$ | |
| 805 | 4—$NH_2$ | H | H | H | $C{\equiv}CH$ | $CH_2OSO_2CH_3$ | |
| 806 | 4—$NH_2$ | 2—$CH_3$ | H | $CH_3$ | $COOCH_3$ | $CH_2OCH_3$ | Fp. 161—167 |
| 807 | 4—$NH_2$ | 2—$CH_3$ | H | $CH_3$ | CN | $CH_2OCH_3$ | |
| 808 | 4—$NH_2$ | 2—$CH_3$ | 3—$CH_3$ | $CH_3$ | $COOCH_3$ | $CH_2OCH_3$ | |
| 809 | 4—$NH_2$ | 2—$CH_3$ | H | $CH_3$ | $COOCH_3$ | 2-Tetrahydrofuryl | Fp. 206—220 |
| 810 | 5—$NH_2$ | 2—$CH_3$ | H | H | $COOCH_3$ | $CH_2OCH_3$ | |
| 811 | 5—$NH_2$ | 2—$CH_3$ | H | $CH_3$ | $COOCH_3$ | $CH_2OCH_3$ | |
| 812 | 5—$NH_2$ | 2—$CH_3$ | H | H | $C{\equiv}CH$ | $CH_2OCH_3$ | |
| 813 | 5—$NH_2$ | 2—$CH_3$ | H | H | $C(Cl){=}CCl_2$ | $CH_2OCH_3$ | |
| 814 | 5—$NH_2$ | 2—$CH_3$ | H | $CH_3$ | $COOCH_3$ | 2-Furyl | |
| 815 | 4—$NH_2$ | H | H | H | $COOCH_3$ | $CH_2OSO_2NHCH_3$ | |
| 816 | 4—$NH_2$ | H | H | $CH_3$ | $COOCH_3$ | $CH_2N(CH_3)_2$ | |

Zur Applikation können die Verbindungen der Formel I in den folgenden Aufarbeitungsformen vorliegen

*Formulierungsbeispiele*

Beispiel 7

Feste Aufarbeitungsformen:

*Stäube- und Streumittel* enthalten im allgemeinen bis zu 100% des Wirkstoffes. Ein 5%iges Stäubemittel kann beispielsweise aus 5 Teilen des Wirkstoffs und 95 Teilen eines Zuschlagstoffes wie Talkum bestehen oder aus 5 Teilen Wirkstoff, 2 Teil hochdisperser Kieselsäure und 93 Teilen Talkum. Darüberhinaus sind weitere Gemische mit solchen und anderen in der Formulierungstechnik gebräuchlichen Trägermaterialien und Zuschlagstoffen denkbar. Bei der Herstellung dieser Stäubemittel werden die Wirkstoffe mit den Träger- und Zuschlagstoffen vermischt und vermahlen und können in dieser Form verstäubt werden.

*Granulate* wie Umhüllungsgranulate, Imprägniergranulate, Homogengranulate und Pellets[=Körner] enthalten üblicherweise 1 bis 80% des Wirkstoffs. So kann sich ein 5%iges Granulat z.B. aus 5 Teilen des Wirkstoffs, 0,25 Teilen epoxidiertem Pflanzenöl, 0,25 Teilen Cetylpolyglykolether, 3,50 Teilen Polyethylenglykol und 91 Teilen Kaolin (bevorzugte Korngrösse 0,3—0,8 mm) zusammensetzen. Man kann bei der Herstellung des Granulates wie folgt vorgehen:

Die Aktivsubstanz wird mit epoxidiertem Pflanzenöl vermischt und mit 6 Teilen Aceton gelöst, hierauf wird Polyethylenglykol und Cetylpolyglykolether zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 8

Flüssige Aufarbeitungsformen:

Man unterscheidet im allgemeinen zwischen Wirkstoffkonzentraten, die in Wasser *dispergierbar* oder *löslich* sind und *Aerosolen*. Zu den in Wasser dispergierbaren Wirkstoffkonzentraten zählen z.B. *Spritzpulver* (wettable powders) und Pasten, die üblicherweise in den Handelspackungen 25—90% und in gebrauchsfertigen Lösungen 0,01 bis 15% des Wirkstoffs enthalten. *Emulsionskonzentrate* enthalten 10 bis 50% und *Lösungskonzentrate* enthalten in der gebrauchsfertigen Lösung 0,0001 bis 20% Aktivsubstanz. So kann ein 70%iges Spritzpulver z.B. aus 70 Teilen des Wirkstoffs, 5 Teilen Natriumdibutylnaphthylsulfonat, dazu 3 Teilen Naphthalinsulfonsäuren — Phenolsulfonsäuren — Formaldehyd-Kondensat (im Mischverhältnis 3:2:1), 10 Teilen Kaolin und 12 Teilen Kreide, z.B. Champagne-Kreide zusammengesetzt sein. Ein 40%iges Spritzpulver kann z.B. aus folgenden Stoffen bestehen: 40 Teile Wirkstoff, 5 Teile Natrium-Ligninsulfonat, 1 Teil Natrium-Dibutylnaphthylsulfonat und 54 Teile Kieselsäure. Die Herstellung eines 25%igen Spritzpulvers kann auf unterschiedliche Art erfolgen. So kann dieses sich z.B. zusammensetzen aus: 25 Teilen der Aktivsubstanz, 4,5 Teilen Calcium-Ligninsulfonat, 1,9 Teilen Kreide, [z.B. Champagne-Kreide]/Hydroxyethylencellulose-Gemisch (1:1), 1,5 Teilen Natrium-Dibutylnaphthylsulfonat, 19,5 Teilen Kieselsäure, 19,5 Teilen Kreide, [z.B. Champagne-Kreide] und 28,1 Teilen Kaolin. Ein 25%iges Spritzpulver kann z.B. auch bestehen aus 25 Teilen Wirkstoff, 2,5 Teilen Isooctylphenoxy-polyoxyethylenethanol, 1,7 Teilen [Champagne]-Kreide/Hydroxyethylcellulosegemisch (1:1), 8,3 Teilen Natriumsilikat, 16,5 Teilen Kieselgur und 46 Teilen Kaolin. Ein 10%iges Spritzpulver lässt sich z.B. herstellen aus 10 Teilen des Wirkstoffes, 3 Teilen eines Gemisches aus Natriumsalzen von gesättigten Fettalkoholsulfonaten, 5 Teilen Naphthalinsulfonsäure/Formaldehyd-Kondensat und 82 Teilen Kaolin. Andere Spritzpulver können Gemische darstellen aus 5 bis 30% der Aktivsubstanz zusammen mit 5 Teilen eines aufsaugenden Trägermaterials wie Kieselsäure, 55 bis 80 Teilen eines Trägermaterials wie Kaolin und eines Dispergiermittelgemisches, bestehend aus 5 Teilen Natrium-Arylsulfonates sowie aus 5 Teilen eines Alkylarylpolyglykolethers. Ein 25%iges Emulsions-Konzentrat kann z.B. folgende emulgierbare Stoffe enthalten: 25 Teile des Wirkstoffs, 2,5 Teile epoxidiertes Pflanzenöl, 10 Teile eines Alkylarylsulfonat/Fettalkoholpolyglykoltehr-Gemisches, 5 Teile Dimethylformamid und 57,5 Teile Xylol.

Aus solchen Konzentraten können durch Verdünnen mit Wasser Emulsionen der gewünschten Anwendungskonzentration hergestellt werden, die besonders zur Blattapplikation geeignet sind. Darüberhinaus können weitere Spritzpulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnt und insbesondere zur Blattapplikation verwenden lassen. Solche Mittel gehören ebenfalls zur Erfindung.

Mittel, die nach der oben beschriebenen Art formuliert wurden und als Wirkungskomponente eine Verbindung der Formel I (z.B. Nr. 2, 6, 8, 10, 28, 31, 33, 48, 57, 59, 73 oder 74) enthielten, liessen sich mit sehr gutem Erfolg zur Bekämpfung von phytopathogenen Mirkoorganismen einsetzen. Mit gleich gutem oder ähnlichem Erfolg können auch andere Verbindungen aus den Tabellen 1 bis 8 eingesetzt werden.

*Biologische Beispiele*

Die in den nachfolgenden Beispielen verwendeten Spritzbrühen wurden, wie oben beschrieben, formuliert.

Beispiel 9

Wirkung gegen Erysiphe graminis auf Gerste

a) *Residual-protektive Wirkung*

Ca. 8 cm hohe Gerstenpflanzen wurden mit einer aus Spritzpulver des Wirkstoffes hergestellten Spritzbrühe (0,02% Aktivsubstanz) besprüht. Nach 3—4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Bei der residual-protektiven Behandlung von Gerstenpflanzen gegen Erysiphe-Pilze mit Aktivsubstanzen der Formel I, wie z.B. mit Verbindungen Nr. 10, 38, 60, 104, 107 oder 142 wurde der Befall der behandelten Pflanzen im Vergleich mit Kontrollpflanzen (100% Befall) auf weniger als 10% zurückgedrängt.

Beispiel 10

Wirkung gegen Phytophthora infestans auf Tomatenfplanzen

a) *Residual-protektive Wirkung*

Tomatenpflanzen wurden nach 3-wöchiger Anzucht mit einer aus Spritzpulver des Wirkstoffes hergestellten Spritzbrühe (0,06% Aktivsubstanz) besprührt. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90—100% relativer Luftfeuchtigkeit und 20°C.

Im Vergleich zu unbehandelten Kontrollpflanzen (100% Befall) zeigten Pflanzen, die mit einer der Verbindungen Nr. 2, 6, 8, 10, 14, 28, 29, 31, 33, 35, 38, 48, 57, 59, 60, 73, 74, 78, 88, 102 bis 107, 111, 117, 122, 138, 139, 141, 142, 144, 158, 159, 161, 400, 606, 607, 611 oder 701 behandelt waren, weniger als 10% Befall.

b) *Systemische Wirkung*

Zu Tomatenplfanzen wurde nach 3-wöchiger Anzucht eine aus Spritzpulver des Wirkstoffes hergestellte Spritzbrühe gegossen (0,06% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90—100% relativer Luftfeuchtigkeit und 20°C.

Unter anderen zeigten in obigem Versuch die Verbindungen Nr. 2, 6, 8, 10, 14, 28, 29, 31, 33, 35, 38, 48, 57, 59, 60, 73, 74, 78, 88, 102 bis 107, 111, 117, 122, 138, 139, 141, 142, 144, 158, 159, 161, 400, 606, 607, 611 und 701 eine sehr gute systemische Wirkung. Gegenüber unbehandelten Kontrollpflanzen (100% Befall) bewirkten diese Verbindungen eine fast vollständige Unterdrückung des Pilzbefalls(0 bis 5%).

c) *Residual-kurative Wirkung*

Tomatenpflanzen wurden nach 3-wöchiger Anzucht mit einer Sporangiensuspension des Pilzes infiziert. Nach einer Inkubation von 22 Stunden in einer Feuchtkammer bei 90—100% relativer Luftfeuchtigkeit und 20°C wurden die infizierten Pflanzen getrocknet und mit einer aus Spritzpulver des Wirkstoffes hergestellten Spritzbrühe (0.06% Aktivsubstanz) besprüht. Nach dem Antrocknen des Spritzbelages wurden die behandelten Pflanzen wieder in die Feuchtkammer gebracht. Die Beurteilung des Pilzbefalls erfolgte 5 Tage nach der Infektion.

Im Vergleich zu unbehandelten, aber infizierten Kontrollpflanzen (100% Befall) zeigten Pflanzen, die mit einer der Verbindungen Nr. 2, 6, 8, 10, 28, 29, 31, 33, 48, 59, 74, 107, 141, 142, 161, 400, 606, 611 und 701 behandelt waren, weniger als 10% Befall.

Beispiel 11

Residual protective Wirkung gegen Venturia inaequalis auf Apfeltrieben

Apfelstecklinge mit 10—20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90—100% relativer Luftfeuchtigkeit inkubiert und während 10 weiteren Tagen in einem Gewächshaus bei 20—24°C aufgestellt. Der Schorfbefall wurde 15 Tage nach der Infektion beurteilt. Unter anderen drängten die Verbindungen Nr. 8, 10, 28, 31 und 701 den Krankheitsbefall auf 10 bis 15% zurück.

Beispiel 12

Wirkung gegen Pythium debaryanum auf Rüben

a) *Wirkung nach Bodenapplikation*

Der Pilz wurde auf Karottenschnitzel-Nährlösung kultiviert und einer Erde-Sand-Mischung beigegeben. Die so infizierte Erde wurde in Blumentöpfe abgefüllt und mit Zuckerrübensamen besät. Gleich nach der Aussaat wurden die als Spritzpulver formulierten Versuchspräparate als wässrige Suspension über die Erde gegossen (20 ppm einer der Verbindungen aus den Tabellen 1 und 2 bezogen auf das Erdvolumen).

Die Töpfe wurden darauf während 2—3 Wochen im Gewächshaus bei ca. 20°C aufgestellt. Die Erde wurde dabei durch leichtes Ueberbrausen stets gleichmässig feucht gehalten. Bei der Auswertung des Tests wurde der Auflauf der Zuckerrübenpflanzen sowie der Anteil gesunder und kranker Pflanzen bestimmt.

b) *Wirkung nach Beizapplikation*

Der Pilz wurde auf Karottenschnitzel-Nährlösung kultiviert und einer Erde-Sand-Mischung beigegeben. Die so infizierte Erde wurde in Erdschalen abgefüllt und mit Zuckerrübensamen besät, die mit den als Beizpulver formulierten Versuchspräparaten gebeizt worden waren (0,06% einer der Verbindungen aus den Tabellen 1 und 2).

Die besäten Töpfe wurden während 2—3 Wochen im Gewächshaus bei ca. 20°C aufgestellt. Die Erde wurde dabei durch leichtes Ueberbrausen stets gleichmässig feucht gehalten. Bei der Auswertung wurde der Auflauf der Zuckerrübenpflanzen bestimmt.

Die Verbindungen Nr. 2, 6, 8, 10, 28, 29, 31, 33, 48, 59, 73, 74, 107, 158, 160, 400, 606, 607 und 701 zeigten in beiden Versuchen a) und b) gegen Pythium-Erreger (über 90% aufgelaufene Pflanzen) volle Wirkung. Die Pflanzen hatten ein gesundes Aussehen.

Eine gleich gute Wirkung wurde bei analogen Versuchen gegen Pythium-Erreger auf Mais, erzielt.

Beispiel 13

Wirkung gegen Podosphaera leucotricha auf Apfelsämlingen Residual-protektive Wirkung

Apfelsämlinge mit ca. 5 entwickelten Blättern wurden mit einer aus Spritzpulver des Wirkstoffes hergesstellten Spritzbrühe (0,06% Aktivsubstanz) besprüht. Nach 24 Stunden wurden die behandelten Pflanzen mit einer Konidiensuspension des Pilzes infiziert und in einer Klimakammer bei 70% relativer Luftfeuchtigkeit und 20°C aufgestellt. Die Beurteilung des Pilzbefalls erfolte 12 Tage nach der Infektion.

Im Vergleich zu unbehandelten aber infizierten Apfelsämlingen (100% Befall), zeigten Sämlinge die mit einer der Verbindungen Nr. 2, 6, 8, 10, 28, 31, 33, 48, 73 oder 606 behandelt waren, weniger als 10% Befall.

Beispiel 14

Messung physikochemischer Kenndaten

Im Vergleich zu dem aus US—PS 4 151 299 bekannt gewordenen Pflanzenfungizid "Metalaxyl" des Handels, N-(2,6-Dimethylphenyl)-N-(2'-methoxy-acetyl)-alaninmethylester, besitzen Wirkstoffe vorliegender Erfindung, die einer ähnlichen chemischen Strukturklasse angehören, physikochemische Kenndaten, die für die Wirkungsdauer eines Pflanzenfungizids deutlich günstiger liegen.

a) *Verringerte Wasserlöslichkeit*

| Verb. Nr. | Löslichkeit bei 25°C |
|---|---|
| Metalaxyl | 8000 ppm |
| 48 | 4000 ppm |
| 28 | 2500 ppm |
| 31 | 2500 ppm |
| 33 | 500 ppm |
| 2 | 300 ppm |

b) *Verringerte Flüchtigkeit*

Im Verdampfungsrohr werden bei 50°C und bei einer Luftströmung von 20 m/h von repräsentativen Fungiziden vorliegender Erfindung die Verdampfungshalbwertzeiten $T_{50}$ ermittelt. Die Messdaten sind im folgenden relativ zu der entsprechenden Halbwertzeit $T_{50}$ des Metalaxyl dargestellt.

| Verb. Nr. | Mehrfaches der Metalaxyl-$T_{50}$ |
|---|---|
| 2 | 5,4 × |
| 48 | 12,3 × |
| 28 | 35,8 × |
| 31 | 41,8 × |
| 33 | 45,6 × |

Die vorstehenden Versuche a) und b) beweisen die physikochemischen Vorteile der erfindungsgemässen Verbindungen der Formel I. Diese Vorteile verhindern einen unerwünschten vorzeitigen Wirkstoffverlust und gewährleisten damit grössere Dauerwirkung der Präparate.

**Patentansprüche für die Vertragsstaaten: BE CH DE FR GB IT LI NL SE**

1. Verbindungen der Formel I

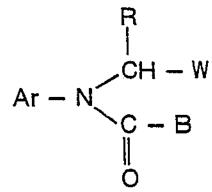

(I)

worin

R für Wasserstoff oder Methyl steht;

Ar eine der aromatischen Gruppen

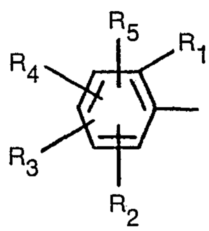

oder

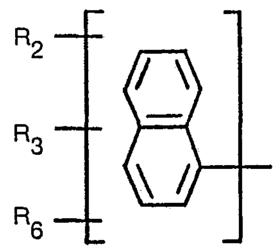

repräsentiert, wobei

$R_1$ $C_1$—$C_3$-Alkyl, $C_1$—$C_3$-Alkoxy oder Halogen bedeutet;

$R_2$ für $NO_2$ oder $NH_2$ steht;

$R_3$ Wasserstoff, $C_1$—$C_3$-Alkyl, $C_1$—$C_3$-Alkoxy oder Halogen;

$R_4$ Wasserstoff oder $C_1$—$C_3$-Alkyl;

$R_5$ Wasserstoff oder $C_1$—$C_3$-Alkyl;

$R_6$Wasserstoff, $C_1$—$C_3$-Alkyl oder Halogen bedeutet;

W für Cyano, —$COOR_8$, —$C(R_9)$=$C(R_{10})(R_{11})$ oder —C≡C—$R_{12}$ steht, wobei

$R_8$ $C_1$—$C_3$-Alkyl;

$R_9$, $R_{10}$, $R_{11}$ und $R_{12}$ unabhängig voneinander Wasserstoff, $C_1$—$C_3$-Alkyl oder Halogen bedeuten und

B für $C_3$—$C_4$-Alkyl, $C_2$—$C_4$-Alkenyl, Cyclopropyl, 2-Furyl, 2-Tetrahydrofuryl, β-($C_1$—$C_2$-Alkoxy)ether oder die Gruppe $CH_2Z$ steht, wobei

Z für 1H-1,2,4-Triazolyl, Methylsulfonyl, X—$R_{13}$, $OSO_2$—$R_{14}$ oder —$N(R_{15})(R_{16})$ steht, wobei

X Sauerstoff oder Schwefel bedeutet;

$R_{13}$ für eine Alkyl-, Alkenyl- oder Alkinylgruppe mit maximal 4 Kohlenstoffatomen steht;

$R_{14}$ $C_1$—$C_3$-Alkyl oder NH($C_1$—$C_3$-Alkyl) bedeutet und

$R_{15}$ und $R_{16}$ unabhängig voneinander für $C_1$—$C_3$-Alkyl stehen.

2. Verbindungen der Formel I nach Anspruch 1, dadurch gekennzeichnet, dass R für Wasserstoff oder Methyl steht, Ar für die Gruppe

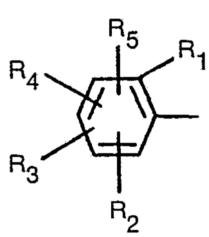

oder

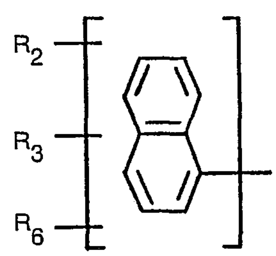

steht; $R_1$ für Methyl, Ethyl, Methoxy, Ethoxy oder Halogen steht; $R_2$ Nitro oder Amino; $R_3$ und $R_6$ unabhängig voneinander Wasserstoff, Methyl, Ethyl, Methoxy, Ethoxy oder Halogen bedeuten; $R_4$ und $R_5$ unabhängig voneinander für Wasserstoff, Methyl oder Ethyl stehen; W für CN, $COOR_8$, —$C(R_9$=$C(R_{10})(R_{11})$ oder —C≡C—$R_{12}$ steht, wobei $R_8$ $C_1$—$C_3$-Alkyl bedeutet; $R_9$, $R_{10}$, $R_{11}$ und $R_{12}$ unabhängig voneinander für Wasserstoff, Methyl, Chlor oder Brom stehen und B für $C_1$—$C_3$-Alkyl, $C_2$—$C_4$-Alkenyl, Cyclopropyl, 2-Furyl, 2-Tetrahydrofuryl, $CH_2O$($C_1$—$C_3$-Alkyl), 1H-Triazolylmethyl, $CH_2SO_2CH_3$, $CH_2OSO_2CH_3$, $CH_2OCH_2CH$=$CH_2$, $CH_2OCH_2C$≡CH, $CH_2OSO_2NHCH_3$, $CH_2N(CH_3)_2$, $CH_2N(C_2H_5)_2$ oder $CH_2N(C_3H_7$-n$)_2$ steht.

3. Verbindungen der Formel I nach Anspruch 2, dadurch gekennzeichnet, dass R Methyl bedeutet; $R_1$

für Methyl, Methoxy, Chlor oder Brom steht; $R_3$ und $R_6$ unabhängig voneinander Wasserstoff, Methyl, Chlor oder Brom bedeuten; $R_4$ und $R_5$ unabhängig voneinander für Wasserstoff oder Methyl stehen; W für CN, $COOR_8$, $—C(R_9)=C(R_{10})(R_{11})$ oder $—C\equiv C—R_{12}$ steht, wobei $R_9$, $R_{10}$, $R_{11}$ und $R_{12}$ unabhängig voneinander für Wasserstoff, Methyl oder Chlor stehen und B 2-Furyl, 2-Tetrahydrofuryl, $CH_2O(C_1—C_3\text{-Alkyl})$, 1H-1,2,4-Triazolylmethyl, $CH_2SO_2CH_3$, $CH_2OSO_2CH_3$, $CH_2OCH_2CH=CH_2$, $CH_2OCH_2C\equiv CH$, $CH_2OSO_2NHCH_3$, $CH_2N(CH_3)_2$, $CH_2N(C_2H_5)_2$ oder $CH_2N(C_3H_7\text{-n})_2$ bedeutet.

4. Verbindungen der Formel I nach Anspruch 1, dadurch gekennzeichnet, dass Ar für die Gruppe

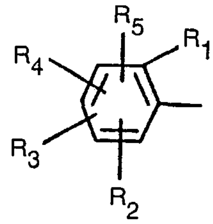

steht;

$R_1$ $C_1—C_3$-Alkyl, $C_1—C_3$-Alkoxy oder Halogen bedeutet,
$R_2$ für $NO_2$ oder $NH_2$ steht,
$R_3$ Wasserstoff, $C_1—C_3$-Alkyl, $C_1—C_3$-Alkoxy oder Halogen,
$R_4$ Wasserstoff oder $C_1—C_3$-Alkyl,
$R_5$ Wasserstoff oder $C_1—C_3$-Alkyl bedeuten, mit der Massgabe, dass stets beide ortho-Positionen im Anilinteil des Moleküls substituiert sind,
R Wasserstoff oder Methyl bedeutet,
W für $COOR_8$ steht,
$R_8$ für $C_1—C_3$-Alkyl steht,
B 2-Furyl, 2-Tetrahydrofuryl, β-($C_1—C_2$-Alkoxy)ethyl oder die Gruppe $CH_2Z$ bedeutet, wobei Z für 1H-1,2,4-Triazolyl, Methylsulfonyl, $X—R_{13}$ oder $OSO_2—R_{14}$ steht, worin X Sauerstoff oder Schwefel bedeutet, $R_{13}$ für eine Alkyl-, Alkenyl- oder Alkinylgruppe mit maximal 4 Kohlenstoffatomen steht und $R_{14}$ $C_1—C_3$-Alkyl oder $NH(C_1—C_3\text{-Alkyl})$ bedeutet.

5. Verbindungen der Formel I nach Anspruch 4, dadurch gekennzeichnet, dass $R_1$ für Methyl, Methoxy oder Halogen steht, $R_2$ $NO_2$ oder $NH_2$ bedeutet, $R_3$ für Wasserstoff, Methyl, Methoxy oder Halogen, $R_4$, $R_5$ und R unabhängig voneinander für Wasserstoff oder Methyl stehen, W für $COOR_8$ steht, $R_8$ für $C_1—C_3$-Alkyl steht und B 2-Furyl, 2-Tetrahydrofuryl, $CH_2O(C_1—C_3)\text{-Alkyl})$, $CH_2CH_2OCH_3$, 1H-1,2,4-Triazolylmethyl, $CH_2—SO_2CH_3$, $CH_2OSO_2CH_3$, $CH_2OCH_2CH=CH_2$, $CH_2OSO_2NHCH_3$ oder $CH_2OCH_2C\equiv CH$ bedeutet.

6. Verbindungen der Formel I nach Anspruch 5, dadurch gekennzeichnet, dass $R_1$ für Methyl, $R_2$ für $NO_2$ oder $NH_2$, $R_3$ für Wasserstoff, Methyl, Chlor oder Brom, $R_4$, $R_5$ und R unabhängig voneinander für Wasserstoff oder Methyl stehen und $R_8$ Methyl oder iso-Propyl bedeutet.

7. Verbindungen der Formel I nach Anspruch 1, dadurch gekennzeichnet, dass Ar für die Gruppe

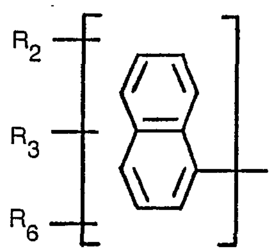

steht

$R_2$ für $NO_2$ oder $NH_2$ steht,
$R_3$ Wasserstoff, $C_1—C_3$-Alkyl, $C_1—C_3$-Alkoxy oder Halogen,
$R_6$ Wasserstoff, $C_1—C_3$-Alkyl oder Halogen bedeuten, mit der Massgabe, dass stets die β-Position im Molekül substituiert ist;
R Wasserstoff oder Methyl bedeutet,
W für $COOR_8$ steht,
$R_8$ für $C_1—C_3$-Alkyl steht,
B 2-Furyl, 2-Tetrahydrofuryl, β-($C_1—C_2$-Alkoxy)ethyl oder die Gruppe $CH_2Z$ bedeutet, wobei Z für 1H-1,2,4-Triazolyl, Sulfonylmethyl, $X—R_{13}$ oder $OSO_2—R_{14}$ steht, worin X Sauerstoff oder Schwefel bedeutet, $R_{13}$ für eine Alkyl-, Alkenyl- oder Alkinylgruppe mit maximal 4 Kohlenstoffatomen steht und $R_{14}$ $C_1—C_3$-Alkyl oder $NH(C_1—C_3\text{-Alkyl})$ bedeutet.

8. Verbindungen der Formel I nach Anspruch 7, dadurch gekennzeichnet, dass $R_2$ $NO_2$ oder $NH_2$ bedeutet, $R_3$ für Wasserstoff, Methyl, Methoxy oder Halogen, $R_6$ für Wasserstoff, Methyl oder Halogen stehen, R für Methyl oder Wasserstoff, W für $COOR_8$ steht, $R_8$ für $C_1—C_3$-Alkyl stehen und B 2-Furyl, 2-Tetrahydrofuryl, $CH_2O(C_1—C_3\text{-Alkyl})$, 1H-1,2,4-Triazolylmethyl, $CH_2SO_2CH_3$, $CH_2OSO_2CH_3$, $CH_2OCH_2CH=CH_2$, $CH_2SO_2NHCH_3$ oder $CH_2OCH_2C\equiv CH$ bedeutet.

9. Verbindungen der Formel I nach Anspruch 8, dadurch gekennzeichnet, dass $R_2$ für $NO_2$ oder $NH_2$, $R_3$ Wasserstoff, Methyl, Methoxy, Chlor oder Brom bedeutet, $R_6$ Wasserstoff, Methyl, Chlor oder Brom bedeutet, R für Methyl steht und $R_8$ Methyl oder iso-Propyl bedeutet.

10. Eine Verbindung der Formel I nach Anspruch 4, ausgewählt aus der Gruppe
N-(1'-Methoxycarbonyl-ethyl)-N-methoxyacetyl-2-methyl-6-aminoanilin,
N-(1'-Methoxycarbonyl-ethyl)-N-methoxyacetyl-2-methyl-6-nitroanilin,
N-(1'-Methoxycarbonyl-ethyl)-N-methoxyacetyl-2,6-dimethyl-3-nitroanilin,
N-(1'-Methoxycarbonyl-ethyl)-N-ethoxyacetyl-2,6-dimethyl-3-nitroanilin,
N-(1'-Isopropyloxycarbonyl-ethyl)-N-methoxyacetyl-2,6-dimethyl-3-nitroanilin,
N-(1'-Methoxycarbonyl-ethyl)-N-methoxyacetyl-2,6-dimethyl-3-aminoanilin,
N-(1'-Methoxycarbonyl-ethyl)-N-methoxyacetyl-2,3-dimethyl-6-aminoanilin,
N-(1'-Methoxycarbonyl-ethyl)-N-methoxyacetyl-2,3-dimethyl-6-nitroanilin,
N-(1'-Methoxycarbonyl-ethyl)-N-(2-furyl)-2-methyl-6-nitroanilin oder
N-(1'-Methoxycarbonyl-ethyl)-N-(2-tetrahydrofuryl)-2-methyl-6-nitroanilin.

11. Eine Verbindung der Formel I nach Anspruch 7, ausgewählt aus der Gruppe:
1N-(1'-Methoxycarbonyl-ethyl)-1N-methoxyacetyl-1,2-diamino-naphthalin
N-(1'-Methoxycarbonyl-ethyl)-N-methoxyacetyl-amino-2-nitro-naphthalin
N-(1'-Methoxycarbonyl-ethyl)-N-methoxyacetyl-amino-2-methyl-3-nitro-naphthalin
N-(1'-Methoxycarbonyl-ethyl)-N-ethoxyacetyl-amino-2-ethyl-3-nitro-naphthalin
N-(1'-Isopropyloxycarbonyl-ethyl)-N-methoxyacetyl-amino-2-methyl-3-nitro-naphthalin
1N-(1'-Methoxycarbonyl-ethyl)-1N-methoxyacetyl-2-methyl-1,3-diamino-naphthalin
1N-(1'-Methoxycarbonyl-ethyl)-1N-methoxyacetyl-3-methyl-1,2-diamino-naphthalin
N-(1'-Methoxycarbonyl-ethyl)-N-methoxyacetyl-amino-3-methyl-2-nitro-naphthalin
N-(1'-Methoxycarbonyl-ethyl)-N-(2-furyl)-amino-2-nitro-naphthalin
N-(1'-Methoxycarbonyl-ethyl)-N-(2-tetrahydrofuryl)-amino-2-nitro-naphthalin.

12. Verfahren zur Herstellung von Verbindungen der in Anspruch 1 definierten Formel I, dadurch gekennzeichnet, dass man wahlweise entweder ein bereits N-alkyliertes Arylamino-derivat der Formel II

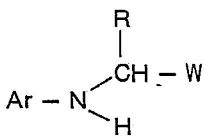

(II)

mit einer Carbonsäure der Formel III,

B—COOH (III)

ihrem Säurehalogenid, Säureanhydrid oder Ester bei Temperaturen zwischen 0° und +180°C N-acyliert, oder dass man ein bereits N-acyliertes Arylamin-derivat der Formel IV

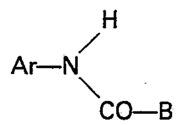

(IV)

mit Butyl-Lithium, Natriumhydrid oder einem Alkali-Carbonat aktiviert und mit einer Verbindung der Formel V

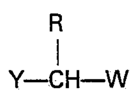

(V)

bei Temperaturen zwischen 0° und +180°C N-alkyliert, wobei die Substituenten Ar, R, W und B wie unter Formel I in Anspruch 1 definiert sind und Y für eine der üblichen Abgangsgruppen steht.

13. Mittel zur Bekämpfung und/oder Verhütung eines Befalls von schädlichen Mikroorganismen, dadurch gekennzeichnet, dass es als mindestens eine aktive Komponente einen Wirkstoff der Formel I nach Anspruch 1 enthält.

14. Mittel nach Anspruch 13, dadurch gekennzeichnet, dass es 0,01 bis 90 Gewichtsprozent eines Wirkstoffes der Formel I nach Anspruch 1, 10 bis 99,99 Gewichtsprozent an Zuschlagstoffen und 0 bis 33 Gewichtsprozent eines Tensides enthält.

15. Mittel nach einem der Ansprüche 13 und 14, dadurch gekennzeichnet, dass es als Aktivsubstanz eine der Verbindungen der Formel I gemäss einem der Ansprüche 2 bis 11 enthält.

16. Verfahren zur Herstellung von Mitteln zur Bekämpfung und/oder Verhütung eines Befalls von Mikroorganismen, dadurch gekennzeichnet, dass man eine Verbindung der Formel I nach einem der Ansprüche 1 bis 11, zusammen mit geeigneten Trägerstoffen und/oder Tensiden innig vermischt.

17. Verwendung von Verbindungen der Formel I nach Anspruch 1, zur Bekämpfung und/oder Verhütung eines Befalls von Pflanzen durch phytopathogene Mikroorganismen.

18. Verwendung nach Anspruch 17, von Verbindungen der Formel I, gemäss einem der Ansprüche 2 bis 11.

19. Verwendung nach Anspruch 17, dadurch gekennzeichnet, dass es sich bei den Mikroorganismen um phytopathogene Pilze handelt.

20. Verfahren zur Bekämpfung und/oder Verhütung eines Befalls der Pflanzen durch phytopathogene Pilze, dadurch gekennzeichnet, dass man eine Verbindung der Formel I nach einem der Ansprüche 1 bis 11, in fein verteiler Form auf die Pflanze oder deren Standort appliziert.

**Patentansprüche für den Vertragsstaat: AT**

1. Mittel zur Bekämpfung und/oder Verhütung eines Befalls von schädlichen Mikroorganismen, dadurch gekennzeichnet, dass es zusammen mit üblichen Trägerstoffen und applikationsfördernden Zusätzen als mindestens eine aktive Komponente einen Wirkstoff der Formel I

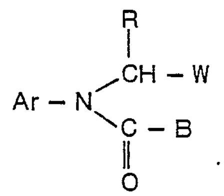

(I)

enthält, worin

R für Wasserstoff oder Methyl steht;

Ar eine der aromatischen Gruppen

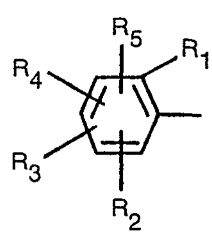

oder

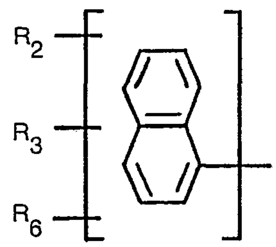

repräsentiert, wobei

$R_1$ $C_1$—$C_3$-Alkyl, $C_1$—$C_3$-Alkoxy oder Halogen bedeutet;

$R_2$ für $NO_2$ oder $NH_2$ steht;

$R_3$ Wasserstoff, $C_1$—$C_3$-Alkyl, $C_1$—$C_3$-Alkoxy oder Halogen;

$R_4$ Wasserstoff oder $C_1$—$C_3$-Alkyl;

$R_5$ Wasserstoff oder $C_1$—$C_3$-Alkyl;

$R_6$Wasserstoff, $C_1$—$C_3$-Alkyl oder Halogen bedeutet;

W für Cyano, $—COOR_8$, $—C(R_9)=C(R_{10})(R_{11})$ oder $—C{\equiv}C—R_{12}$ steht, wobei

$R_8$ $C_1$—$C_3$-Alkyl;

$R_9$, $R_{10}$, $R_{11}$ und $R_{12}$ unabhängig voneinander Wasserstoff, $C_1$—$C_3$-Alkyl oder Halogen bedeuten und

B für $C_3$—$C_4$-Alkyl, $C_2$—$C_4$-Alkenyl, Cyclopropyl, 2-Furyl, 2-Tetrahydrofuryl, β-($C_1$—$C_2$-Alkoxy)ether oder die Gruppe $CH_2Z$ steht, wobei

Z für 1H-1,2,4-Triazolyl, Methylsulfonyl, X—$R_{13}$, $OSO_2$—$R_{14}$ oder —$N(R_{15})(R_{16})$ steht, wobei

X Sauerstoff oder Schwefel bedeutet;

$R_{13}$ für eine Alkyl-, Alkenyl- oder Alkinylgruppe mit maximal 4 Kohlenstoffatomen steht;

$R_{14}$ $C_1$—$C_3$-Alkyl oder NH($C_1$—$C_3$-Alkyl) bedeutet und

$R_{15}$ und $R_{16}$ unabhängig voneinander für $C_1$—$C_3$-Alkyl stehen.

2. Mittel nach Anspruch 1, dadurch gekennzeichnet, dass in Formel I R für Wasserstoff oder Methyl steht, Ar für die Gruppe

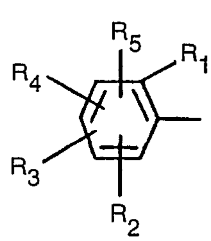

oder

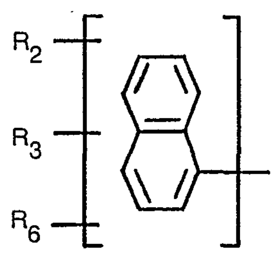

steht; $R_1$ für Methyl, Ethyl, Methoxy, Ethoxy oder Halogen steht; $R_2$ Nitro oder Amino; $R_3$ und $R_6$ unabhängig voneinander Wasserstoff, Methyl, Ethyl, Methoxy, Ethoxy oder Halogen bedeuten; $R_4$ und $R_5$ unabhängig voneinander für Wasserstoff, Methyl oder Ethyl stehen; W für CN, $COOR_8$, $—C(R_9{=}C(R_{10})(R_{11})$ oder $—C{\equiv}C—R_{12}$ steht, wobei $R_8$ $C_1—C_3$-Alkyl bedeutet; $R_9$, $R_{10}$, $R_{11}$ und $R_{12}$ unabhängig voneinander für Wasserstoff, Methyl, Chlor oder Brom stehen und B für $C_1—C_3$-Alkyl, $C_2—C_4$-Alkenyl, Cyclopropyl, 2-Furyl, 2-Tetrahydrofuryl, $CH_2O(C_1—C_3$-Alkyl), 1H-Triazolylmethyl, $CH_2SO_2CH_3$, $CH_2OSO_2CH_3$, $CH_2OCH_2CH{=}CH_2$, $CH_2OCH_2C{\equiv}CH$, $CH_2OSO_2NHCH_3$, $CH_2N(CH_3)_2$, $CH_2N(C_2H_5)_2$ oder $CH_2N(C_3H_7\text{-n})_2$ steht.

3. Mittel nach Anspruch 2, dadurch gekennzeichnet, dass in Formel I R Methyl bedeutet; $R_1$ für Methyl, Methoxy, Chlor oder Brom steht; $R_3$ und $R_6$ unabhängig voneinander Wasserstoff, Methyl, Chlor oder Brom bedeuten; $R_4$ und $R_5$ unabhängig voneinander für Wasserstoff oder Methyl stehen; W für CN, $COOR_8$, $—C(R_9){=}C(R_{10})(R_{11})$ oder $—C{\equiv}C—R_{12}$ steht, wobei $R_9$, $R_{10}$, $R_{11}$ und $R_{12}$ unabhängig voneinander für Wasserstoff, Methyl oder Chlor stehen und B 2-Furyl, 2-Tetrahydrofuryl, $CH_2O(C_1—C_3$-Alkyl), 1H-1,2,4-Triazolylmethyl, $CH_2SO_2CH_3$, $CH_2OSO_2CH_3$, $CH_2OCH_2CH{=}CH_2$, $CH_2OCH_2C{\equiv}CH$, $CH_2OSO_2NHCH_3$, $CH_2N(CH_3)_2$, $CH_2N(C_2H_5)_2$ oder $CH_2N(C_3H_7\text{-n})_2$ bedeutet.

4. Mittel nach Anspruch 1, dadurch gekennzeichnet, dass in Formel I
Ar für die Gruppe

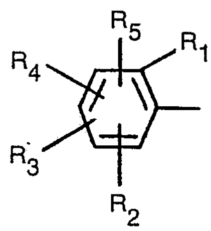

steht;

$R_1$ $C_1—C_3$-Alkyl, $C_1—C_3$-Alkoxy oder Halogen bedeutet,
$R_2$ für $NO_2$ oder $NH_2$ steht,
$R_3$ Wasserstoff, $C_1—C_3$-Alkyl, $C_1—C_3$-Alkoxy oder Halogen,
$R_4$ Wasserstoff oder $C_1—C_3$-Alkyl,
$R_5$ Wasserstoff oder $C_1—C_3$-Alkyl bedeuten, mit der Massgabe, dass stets beide ortho-Positionen im Anilinteil des Moleküls substituiert sind,
R Wasserstoff oder Methyl bedeutet,
W für $COOR_8$ steht,
$R_8$ für $C_1—C_3$-Alkyl steht,
B 2-Furyl, 2-Tetrahydrofuryl, β-($C_1—C_2$-Alkoxy)ethyl oder die Gruppe $CH_2Z$ bedeutet, wobei Z für 1H-1,2,4-Triazolyl, Methylsulfonyl, $X—R_{13}$ oder $OSO_2—R_{14}$ steht, worin X Sauerstoff oder Schwefel bedeutet, $R_{13}$ für eine Alkyl-, Alkenyl- oder Alkinylgruppe mit maximal 4 Kohlenstoffatomen steht und $R_{14}$ $C_1—C_3$-Alkyl oder NH($C_1—C_3$-Alkyl) bedeutet.

5. Mittel nach Anspruch 4, dadurch gekennzeichnet, dass in Formel I $R_1$ für Methyl, Methoxy oder Halogen steht, $R_2$ $NO_2$ oder $NH_2$ bedeutet, $R_3$ für Wasserstoff, Methyl, Methoxy oder Halogen, $R_4$, $R_5$ und R unabhängig voneinander für Wasserstoff oder Methyl stehen, W für $COOR_8$ steht, $R_8$ für $C_1—C_3$-Alkyl steht und B 2-Furyl, 2-Tetrahydrofuryl, $CH_2O(C_1—C_3)$-Alkyl), $CH_2CH_2OCH_3$, 1H-1,2,4-Triazolylmethyl, $CH_2—SO_2CH_3$, $CH_2OSO_2CH_3$, $CH_2OCH_2CH{=}CH_2$, $CH_2OSO_2NHCH_3$ oder $CH_2OCH_2C{\equiv}CH$ bedeutet.

6. Mittel nach Anspruch 5, dadurch gekennzeichnet, dass in Formel I $R_1$ für Methyl, $R_2$ für $NO_2$ oder $NH_2$, $R_3$ für Wasserstoff, Methyl, Chlor oder Brom, $R_4$, $R_5$ und R unabhängig voneinander für Wasserstoff oder Methyl stehen und $R_8$ Methyl oder iso-Propyl bedeutet.

7. Mittel nach Anspruch 1, dadurch gekennzeichnet, dass in Formel I Ar für die Gruppe

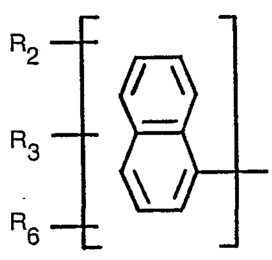

steht

$R_2$ für $NO_2$ oder $NH_2$ steht,
$R_3$ Wasserstoff, $C_1—C_3$-Alkyl, $C_1—C_3$-Alkoxy oder Halogen,
$R_6$ Wasserstoff, $C_1—C_3$-Alkyl oder Halogen bedeuten, mit der Massgabe, dass stets die β-Position im Molekül substituiert ist;
R Wasserstoff oder Methyl bedeutet,
W für $COOR_8$ steht,
$R_8$ für $C_1—C_3$-Alkyl steht,
B 2-Furyl, 2-Tetrahydrofuryl, β-($C_1—C_2$-Alkoxy)ethyl oder die Gruppe $CH_2Z$ bedeutet, wobei Z für 1H-

1,2,4-Triazolyl, Sulfonylmethyl, X—$R_{13}$ oder $OSO_2$—$R_{14}$ steht, worin X Sauerstoff oder Schwefel bedeutet, $R_{13}$ für eine Alkyl-, Alkenyl- oder Alkinylgruppe mit maximal 4 Kohlenstoffatomen steht und $R_{14}$ $C_1$—$C_3$-Alkyl oder NH($C_1$—$C_3$-Alkyl) bedeutet.

8. Mittel nach Anspruch 7, dadurch gekennzeichnet, dass in Formel I $R_2$ $NO_2$ oder $NH_2$ bedeutet, $R_3$ für Wasserstoff, Methyl, Methoxy oder Halogen, $R_6$ für Wasserstoff, Methyl oder Halogen stehen, R für Methyl oder Wasserstoff, W für $COOR_8$ steht, $R_8$ für $C_1$—$C_3$-Alkyl stehen und B 2-Furyl, 2-Tetrahydrofuryl, $CH_2O$($C_1$—$C_3$-Alkyl), 1H-1,2,4-Triazolylmethyl, $CH_2SO_2CH_3$, $CH_2OSO_2CH_3$, $CH_2OCH_2CH{=}CH_2$, $CH_2SO_2NHCH_3$ oder $CH_2OCH_2C{\equiv}CH$ bedeutet.

9. Mittel nach Anspruch 8 dadurch gekennzeichnet, dass in Formel I $R_2$ für $NO_2$ oder $NH_2$, $R_3$ Wasserstoff, Methyl, Methoxy, Chlor oder Brom bedeutet, $R_6$ Wasserstoff, Methyl, Chlor oder Brom bedeutet, R für Methyl steht und $R_8$ Methyl oder iso-Propyl bedeutet.

10. Mittel nach Anspruch 4, dadurch gekennzeichnet, dass der Wirkstoff aus der Gruppe
N-(1'-Methoxycarbonyl-ethyl)-N-methoxyacetyl-2-methyl-6-aminoanilin,
N-(1'-Methoxycarbonyl-ethyl)-N-methoxyacetyl-2-methyl-6-nitroanilin,
N-(1'-Methoxycarbonyl-ethyl)-N-methoxyacetyl-2,6-dimethyl-3-nitroanilin,
N-(1'-Methoxycarbonyl-ethyl)-N-ethoxyacetyl-2,6-dimethyl-3-nitroanilin,
N-(1'-Isopropyloxycarbonyl-ethyl)-N-methoxyacetyl-2,6-dimethyl-3-nitroanilin,
N-(1'-Methoxycarbonyl-ethyl)-N-methoxyacetyl-2,6-dimethyl-3-aminoanilin,
N-(1'-Methoxycarbonyl-ethyl)-N-methoxyacetyl-2,3-dimethyl-6-aminoanilin,
N-(1'-Methoxycarbonyl-ethyl)-N-methoxyacetyl-2,3-dimethyl-6-nitroanilin,
N-(1'-Methoxycarbonyl-ethyl)-N-(2-furyl)-2-methyl-6-nitroanilin oder
N-(1'-Methoxycarbonyl-ethyl)-N-(2-tetrahydrofuryl)-2-methyl-6-nitroanilin ausgewählt wird.

11. Mittel nach Anspruch 7, dadurch gekennzeichnet, dass der Wirkstoff aus der Gruppe:
1N-(1'-Methoxycarbonyl-ethyl)-1N-methoxyacetyl-1,2-diamino-naphthalin
N-(1'-Methoxycarbonyl-ethyl)-N-methoxyacetyl-amino-2-nitro-naphthalin
N-(1'-Methoxycarbonyl-ethyl)-N-methoxyacetyl-amino-2-methyl-3-nitro-naphthalin
N-(1'-Methoxycarbonyl-ethyl)-N-ethoxyacetyl-amino-2-ethyl-3-nitro-naphthalin
N-(1'-Isopropyloxycarbonyl-ethyl)-N-methoxyacetyl-amino-2-methyl-3-nitro-naphthalin
1N-(1'-Methoxycarbonyl-ethyl)-1N-methoxyacetyl-2-methyl-1,3-diamino-naphthalin
1N-(1'-Methoxycarbonyl-ethyl)-1N-methoxyacetyl-3-methyl-1,2-diamino-naphthalin
N-(1'-Methoxycarbonyl-ethyl)-N-methoxyacetyl-amino-3-methyl-2-nitro-naphthalin
N-(1'-Methoxycarbonyl-ethyl)-N-(2-furyl)-amino-2-nitro-naphthalin
N-(1'-Methoxycarbonyl-ethyl)-N-(2-tetrahydrofuryl)-amino-2-nitro-naphthalin ausgewählt wird.

12. Verfahren zur Herstellung von Verbindungen der in Anspruch 1 definierten Formel I, dadurch gekennzeichnet, dass man wahlweise entweder ein bereits N-alkyliertes Arylamino-derivat der Formel II

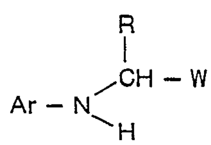

(II)

mit einer Carbonsäure der Formel III,

$$B{-}COOH \qquad (III)$$

ihrem Säurehalogenid, Säureanhydrid oder Ester bei Temperaturen zwischen 0° und +180°C N-acyliert, oder dass man ein bereits N-acyliertes Arylamin-derivat der Formel IV

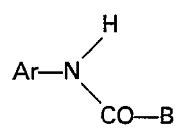

(IV)

mit Butyl-Lithium, Natriumhydrid oder einem Alkali-Carbonat aktiviert und mit einer Verbindung der Formel V

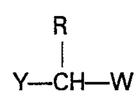

(V)

bei Temperaturen zwischen 0° und +180°C N-alkyliert, wobei die Substituenten Ar, R, W und B wie unter Formel I in Anspruch 1 definiert sind und Y für eine der üblichen Abgangsgruppen steht.

13. Verfahren nach Anspruch 12, dadruch gekennzeichnet, dass es sich bei dem Säurehalogenid um ein -chlorid oder -bromid handelt.

14. Verfharen nach Anspruch 12, dadurch gekennzeichnet, dass die N-Acylierung in Gegenwart eines säurebindenden Mittels oder eines Kondensationsmittels durchgeführt wird.

15. Verfahren nach Anspruch 12, dadurch gekennzeichnet, dass es in Gegenwart eines reaktionsinerten Lösungsmittels oder Lösungsmittelgemisches durchgeführt wird.

16. Verwendung von Verbindungen der Formel I nach Anspruch 1, zur Bekämpfung und/oder Verhütung eines Befalls von Pflanzen durch phytopathogene Mikroorganismen.

17. Verwendung nach Anspruch 16, von Verbindungen der Formel I, gemäss einem der Ansprüche 2 bis 11.

18. Verwendung nach Anspruch 16, dadurch gekennzeichnet, dass es sich bei den Mikroorganismen um phytopathogene Pilze handelt.

19. Verfahren zur Bekämpfung und/oder Verhütung eines Befalls der Pflanzen durch phytopathogene Pilze, dadurch gekennzeichnet, dass man eine Verbindung der Formel I nach einem der Ansprüche 1 bis 11, in fein verteilter Form auf die Pflanze oder deren Standort appliziert.

**Claims for the Contracting States: BE CH LI DE FR GB IT NL SE**

1. A compound of the formula I

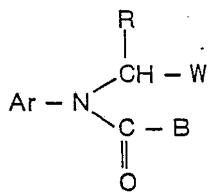
(I)

wherein
R is hydrogen or methyl,
Ar isd one of the aromatic groups

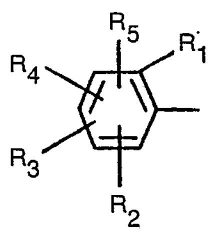
or
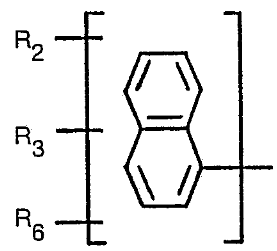

wherein
$R_1$ is $C_1$—$C_3$alkyl, $C_1$—$C_3$alkoxy or halogen,
$R_2$ is $NO_2$ or $NH_2$,
$R_3$ is hydrogen, $C_1$—$C_3$alkyl, $C_1$—$C_3$alkoxy or halogen,
$R_4$ is hydrogen or $C_1$—$C_3$alkyl,
$R_5$ is hydrogen or $C_1$—$C_3$alkyl, and
$R_6$ is hydrogen, $C_1$—$C_3$-alkyl or halogen;
W is cyano, —$COOR_8$, —$C(R_9)$=$C(R_{10})(R_{11})$ or —C≡C—$R_{12}$, wherein
$R_8$ is $C_1$—$C_3$alkyl,
$R_9$, $R_{10}$, $R_{11}$ and $R_{12}$ are each independently hydrogen, $C_1$—$C_3$alkyl or halogen;
B is $C_3$—$C_4$ alkyl, $C_2$—$C_4$alkenyl, cyclopropyl, 2-furyl 2-tetrahydrofuryl, β-($C_1$—$C_2$alkoxy)ethyl or the group $CH_2Z$, in which
Z is 1H-1,2,4-triazolyl, methylsulfonyl, X—$R_{13}$, $OSO_2$—$R_{14}$ or —$N(R_{15})(R_{16})$, in which
X is oxygen or sulfur,
$R_{13}$ is an alkyl, alkenyl or alkynyl group, each containing at most 4 carbon atoms.
$R_{14}$ is $C_1$—$C_3$alkyl or NH($C_1$—$C_3$alkyl), and
$R_{15}$ and $R_{16}$ are each independently $C_1$—$C_3$alkyl.

2. A compound of the formula I according to claim 1, wherein R is hydrogen or methyl, Ar is the group

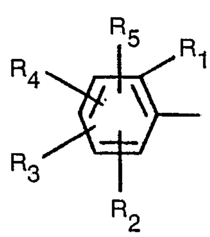
or
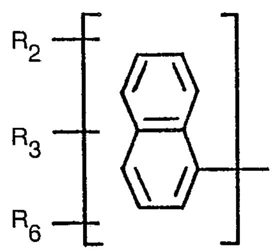

$R_1$ is methyl, ethyl, methoxy, ethoxy or halogen; $R_2$ is nitro or amino; each of $R_3$ and $R_6$ independently is

hydrogen, methyl, ethyl, methoxy, ethoxy or halogen; each of $R_4$ and $R_5$ independently is hydrogen, methyl or ethyl; W is CN, $COOR_8$, $—C(R_9=C(R_{10})(R_{11})$ or $—C{\equiv}C—R_{12}$, in which $R_8$ is $C_1—C_3$alkyl; each of $R_9$, $R_{10}$, $R_{11}$ and $R_{12}$ independently is hydrogen, methyl, chlorine or bromine; and B is $C_1—C_3$alkyl, $C_2—C_4$alkenyl, cyclopropyl, 2-furyl, 2-tetrahydrofuryl, $CH_2O(C_1—C_3alkyl)$, 1H-1,2,4-triazolylmethyl, $CH_2SO_2CH_3$, $CH_2OSO_2CH_3$, $CH_2OCH_2CH{=}CH_2$, $CH_2OCH_2C{\equiv}CH$, $CH_2OSO_2NHCH_3$, $CH_2N(CH_3)_2$, $CH_2N(C_2H_5)_2$ or $CH_2N(C_3H_7\text{-}n)_2$.

3. A compound of the formula I according to claim 2, wherein R is methyl; $R_1$ is methyl, methoxy, chlorine or bromine; each of $R_3$ and $R_6$ independently is hydrogen, methyl, chlorine or bromine; each of $R_4$ and $R_5$ independently is hydrogen or methyl; W is CN, $COOR_8$, $—C(R_9){=}C(R_{10})(R_{11})$ or $—C{\equiv}C—R_{12}$, wherein each of $R_9$, $R_{10}$, $R_{11}$ and $R_{12}$ independently is hydrogen, methyl or chlorine; and B is 2-furyl, 2-tetrahydrofuryl, $CH_2O(C_1—C_3alkyl)$, 1H-1,2,4-triazolylmethyl, $CH_2SO_2CH_3$, $CH_2OSO_2CH_3$, $CH_2OCH_2CH{=}CH_2$, $CH_2OCH_2C{\equiv}CH$, $CH_2OSO_2NHCH_3$, $CH_2N(CH_3)_2$, $CH_2N(C_2H_5)_2$ or $CH_2N(C_3H_7\text{-}n)_2$.

4. A compound of formula I according to claim 1, wherein Ar is the group

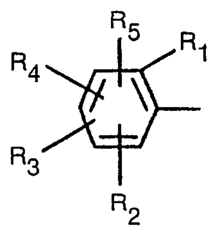

$R_1$ is $C_1—C_3$alkyl, $C_1—C_3$alkoxy or halogen,
$R_2$ is $NO_2$ or $NH_2$,
$R_3$ is hydrogen, $C_1—C_3$alkyl, $C_1—C_3$alkoxy or halogen,
$R_4$ is hydrogen or $C_1—C_3$alkyl,
$R_5$ is hydrogen or $C_1—C_3$alkyl, with the proviso that both ortho-positions in the aniline part of the molecule are always substituted,
R is hydrogen or methyl,
W is $COOR_8$,
$R_8$ is $C_1—C_3$alkyl,
B is 2-furyl, 2-tetrahydrofuryl, β-($C_1—C_2$alkoxy)ethyl or the group $CH_2Z$, in which Z is 1H-1,2,4-triazolyl, methylsulfonyl, $X—R_{13}$ or $OSO_2—R_{14}$, wherein X is oxygen or sulfur, $R_{13}$ is an alkyl, alkenyl or alkynyl group, each of which contains at most 4 carbon atoms, and $R_{14}$ is $C_1—C_3$alkyl or $NH(C_1—C_3$ alkyl).

5. A compound of the formula I according to claim 4, wherein $R_1$ is methyl, methoxy or halogen; $R_2$ is $NO_2$ or $NH_2$; $R_3$ is hydrogen, methyl, methoxy or halogen; each of $R_4$, $R_5$ and R independently is hydrogen or methyl; W is $COOR_8$, wherein $R_8$ is $C_1—C_3$alkyl; and B is 2-furyl, 2-tetrahydrofuryl, $CH_2O(C_1—C_3)$alkyl, $CH_2CH_2OCH_3$, 1H-1,2,4-triazolylmethyl, $CH_2—SO_2CH_3$, $CH_2OSO_2CH_3$, $CH_2OCH_2CH{=}CH_2$, $CH_2OSO_2NHCH_3$ or $CH_2OCH_2C{\equiv}CH$.

6. A compound of the formula I according to claim 5, wherein $R_1$ is methyl, $R_2$ is $NO_2$ or $NH_2$, $R_3$ is hydrogen, methyl, chlorine or bromine, each of $R_4$, $R_5$ and R independently is hydrogen or methyl, and $R_8$ is methyl or isopropyl.

7. A compound of the formula I according to claim 1, wherein Ar is the group

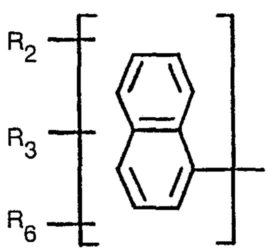

$R_2$ is $NO_2$ or $NH_2$,
$R_3$ is hydrogen, $C_1—C_3$alkyl, $C_1—C_3$alkoxy or halogen,
$R_6$ is hydrogen, $C_1—C_3$alkyl or halogen, with the proviso that the β-position in the molecule is always substituted,
R is hydrogen or methyl,
W is $COOR_8$,
$R_8$ is $C_1—C_3$alkyl,
B is 2-furyl, 2-tetrahydrofuryl, β-($C_1—C_2$alkoxy)ethyl or the group $CH_2Z$, in which Z is 1H-1,2,4-triazolyl, sulfonylmethyl, $X—R_{13}$ or $OSO_2—R_{14}$, wherein X is oxygen or sulfur, $R_{13}$ is an alkyl, alkenyl or alkynyl group, each containing at most 4 carbon atoms, and $R_{14}$ is $C_1—C_3$alkyl or $NH(C_1—C_3alkyl)$.

8. A compound of the formula I according to claim 7, wherein $R_2$ is $NO_2$ or $NH_2$; $R_3$ is hydrogen, methyl, methoxy or halogen; $R_6$ is hydrogen, methyl or halogen; R is methyl or hydrogen; W is $COOR_8$, wherein $R_8$

is $C_1$—$C_3$alkyl; and B is 2-furyl, 2-tetrahydrofuryl, $CH_2O(C_1—C_3alkyl)$, 1H-1,2,4-triazolylmethyl, $CH_2SO_2CH_3$, $CH_2OSO_2CH_3$, $CH_2OCH_2CH=CH_2$, $CH_2SO_2NHCH_3$ or $CH_2OCH_2C{\equiv}CH$.

9. A compound of the formula I according to claim 8, wherein $R_2$ is $NO_2$ or $NH_2$; $R_3$ is hydrogen, methyl, methoxy, chlorine or bromine; $R_6$ is hydrogen, methyl, chlorine or bromine; R is methyl; and $R_8$ is methyl or isopropyl.

10. A compound of the formula I according to claim 4 selected from the group consisting of
N-(1'-methoxycarbonylethyl)-N-methoxyacetyl-2-methyl-6-aminoaniline,
N-(1'-methoxycarbonylethyl)-N-methoxyacetyl-2-methyl-6-nitroaniline,
N-(1'-methoxycarbonylethyl)-N-methoxyacetyl-2,6-dimethyl-3-nitroaniline,
N-(1'-methoxycarbonylethyl)-N-ethoxyacetyl-2,6-dimethyl-3-nitroaniline,
N-(1'-isopropyloxycarbonylethyl)-N-methoxyacetyl-2,6-dimethyl-3-nitroaniline,
N-(1'-methoxycarbonylethyl)-N-methoxyacetyl-2,6-dimethyl-3-aminoaniline,
N-(1'-methoxycarbonylethyl)-N-methoxyacetyl-2,3-dimethyl-6-aminoaniline,
N-(1'-methoxycarbonylethyl)-N-methoxyacetyl-2,3-dimethyl-6-nitroaniline,
N-(1'-methoxycarbonylethyl)-N-(2-furyl)-2-methyl-6-nitroaniline and
N-(1'-methoxycarbonylethyl)-N-(2-tetrahydrofuryl)-2-methyl-6-nitroaniline.

11. A compound of the formula I according to claim 7 selected from the group consisting of
1N-(1'-methoxycarbonylethyl)-1N-methoxyacetyl-1,2-diaminonaphthalene,
N-(1'-methoxycarbonylethyl)-N-methoxyacetylamino-2-nitronaphthalene,
N-(1'-methoxycarbonylethyl)-N-methoxyacetylamino-2-methyl-3-nitronaphthalene,
N-(1'-methoxycarbonylethyl)-N-ethoxyacetylamino-2-ethyl-3-nitronaphthalene,
N-(1'-isopropyloxycarbonylethyl)-N-methoxyacetylamino-2-methyl-3-nitronaphthalene,
1N-(1'-methoxycarbonylethyl)-1N-methoxyacetyl-2-methyl-1,3-diaminonaphthalene,
1N-(1'-methoxycarbonylethyl)-1N-methoxyacetyl-3-methyl-1,2-diaminonaphthalene,
N-(1'-methoxycarbonylethyl)-N-methoxyacetyl-amino-3-methyl-2-nitronaphthalene,
N-(1'-methoxycarbonylethyl)-N-(2-furyl)amino-2-nitronaphthalene and
N-(1'-methoxycarbonylethyl)-N-(2-tetrahydrofuryl)-amino-2-nitronaphthalene.

12. A process for the production of a compound of the formula I as defined in claim 1, which process comprises N-acylating either an already N-alkylated arylamino derivate of the formula II

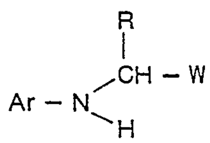

(II)

with a carboxylic acid of the formula III

B—COOH (III)

or an acid halide, acid anhydride or ester thereof, in the temperature range from 0° to 180°C, or activating an already N-acylated arylamino derivative of the formula IV

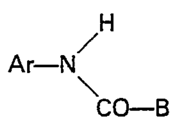

(IV)

with butyl lithium, sodium hydride or an alkali carbonate, and N-alkylating said compound with a compound of the formula V

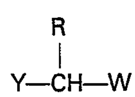

(V)

in the temperature range from 0° to 180°C, in which formulae II, III, IV and V above the substituents Ar, R, W and B are as defined for formula I in claim 1 and Y is one of the customary leaving groups.

13. A composition for controlling and/or preventing attack by harmful microorganisms, which composition contains, as active ingredient, at least one compound of the formula I according to claim 1.

14. A composition according to claim 13, which contains 0.01 to 90% by weight of a compound of the formula I according to claim 1, 10 to 99.99% by weight of adjuvants, and 0 to 33% by weight of a surfactant.

15. A composition according to either of claims 13 or 14, which contains a compound of the formula I according to any one of claims 2 to 11.

16. A process for the preparation of a composition for controlling and/or preventing attack by microorganisms, which process comprises homogenously mixing a compound of the formula I according to any one of claims 1 to 11 with suitable carriers and/or surfactants.

17. A method of controlling and/or protecting plants from attack by phytopathogenic microorganisms,

which method comprises applying to said plants or to the locus thereof a microbicidally effective amount of a compound of the formula I according to claim 1.

18. A method according to claim 17 which comprises the use of a compound of the formula I according to any one of claims 2 to 11.

19. A method according to claim 17, wherein the microorganisms to be controlled are phytopathogenic fungi.

20. A method of controlling and/or protecting plants from attack by phytopathogenic fungi, which method comprises applying to said plants or to the locus thereof a compound of the formula I according to any one of claims 1 to 11 in finely dispersed form.

**Claims for the Contracting State: AT**

1. A composition for controlling and/or preventing attack by harmful microorganisms, which composition contains, as active ingredient, at least one compound of the formula I

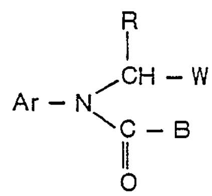

(I)

wherein

R is hydrogen or methyl,

Ar is one of the aromatic groups

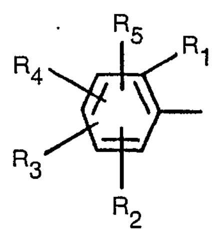

or

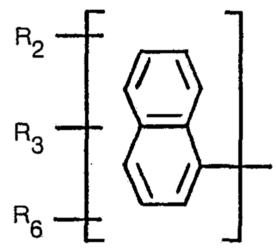

wherein

$R_1$ is $C_1$—$C_3$alkyl, $C_1$—$C_3$alkoxy or halogen,

$R_2$ is $NO_2$ or $NH_2$,

$R_3$ is hydrogen, $C_1$—$C_3$alkyl, $C_1$—$C_3$alkoxy or halogen,

$R_4$ is hydrogen or $C_1$—$C_3$alkyl,

$R_5$ is hydrogen or $C_1$—$C_3$alkyl, and

$R_6$ is hydrogen, $C_1$—$C_3$-alkyl or halogen;

W is cyano, —$COOR_8$, —$C(R_9)$=$C(R_{10})(R_{11})$ or —C≡C—$R_{12}$, wherein

$R_8$ is $C_1$—$C_3$alkyl,

$R_9$, $R_{10}$, $R_{11}$ and $R_{12}$ are each independently hydrogen, $C_1$—$C_3$alkyl or halogen;

B is $C_3$—$C_4$ alkyl, $C_2$—$C_4$alkenyl, cyclopropyl, 2-furyl 2-tetrahydrofuryl, β-($C_1$—$C_2$alkoxy)ethyl or the group $CH_2Z$, in which

Z is 1H-1,2,4-triazolyl, methylsulfonyl, X—$R_{13}$, $OSO_2$—$R_{14}$ or —$N(R_{15})(R_{16})$, in which

X is oxygen or sulfur,

$R_{13}$ is an alkyl, alkenyl or alkynyl group, each containing at most 4 carbon atoms.

$R_{14}$ is $C_1$—$C_3$alkyl or NH($C_1$—$C_3$alkyl), and

$R_{15}$ and $R_{16}$ are each independently $C_1$—$C_3$alkyl, together with customary carriers and application promotors.

2. A composition according to claim 1, wherein in formula I R is hydrogen or methyl, Ar is the group

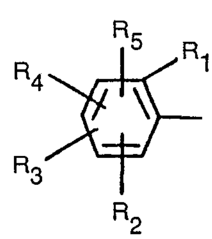

or

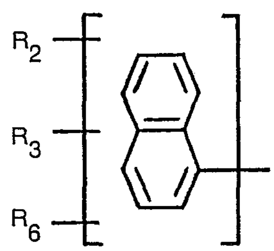

$R_1$ is methyl, ethyl, methoxy, ethoxy or halogen; $R_2$ is nitro or amino; each of $R_3$ and $R_6$ independently is hydrogen, methyl, ethyl, methoxy, ethoxy or halogen; each of $R_4$ and $R_5$ independently is hydrogen, methyl or ethyl; W is CN, $COOR_8$, $—C(R_9=C(R_{10})(R_{11})$ or $—C{\equiv}C—R_{12}$, in which $R_8$ is $C_1—C_3$alkyl; each of $R_9$, $R_{10}$, $R_{11}$ and $R_{12}$ independently is hydrogen, methyl, chlorine or bromine; and B is $C_1—C_3$alkyl, $C_2C_4$alkenyl, cyclopropyl, 2-furyl, 2-tetrahydrofuryl, $CH_2O(C_1—C_3alkyl)$, 1H-1,2,4-triazolylmethyl, $CH_2SO_2CH_3$, $CH_2OSO_2CH_3$, $CH_2OCH_2CH=CH_2$, $CH_2OCH_2C{\equiv}CH$, $CH_2OSO_2NHCH_3$, $CH_2N(CH_3)_2$, $CH_2N(C_2H_5)_2$ or $CH_2N(C_3H_7\text{-}n)_2$.

3. A composition according to claim 2, wherein in formula I R is methyl; $R_1$ is methyl, methoxy, chlorine or bromine; each of $R_3$ and $R_6$ independently is hydrogen, methyl, chlorine or bromine; each of $R_4$ and $R_5$ independently is hydrogen or methyl; W is CN, $COOR_8$, $—C(R_9)=C(R_{10})(R_{11})$ or $—C{\equiv}C—R_{12}$, wherein each of $R_9$, $R_{10}$, $R_{11}$ and $R_{12}$ independently is hydrogen, methyl or chlorine; and B is 2-furyl, 2-tetrahydrofuryl, $CH_2O(C_1—C_3alkyl)$, 1H-1,2,4-triazolylmethyl, $CH_2SO_2CH_3$, $CH_2OSO_2CH_3$, $CH_2OCH_2CH=CH_2$, $CH_2OCH_2C{\equiv}CH$, $CH_2OSO_2NHCH_3$, $CH_2N(CH_3)_2$, $CH_2N(C_2H_5)_2$ or $CH_2N(C_3H_7\text{-}n)_2$.

4. A composition according to claim 1, wherein in formula I Ar is the group

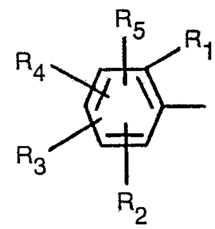

$R_1$ is $C_1—C_3$alkyl, $C_1—C_3$alkoxy or halogen,
$R_2$ is $NO_2$ or $NH_2$,
$R_3$ is hydrogen, $C_1—C_3$alkyl, $C_1—C_3$alkoxy or halogen,
$R_4$ is hydrogen or $C_1—C_3$alkyl,
$R_5$ is hydrogen or $C_1—C_3$alkyl, with the proviso that both ortho-positions in the aniline part of the molecule are always substituted,
R is hydrogen or methyl,
W is $COOR_8$,
$R_8$ is $C_1—C_3$alkyl,
B is 2-furyl, 2-tetrahydrofuryl, β-($C_1—C_2$alkoxy)ethyl or the group $CH_2Z$, in which Z is 1H-1,2,4-triazolyl, methylsulfonyl, $X—R_{13}$ or $OSO_2—R_{14}$, wherein X is oxygen or sulfur, $R_{13}$ is an alkyl, alkenyl or alkynyl group, each of which contains at most 4 carbon atoms, and $R_{14}$ is $C_1—C_3$alkyl or $NH(C_1—C_3\ alkyl)$.

5. A composition according to claim 4, wherein in formula I $R_1$ is methyl, methoxy or halogen; $R_2$ is $NO_2$ or $NH_2$; $R_3$ is hydrogen, methyl, methoxy or halogen; each of $R_4$, $R_5$ and R independently is hydrogen or methyl; W is $COOR_8$, wherein $R_8$ is $C_1—C_3$alkyl; and B is 2-furyl, 2-tetrahydrofuryl, $CH_2O(C_1—C_3)$alkyl, $CH_2CH_2OCH_3$, 1H-1,2,4-triazolylmethyl, $CH_2—SO_2CH_3$, $CH_2OSO_2CH_3$, $CH_2OCH_2CH=CH_2$, $CH_2OSO_2NHCH_3$ or $CH_2OCH_2C{\equiv}CH$.

6. A composition according to claim 5, wherein in formula I $R_1$ is methyl, $R_2$ is $NO_2$ or $NH_2$, $R_3$ is hydrogen, methyl, chlorine or bromine, each of $R_4$, $R_5$ and R independently is hydrogen or methyl, and $R_8$ is methyl or isopropyl.

7. A composition according to claim 1, wherein in formula I Ar is the group

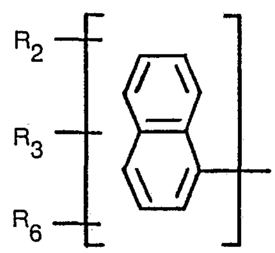

$R_2$ is $NO_2$ or $NH_2$,
$R_3$ is hydrogen, $C_1—C_3$alkyl, $C_1—C_3$alkoxy or halogen,
$R_6$ is hydrogen, $C_1—C_3$alkyl or halogen, with the proviso that the β-position in the molecule is always substituted,
R is hydrogen or methyl,
W is $COOR_8$,
$R_8$ is $C_1—C_3$alkyl,
B is 2-furyl, 2-tetrahydrofuryl, β-($C_1—C_2$alkoxy)ethyl or the group $CH_2Z$, in which Z is 1H-1,2,4-triazolyl, sulfonylmethyl, $X—R_{13}$ or $OSO_2—R_{14}$, wherein X is oxygen or sulfur, $R_{13}$ is an alkyl, alkenyl or alkynyl group, each containing at most 4 carbon atoms, and $R_{14}$ is $C_1—C_3$alkyl or $NH(C_1—C_3alkyl)$.

8. A composition according to claim 7, wherein in formula I $R_2$is $NO_2$ or $NH_2$; $R_3$ is hydrogen, methyl,

methoxy or halogen; $R_6$ is hydrogen, methyl or halogen; R is methyl or hydrogen; W is $COOR_8$, wherein $R_8$ is $C_1$—$C_3$alkyl; and B is 2-furyl, 2-tetrahydrofuryl, $CH_2O(C_1$—$C_3$alkyl), 1H-1,2,4-triazolylmethyl, $CH_2SO_2CH_3$, $CH_2OSO_2CH_3$, $CH_2OCH_2CH{=}CH_2$, $CH_2SO_2NHCH_3$ or $CH_2OCH_2C{\equiv}CH$.

9. A composition according to claim 8, wherein in formula I $R_2$ is $NO_2$ or $NH_2$; $R_3$ is hydrogen, methyl, methoxy, chlorine or bromine; $R_6$ is hydrogen, methyl, chlorine or bromine; R is methyl; and $R_8$ is methyl or isopropyl.

10. A composition according to claim 4, wherein the active ingredient is selected from the group consisting of
N-(1'-methoxycarbonylethyl)-N-methoxyacetyl-2-methyl-6-aminoaniline,
N-(1'-methoxycarbonylethyl)-N-methoxyacetyl-2-methyl-6-nitroaniline,
N-(1'-methoxycarbonylethyl)-N-methoxyacetyl-2,6-dimethyl-3-nitroaniline,
N-(1'-methoxycarbonylethyl)-N-ethoxyacetyl-2,6-dimethyl-3-nitroaniline,
N-(1'-isopropyloxycarbonylethyl)-N-methoxyacetyl-2,6-dimethyl-3-nitroaniline,
N-(1'-methoxycarbonylethyl)-N-methoxyacetyl-2,6-dimethyl-3-aminoaniline,
N-(1'-methoxycarbonylethyl)-N-methoxyacetyl-2,3-dimethyl-6-aminoaniline,
N-(1'-methoxycarbonylethyl)-N-methoxyacetyl-2,3-dimethyl-6-nitroaniline,
N-(1'-methoxycarbonylethyl)-N-(2-furyl)-2-methyl-6-nitroaniline and
N-(1'-methoxycarbonylethyl)-N-(2-tetrahydrofuryl)-2-methyl-6-nitroaniline.

11. A composition according to claim 7, wherein the active ingredient is selected from the group consisting of
1N-(1'-methoxycarbonylethyl)-1N-methoxyacetyl-1,2-diaminonaphthalene,
N-(1'-methoxycarbonylethyl)-N-methoxyacetylamino-2-nitronaphthalene,
N-(1'-methoxycarbonylethyl)-N-methoxyacetylamino-2-methyl-3-nitronaphthalene,
N-(1'-methoxycarbonylethyl)-N-ethoxyacetylamino-2-ethyl-3-nitronaphthalene,
N-(1'-isopropyloxycarbonylethyl)-N-methoxyacetylamino-2-methyl-3-nitronaphthalene,
1N-(1'-methoxycarbonylethyl)-1N-methoxyacetyl-2-methyl-1,3-diaminonaphthalene,
1N-(1'-methoxycarbonylethyl)-1N-methoxyacetyl-3-methyl-1,2-diaminonaphthalene,
N-(1'-methoxycarbonylethyl)-N-methoxyacetyl-amino-3-methyl-2-nitronaphthalene,
N-(1'-methoxycarbonylethyl)-N-(2-furyl)amino-2-nitronaphthalene and
N-(1'-methoxycarbonylethyl)-N-(2-tetrahydrofuryl)-amino-2-nitronaphthalene.

12. A process for the production of a compound of the formula I as defined in claim 1, which process comprises N-acylating either an already N-alkylated arylamino derivative of the formula II

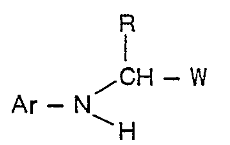 (II)

with a carboxylic acid of the formula III

B—COOH (III)

or an acid halide, acid anhydride or ester thereof, in the temperature range from 0° to 180°C, or activating an already N-acylated arylamino derivative of the formula IV

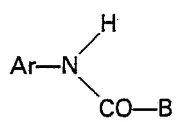 (IV)

with butyl lithium, sodium hydride or an alkali carbonate, and N-alkylating said compound with a compound of the formula V

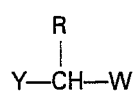 (V)

in the temperature range from 0° to 180°C, in which formulae II, III, IV and V above the substituents Ar, R, W and B are as defined for formula I in claim 1 and Y is one of the customary leaving groups.

13. A process according to claim 12, wherien the acid halide is an acid chloride or bromide.

14. A process according to claim 12, wherein the N-acylation is carried out in the presence of an acid acceptor or a condensing agent.

15. A process according to claim 12, which process is carried out in the presence of an inert solvent or mixture of solvents.

16. A method of controlling and/or protecting plants from attack by phytopathogenic microorganisms, which method comprises applying to said plants or to the locus thereof a microbicidally effective amount of a compound of the formula I according to claim 1.

17. A method according to claim 16 which comprises the use of a compound of the formula I according to any one of claims 2 to 11.

18. A method according to claim 16, wherein the microorganisms to be controlled are phytopathogenic fungi.

19. A method of controlling and/or protecting plants from attack by phytopathogenic fungi, which method comprises applying to said plants or the locus thereof a compound of the formula I according to any one of claims 1 to 11 in finely dispersed form.

**Revendications pour les Etats contractants: BE CH LI DE FR GB IT NL SE**

1. Composé de formule I

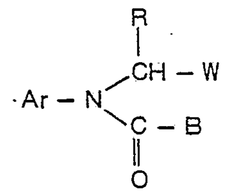

(I)

où

R représente un hydrogène ou un méthyle;

Ar représente l'un des groupes aromatiques

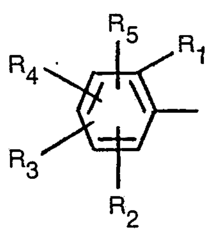

ou

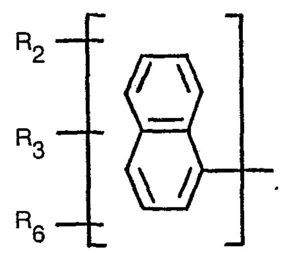

où

$R_1$ représente un alcoyle en $C_1$ à $C_3$, un alcoxy en $C_1$ à $C_3$ ou un halogène;

$R_2$ représente $NO_2$ ou $NH_2$;

$R_3$ représente un hydrogène, un alcoyle en $C_1$ à $C_3$, un alcoxy en $C_1$ à $C_3$ ou un halogène;

$R_4$ représente un hydrogène ou un alcoyle en $C_1$ à $C_3$;

$R_5$ représente un hydrogène ou un alcoyle en $C_1$ à $C_3$;

$R_6$ représente un hydrogène, un alcoyle en $C_1$ à $C_3$ ou un halogène;

W représente un cyano, $—COOR_8$, $—C(R_9)\equiv C(R_{10})(R_{11})$ ou $—C=C—R_{12}$, où

$R_8$ représente un alcoyle en $C_1$ à $C_3$;

$R_9$, $R_{10}$, $R_{11}$ et $R_{12}$ représentent indépendamment l'un de l'autre un hydrogène, un alcoyle en $C_1$ à $C_3$ ou un halogène et

B représente un alcoyle en $C_3$ à $C_4$, un alcényle en $C_2$ à $C_4$, un cyclopropyle, un 2-furyle, un 2-tétrahydrofuryle, un β-($C_1$—$C_2$-alcoxy)éthyle ou le groupe $CH_2Z$, où

Z représente un 1H-1,2,4-triazolyle, un méthylsulfonyle, $X—R_{13}$, $OSO_2—R_{14}$ ou $—N(R_{15})(R_{16})$, où

X représente un oxygène ou un soufre;

$R_{13}$ représente un groupe alcoyle, alcényle ou alcynyle avec au maximum 4 atomes de carbone;

$R_{14}$ représente un alcoyle en $C_1$ à $C_3$ ou $NH(C_1—C_3\text{-alcoyle})$ et

$R_{15}$ et $R_{16}$ représentent indépendamment l'un de l'autre un alcoyle en $C_1$ à $C_3$.

2. Composés de formule I selon la revendication 1, caractérisés en ce que R représente un hydrogène ou un méthyle, Ar représente le groupe

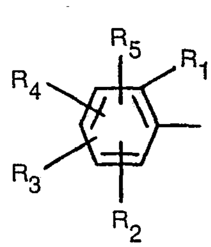

ou

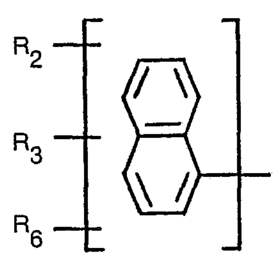

$R_1$ représente un méthyle, éthyle, méthoxy, éthoxy ou halogène; $R_2$ représente un nitro ou un amino; $R_3$ et

$R_6$ représentent indépendamment l'un de l'autre un hydrogène, un méthyle, éthyle, méthoxy, éthoxy ou halogène; $R_4$ et $R_5$ représentent indépendamment l'un de l'autre un hydrogène, un méthyle ou un éthyle; W représente CN, $COOR_8$, $-C(R_9)=C(R_{10})(R_{11})$ ou $-C\equiv C-R_{12}$, où $R_8$ représente un alcoyle en $C_1$ à $C_3$; $R_9$, $R_{10}$, $R_{11}$ et $R_{12}$ représentent indépendamment l'un de l'autre un hydrogène, un méthyle, un chlore ou un brome et B représente un $C_1$—$C_3$-Alcoyle, $C_2$—$C_4$-Alcényle, Cyclopropyle, 2-Furyle, 2-Tétrahydrofuryle, $CH_2O(C_1$—$C_3$-Alcoyle), 1H-Triazolylméthyle, $CH_2SO_2CH_3$, $CH_2OSO_2CH_3$, $CH_2OCH_2CH=CH_2$, $CH_2OCH_2C\equiv CH$, $CH_2OSO_2NHCH_3$, $CH_2N(CH_3)_2$, $CH_2N(C_2H_5)_2$ ou $CH_2N(C_3H_7\text{-}n)_2$.

3. Composés de formule I selon la revendication 2, caractérisés en ce que R représente un méthyle; $R_1$ représente un méthyle, un méthoxy, un chlore ou un brome; $R_3$ et $R_6$ représentent indépendamment l'un de l'autre un hydrogène, un méthyle, un chlore ou un brome; $R_4$ et $R_5$ représentent indépendamment l'un de l'autre un hydrogène ou un méthyle; W représente CN, $COOR_8$, $-C(R_9)=C(R_{10})(R_{11})$ ou $-C\equiv C-R_{12}$, où $R_9$, $R_{10}$, $R_{11}$ et $R_{12}$ représentent indépendamment l'un de l'autre un hydrogène, un méthyle ou un chlore et B représente un 2-Furyle, 2-Tétrahydrofuryle, $CH_2O(C_1$—$C_3$-Alcoyle), 1H-1,2,4-Triazolylméthyle, $CH_2SO_2CH_3$, $CH_2OSO_2CH_3$, $CH_2OCH_2CH=CH_2$, $CH_2OCH_2C\equiv CH$, $CH_2OSO_2NHCH_3$, $CH_2N(CH_3)_2$, $CH_2N(C_2H_5)_2$ ou $CH_2N(C_3H_7\text{-}n)_2$.

4. Composés de formule I selon la revendication 1, caractérisés en ce que Ar représente le groupe

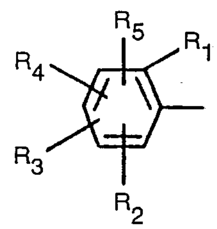

$R_1$ représente un alcoyle en $C_1$ à $C_3$, un alcoxy en $C_1$ à $C_3$ ou un halogène,
$R_2$ représente $NO_2$ ou $NH_2$,
$R_3$ représente un hydrogène, un alcoyle en $C_1$ à $C_3$, un alcoxy en $C_1$ à $C_3$ ou un halogène,
$R_4$ représente un hydrogène ou un alcoyle en $C_1$ à $C_3$,
$R_5$ représente un hydrogène ou un alcoyle en $C_1$ à $C_3$, avec la précision que les deux positions ortho dans la fraction aniline de la molécule sont substituées,
R représente un hydrogène ou un méthyle,
W représente $COOR_8$,
$R_8$ représente un alcoyle en $C_1$ à $C_3$,
B représente un 2-furyle, 2-tétrahydrofuryle, β-($C_1$—$C_2$-alcoxy)éthyle ou le groupe $CH_2Z$, où Z représente un 1H-1,2,4-triazolyle, un méthylsulfonyle, $X-R_{13}$ ou $OSO_2-R_{14}$, où X représente un oxygène ou un soufre, $R_{13}$ représente un groupe alcoyle, alcényle ou alcynyle avec au maximum 4 atomes de carbone et $R_{14}$ représente un alcoyle en $C_1$ à $C_3$ ou $NH(C_1$—$C_3$-alcoyle).

5. Composés de formule I selon la revendication 4, caractérisés en ce que $R_1$ représente un méthyle, un méthoxy ou un halogéne, $R_2$ représente $NO_2$ ou $NH_2$, $R_3$ représente unhydrogène, un méthyle, un méthoxy ou un halogène, $R_4$, $R_5$ et R représentent indépendamment l'un de l'autre un hydrogène ou un méthyle, W représente $COOR_8$, $R_8$ représente un alcoyle en $C_1$ à $C_3$ et B représente un 2-furyle, un 2-tétrahydrofuryle, $CH_2O(C_1$—$C_3$)-alcoyle), $CH_2CH_2OCH_3$, 1H-1,2,4-triazolylméthyle, $CH_2-SO_2CH_3$, $CH_2OSO_2CH_3$, $CH_2OCH_2CH=CH_2$, $CH_2OSO_2NHCH_3$ ou $CH_2OCH_2C\equiv CH$.

6. Composés de formule I selon la revendication 5, caractérisés en ce que $R_1$ représente un méthyle, $R_2$ représente $NO_2$ ou $NH_2$, $R_3$ représente un hydrogène, un méthyle, un chlore ou un brome, $R_4$, $R_5$ et R représentent indépendamment l'un de l'autre un hydrogène ou un méthyle et $R_8$ représente un méthyle ou un isopropyle.

7. Composés de formule I selon la revendication 1, caractérisés en ce que Ar représente le groupe

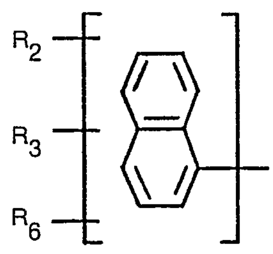

$R_2$ représente $NO_2$ ou $NH_2$,
$R_3$ représente un hydrogène, un alcoyle en $C_1$ à $C_3$, un alcoxy en $C_1$ à $C_3$ ou un halogène,
$R_6$ représente un hydrogène, un alcoyle en $C_1$ à $C_3$ ou un halogène, avec la précision que la position β dans la molécule est toujours substituée;
R représente un hydrogène ou un méthyle,

W représente $COOR_8$,
$R_8$ représente un alcoyle en $C_1$ à $C_3$,
B représente un 2-furyle, un 2-tétrahydrofuryle, un β-($C_1$—$C_2$-alcoxy)éthyle ou le groupe $CH_2Z$, où Z représente un 1H-1,2,4-triazolyle, un sulfonylméthyle, X—$R_{13}$ ou $OSO_2$—$R_{14}$, où X représente un oxygène ou un soufre, $R_{13}$ représente un groupe alcoyle, alcényle ou alcynyle avec au maximum 4 atomes de carbone et $R_{14}$ représente un alcoyle en $C_1$ à $C_3$ ou NH($C_1$—$C_3$-alcoyle).

8. Composés de formule I selon la revendication 7, caractérisés en ce que $R_2$ représente $NO_2$ ou $NH_2$, $R_3$ représente un hydrogène, un méthyle, un méthoxy ou un halogène, $R_6$ représente un hydrogène, un méthyle ou un halogène, R représente un méthyle ou un hydrogène, W représente $COOR_8$, $R_8$ représente un alcoyle en $C_1$ à $C_3$ et B représente un 2-Furyle, 2-Tétrahydrofuryle $CH_2O$($C_1$—$C_3$-Alcoyle), 1H-1,2,4-Triazolyleméthyle, $CH_2SO_2CH_3$, $CH_2OSO_2CH_3$, $CH_2OCH_2CH{=}CH_2$, $CH_2SO_2NHCH_3$ ou $CH_2OCH{\equiv}CH$.

9. Composés de formule I selon la revendication 8, caractérisés en ce que $R_2$ représente $NO_2$ ou $NH_2$, $R_3$ représente un hydrogène, un méthyle, un méthoxy, un chlore ou un brome, $R_6$ représente un hydrogène, un méthyle, un chlore ou un brome R représente un méthyle et $R_8$ représente un méthyle ou un iso-propyle.

10. Composé de formule I selon la revendication 4, choisi dans le groupe
N-(1'-Méthoxycarbonyl-éthyl)-N-méthoxyacétyl-2-méthyl-6-aminoaniline
N-(1'-Méthoxycarbonyl-éthyl)-N-méthoxyacétyl-2-méthyl-6-nitroaniline
N-(1'-Méthoxycarbonyl-éthyl)-N-méthoxyacétyl-2,6-diméthyl-3-nitroaniline
N-(1'-Méthoxycarbonyl-éthyl)-N-éthoxyacétyl-2,6-diméthyl-3-nitroaniline
N-(1'-Isopropyloxycarbonyl-éthyl)-N-méthoxyacétyl-2,6-diméthyl-3-nitroaniline
N-(1'-Méthoxycarbonyl-éthyl)-N-méthoxyacétyl-2,6-diméthyl-3-aminoaniline
N-(1'-Méthoxycarbonyl-éthyl)-N-méthoxyacétyl-2,3-diméthyl-6-aminoaniline
N-(1'-Méthoxycarbonyl-éthyl)-N-méthoxyacétyl-2,3-diméthyl-6-nitroaniline
N-(1'-Méthoxycarbonyl-éthyl)-N-(2-furyl)-2-méthyl-6-nitroaniline ou
N-(1'-Méthoxycarbonyl-éthyl)-N-(2-tétrahydrofuryl)-2-méthyl-6-nitroaniline.

11. Composé de formule I selon la revendication 7, choisi dans le groupe:
1N-(1'-Méthoxycarbonyl-éthyl)-1N-méthoxyacétyl-1,2-diaminonaphtaline
N-(1'-Méthoxycarbonyl-éthyl)-N-méthoxyacétyl-amino-2-nitro-naphtaline
N-(1'-Méthoxycarbonyl-éthyl)-N-méthoxyacétyl-amino-2-méthyl-3-nitro-naphtaline
N-(1'-Méthoxycarbonyl-éthyl)-N-éthoxyacétyl-amino-2-éthyl-3-nitro-naphtaline
N-(1'-Isopropyloxycarbonyl-éthyl)-N-méthoxyacétyl-amino-2-méthyl-3-nitro-naphtaline
1N-(1'-Méthoxycarbonyl-éthyl)-1N-méthoxyacétyl-2-méthyl-1,3-diamino-naphtaline
1N-(1'-Méthoxycarbonyl-éthyl)-1N-méthoxyacétyl-3-méthyl-1,2-diamino-naphtaline
N-(1'-Méthoxycarbonyl-éthyl)-N-méthoxyacétyl-amino-3-méthyl-2-nitro-naphtaline
N-(1'-Méthoxycarbonyl-éthyl)-N-(2-furyl)-amino-2-nitro-naphtaline
N-(1'-Méthoxycarbonyl-éthyl)-N-(2-tétrahydrofuryl)-amino-2-nitro-naphtaline.

12. Procédé de préparation de composés de formule I définie dans la revendication 1, caractérisé au choix en ce qu'on N-acyle un dérivé arylamino de formule II déjà N-alcoylé.

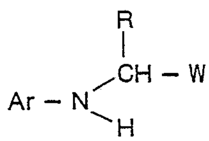
(II)

avec un acide carboxylique de formule III,

B—COOH (III)

son halogénure d'acide, anhydride d'acide ou ester à des températures comprises entre 0° et +180°C, ou en ce qu'on active un dérivé d'arylamine déjà N-acylé de formule IV

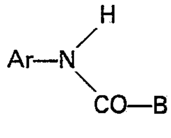
(IV)

avec du butyl-lithium, de l'hydrure de sodium ou un carbonate alcalin et en ce qu'on le N-alcoyle avec un composé de formule V

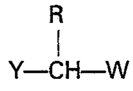
(V)

à des températures comprises entre 0° et +180°C, où les substituants Ar, R, W et B sont définis comme pour la formule I de la revendication 1, et Y représente l'un des groupes sortants habituels.

13. Agent pour combattre et/ou prévenir une attaque de microorganismes nocifs, caractérisé en ce qu'il contient comme composant actif au moins une matière active de formule I selon la revendication 1.

14. Agent selon la revendication 13, caractérisé en ce qu'il contient de 0,01 à 90% en poids d'une matière active de formule I selon la revendication 1, de 10 à 99,99% d'additifs et de 0 à 33% en poids d'un agent tensio-actif.

15. Agent selon l'une des revendications 13 et 14, caractérisé en ce qu'il contient comme matière active l'un des composés de formule I selon l'une des revendications 2 à 11.

16. Procédé de préparation d'agent pour combattre et/ou prévenir une attaque de microorganismes, caractérisé en ce qu'on mélange intimement un composé de formule I selon l'une des revendications 1 à 11 avec des supports et/ou agents tensio-actifs appropriés.

17. Application de composés de formule I selon la revendication 1 pour combattre et/ou prévenir une attaque de plantes par des microorganismes phytopathogènes.

18. Application selon la revendication 17 de composés de formule I selon l'une des revendications 2 à 11.

19. Application selon la revendication 17, caractérisée en ce qu'il s'agit en ce qui concerne les microorganismes de champignons phytopathogènes.

20. Procédé pour combattre et/ou prévenir une attaque des plantes par des champignons phytopathogènes, caractérisé en ce qu'on applique un composé de formule I selon l'une des revendications 1 à 11, sous forme finement divisée sur les plantes ou le lieu où elles se trouvent.

**Revendications pour l'Etat contractant: AT**

1. Agent pour combattre et/ou prévenir une attaque de microoranismes nocifs, caractérisé en ce qu'il contient avec des supports habituels et des additifs favorisant l'application, comme composant actif au moins une matière active de formule I

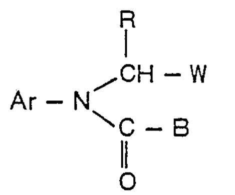

(I)

où

R représente un hydrogène ou un méthyle;

Ar représente l'un des groupes aromatiques

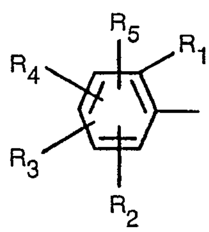

ou

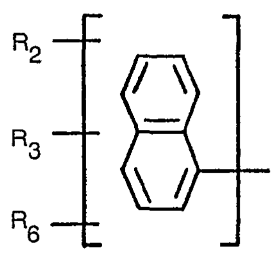

où

$R_1$ représente un alcoyle en $C_1$ à $C_3$, un alcoxy en $C_1$ à $C_3$ ou un halogène;

$R_2$ représente $NO_2$ ou $NH_2$;

$R_3$ représente un hydrogène, un alcoyle en $C_1$ à $C_3$, un alcoxy en $C_1$ à $C_3$ ou un halogène;

$R_4$ représente un hydrogène ou un alcoyle en $C_1$ à $C_3$;

$R_5$ représente un hydrogène ou un alcoyle en $C_1$ à $C_3$;

$R_6$ représente un hydrogène, un alcoyle en $C_1$ à $C_3$ ou un halogène;

W représente un cyano, $-COOR_8$, $-C(R_9){\equiv}C(R_{10})(R_{11})$ ou $-C{=}C-R_{12}$, où

$R_8$ représente un alcoyle en $C_1$ à $C_3$;

$R_9$, $R_{10}$, $R_{11}$ et $R_{12}$ représentent indépendamment l'un de l'autre un hydrogène, un alcoyle en $C_1$ à $C_3$ ou un halogène et

B représente un alcoyle en $C_3$ à $C_4$, un alcényle en $C_2$ à $C_4$, un cyclopropyle, un 2-furyle, un 2-tétrahydrofuryle, un β-($C_1$—$C_2$-alcoxy)éthyle ou le groupe $CH_2Z$, où

Z représente un 1H-1,2,4-triazolyle, un méthylsulfonyle, X—$R_{13}$, $OSO_2$—$R_{14}$ ou —$N(R_{15})(R_{16})$, où

X représente un oxygène ou un soufre;

$R_{13}$ représente un groupe alcoyle, alcényle ou alcynyle avec au maximum 4 atomes de carbone;

$R_{14}$ représente un alcoyle en $C_1$ à $C_3$ ou NH($C_1$—$C_3$-alcoyle) et

$R_{15}$ et $R_{16}$ représentent indépendamment l'un de l'autre un alcoyle en $C_1$ à $C_3$.

2. Agent selon la revendication 1, caractérisés en ce que dans la formule I R représente un hydrogène ou un méthyle, Ar représente le groupe

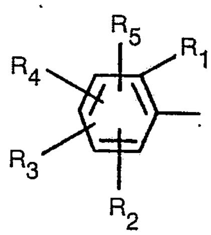

ou

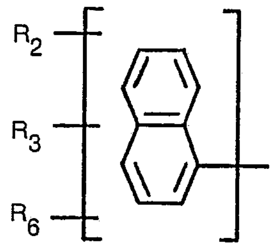

$R_1$ représente un méthyle, éthyle, méthoxy, éthoxy ou halogène; $R_2$ représente un nitro ou un amino; $R_3$ et $R_6$ représentent indépendamment l'un de l'autre un hydrogène, un méthyle, éthyle, méthoxy, éthoxy ou halogène; $R_4$ et $R_5$ représentent indépendamment l'un de l'autre un hydrogène, un méthyle ou un éthyle; W représente CN, $COOR_8$, $—C(R_9)=C(R_{10})(R_{11})$ ou $—C{\equiv}C—R_{12}$, où $R_8$ représente un alcoyle en $C_1$ à $C_3$; $R_9$, $R_{10}$, $R_{11}$ et $R_{12}$ représentent indépendamment l'un de l'autre un hydrogène, un méthyle, un chlore ou un brome et B représente un $C_1—C_3$-Alcoyle, $C_2—C_4$-Alcényle, Cyclopropyle, 2-Furyle, 2-Tétrahydrofuryle, $CH_2O(C_1—C_3$-Alcoyle), 1H-Triazolylméthyle, $CH_2SO_2CH_3$, $CH_2OSO_2CH_3$, $CH_2OCH_2CH=CH_2$, $CH_2OCH_2C{\equiv}CH$, $CH_2OSO_2NHCH_3$, $CH_2N(CH_3)_2$, $CH_2N(C_2H_5)_2$ ou $CH_2N(C_3H_7\text{-}n)_2$.

3. Agent selon la revendication 2, caractérisé en ce que dans la formule I R représente un méthyle; $R_1$ représente un méthyle, un méthoxy, un chlore ou un brome; $R_3$ et $R_6$ représentent indépendamment l'un de l'autre un hydrogène, un méthyle, un chlore ou un brome; $R_4$ et $R_5$ représentent indépendamment l'un de l'autre un hydrogène ou un méthyle; W représente CN, $COOR_8$, $—C(R_9)=C(R_{10})(R_{11})$ ou $—C{\equiv}C—R_{12}$, où $R_9$, $R_{10}$, $R_{11}$ et $R_{12}$ représentent indépendamment l'un de l'autre un hydrogène, un méthyle ou un chlore et B représente un 2-Furyle, 2-Tétrahydrofuryle, $CH_2O(C_1—C_3$-Alcoyle), 1H-1,2,4-Triazolylméthyle, $CH_2SO_2CH_3$, $CH_2OSO_2CH_3$, $CH_2OCH_2CH=CH_2$, $CH_2OCH_2C{\equiv}CH$, $CH_2OSO_2NHCH_3$, $CH_2N(CH_3)_2$, $CH_2N(C_2H_5)_2$ ou $CH_2N(C_3H_7\text{-}n)_2$.

4. Agent selon la revendication 1, caractérisé en ce que dans la formule I Ar représente le groupe

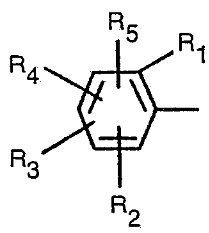

$R_1$ représente un alcoyle en $C_1$ à $C_3$, un alcoxy en $C_1$ à $C_3$ ou un halogène,

$R_2$ représente $NO_2$ ou $NH_2$,

$R_3$ représente un hydrogène, un alcoyle en $C_1$ à $C_3$, un alcoxy en $C_1$ à $C_3$ ou un halogène,

$R_4$ représente un hydrogène ou un alcoyle en $C_1$ à $C_3$,

$R_5$ représente un hydrogène ou un alcoyle en $C_1$ à $C_3$, avec la précision que les deux positions ortho dans la fraction aniline de la molécule sont substituées,

R représente un hydrogène ou un méthyle,

W représente $COOR_8$,

$R_8$ représente un alcoyle en $C_1$ à $C_3$,

B représente un 2-furyle, 2-tétrahydrofuryle, un β-($C_1—C_2$-alcoxy)éthyle ou le groupe $CH_2Z$, où Z représente un 1H-1,2,4-triazolyle, un méthylsulfonyle, $X—R_{13}$ ou $OSO_2—R_{14}$, où X représente un oxygène ou un soufre, $R_{13}$ représente un groupe alcoyle, alcényle ou acynyle avec au maximum 4 atomes de carbone et $R_{14}$ représente un alcoyle en $C_1$ à $C_3$ ou $NH(C_1—C_3$-alcoyle).

5. Agent selon la revendication 4, caractérisé en ce que dans la formule I $R_1$ représente un méthyle, un méthoxy ou un halogéne, $R_2$ $NO_2$ ou $NH_2$, $R_3$ représente unhydrogène, un méthyle, un méthoxy ou un halogène, $R_4$, $R_5$ et R représentent indépendamment l'un de l'autre un hydrogène ou un méthyle, W représente $COOR_8$, $R_8$ représente an alcoyle en $C_1$ à $C_3$ et B représente un 2-Furyle, 2-Tétrahydrofuryle, $CH_2O(C_1—C_3)$-Alcoyle), $CH_2CH_2OCH_3$, 1H-1,2,4-Triazolylméthyle, $CH_2—SO_2CH_3$, $CH_2OSO_2CH_3$, $CH_2OCH_2CH=CH_2$, $CH_2OSO_2NHCH_3$ ou $CH_2OCH_2C{\equiv}CH$.

6. Agent selon la revendication 5 caractérisé en ce que dans la formule I $R_1$ représente un méthyle, $R_2$ représente $NO_2$ ou $NH_2$, $R_3$ représente un hydrogène, un méthyle, un chlore ou un brome, $R_4$, $R_5$ et R représentent indépendamment l'un de l'autre un hydrogène ou un méthyle et $R_8$ représente un méthyle ou un isopropyle.

7. Agent selon la revendication 1, caractérisé en ce que dans la formule I Ar représente le groupe

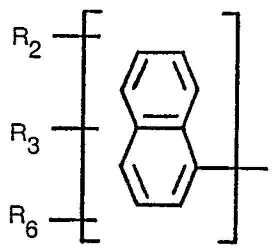

$R_2$ représente $NO_2$ ou $NH_2$,
$R_3$ représente un hydrogène, un alcoyle en $C_1$ à $C_3$, un alcoxy en $C_1$ à $C_3$ ou un halogène,
$R_6$ représente un hydrogène, un alcoyle en $C_1$ à $C_3$ ou un halogène, avec la précision que la position β dans la molécule est toujours substituée;
R représente un hydrogène ou un méthyle,
W représente $COOR_8$,
$R_8$ représente un alcoyle en $C_1$ à $C_3$,
B représente un 2-furyle, un 2-tétrahydrofuryle, un β-($C_1$—$C_2$-alcoxy)éthyle ou le groupe $CH_2Z$, où Z représente un 1H-1,2,4-triazolyle, un sulfonylméthyle, X—$R_{13}$ ou $OSO_2$—$R_{14}$, où X représente un oxygène ou un soufre, $R_{13}$ représente un groupe alcoyle, alcényle ou alcynyle avec au maximum 4 atomes de carbone et $R_{14}$ représente un alcoyle en $C_1$ à $C_3$ ou NH($C_1$—$C_3$-alcoyle).

8. Agent selon la revendication 7, caractérisé en ce que dans la formule I $R_2$ représente $NO_2$ ou $NH_2$, $R_3$ représente un hydrogène, un méthyle, un méthoxy ou un halogène, $R_6$ représente un hydrogène, un méthyle ou un halogène, R représente un méthyle ou un hydrogène, W représente $COOR_8$, $R_8$ représente un alcoyle en $C_1$ à $C_3$ et B représente un 2-Furyle, 2-Tétrahydrofuryle $CH_2O$($C_1$—$C_3$-Alcoyle), 1H-1,2,4-Triazolyleméthyle, $CH_2SO_2CH_3$, $CH_2OSO_2CH_3$, $CH_2OCH_2CH{=}CH_2$, $CH_2SO_2NHCH_3$ ou $CH_2OCH_2C{\equiv}CH$.

9. Agent selon la revendication 8, caractérisé en ce que dans la formule I $R_2$ représente $NO_2$ ou $NH_2$, $R_3$ représente un hydrogène, un méthyle, un méthoxy, un chlore ou un brome, $R_6$ représente un hydrogène, un méthyle, un chlore ou un brome, R représente un méthyle et $R_8$ représente un méthyle ou un isopropyle.

10. Agent selon la revendication 4, caractérisé on ce qu'on choisit la matière active dans le groupe
N-(1'-Méthoxycarbonyl-éthyl)-N-méthoxyacétyl-2-méthyl-6-aminoaniline
N-(1'-Méthoxycarbonyl-éthyl)-N-méthoxyacétyl-2-méthyl-6-nitroaniline
N-(1'-Méthoxycarbonyl-éthyl)-N-méthoxyacétyl-2,6-diméthyl-3-nitroaniline
N-(1'-Méthoxycarbonyl-éthyl)-N-éthoxyacétyl-2,6-diméthyl-3-nitroaniline
N-(1'-Isopropyloxycarbonyl-éthyl)-N-méthoxyacétyl-2,6-diméthyl-3-nitroaniline
N-(1'-Méthoxycarbonyl-éthyl)-N-méthoxyacétyl-2,6-diméthyl-3-aminoaniline
N-(1'-Méthoxycarbonyl-éthyl)-N-méthoxyacétyl-2,3-diméthyl-6-aminoaniline
N-(1'-Méthoxycarbonyl-éthyl)-N-méthoxyacétyl-2,3-diméthyl-6-nitroaniline
N-(1'-Méthoxycarbonyl-éthyl)-N-(2-furyl)-2-méthyl-6-nitroaniline ou
N-(1'-Méthoxycarbonyl-éthyl)-N-(2-tétrahydrofuryl)-2-méthyl-6-nitroaniline.

11. Agent selon la revendication 7, caractérisé en ce qu'on choisit la matière active dans le groupe
1N-(1'-Méthoxycarbonyl-éthyl)-1N-méthoxyacétyl-1,2-diaminonaphtaline
N-(1'-Méthoxycarbonyl-éthyl)-N-méthoxyacétyl-amino-2-nitro-naphtaline
N-(1'-Méthoxycarbonyl-éthyl)-N-méthoxyacétyl-amino-2-méthyl-3-nitro-naphtaline
N-(1'-Méthoxycarbonyl-éthyl)-N-éthoxyacétyl-amino-2-éthyl-3-nitro-naphtaline
N-(1'-Isopropyloxycarbonyl-éthyl)-N-méthoxyacétyl-amino-2-méthyl-3-nitro-naphtaline
1N-(1'-Méthoxycarbonyl-éthyl)-ethyl-1N-méthoxyacétyl-2-méthyl-1,3-diamino-naphtaline
1N-(1'-Méthoxycarbonyl-éthyl)-1N-méthoxyacétyl-3-méthyl-1,2-diamino-naphtaline
N-(1'-Méthoxycarbonyl-éthyl)-N-méthoxyacétyl-amino-3-méthyl-2-nitro-naphtaline
N-(1'-Méthoxycarbonyl-éthyl)-N-(2-furyl)-amino-2-nitro-naphtaline
N-(1'-Méthoxycarbonyl-éthyl)-N-(2-tétrahydrofuryl)-amino-2-nitro-naphtaline.

12. Procédé de préparation de composés de formule I définie dans la revendication 1, caractérisé en ce qu'au choix on N-acyle un dérivé arylamino N-alcoylé de formule II

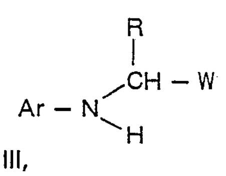
(II)

avec un acide carboxylique de formule III,

B—COOH (III)

son halogénure d'acide, anhydride d'acide ou ester à des températures comprises entre 0° et +180°C, ou en ce qu'on active un dérivé d'arylamine déjà N-acylé de formule IV

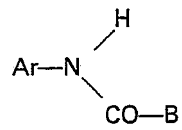

(IV)

avec du butyl-lithium, de l'hydrure de sodium ou un carbonate alcalin et en ce qu'on N-alcoyle avec un composé de formule V

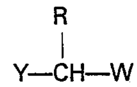

(V)

à des températures comprises entre 0° et +180°C, où les substituants Ar, R, W et B sont tels que définis pour la formule I dans la revendication 1 et Y représente l'un des groupes sortants habituels.

13. Procédé selon la revendication 12, caractérisé en ce qu'il s'agit pour ce qui est de l'halogénure d'acide d'un chlorure ou bromure.

14. Procédé selon la revendication 12, caractérisé en ce que la N-acylation est conduite en présence d'un agent liant acide ou d'un agent de condensation.

15. Procédé selon la revendication 12, caractérisé en ce qu'on le conduit en présence d'un solvant ou mélange de solvants inerte vis-à-vis de la réaction.

16. Application de composés de formule I selon la revendication 1, pour combattre et/ou prévenir une attaque de plantes par des microorganismes phytopathogènes.

17. Application selon la revendication 16 de composés de formule I selon l'une des revendications 2 à 11.

18. Application selon la revendication 16, caractérisée en ce qu'il, s'agit pour ce qui est des microorganismes de champignons phytopathogènes.

19. Procédé pour combattre et/ou prévenir une attaque des plantes par des champignons phytopathogènes, caractérisé en ce qu'on applique un composé de formule I selon l'une des revendications 1 à 11 sous une forme finement divisée sur les plantes ou l'endroiut où elles se trouvent.